# EUROPEAN PATENT SPECIFICATION

(11) **EP 3 146 071 B1**
(45) Date of publication and mention of the grant of the patent: **02.09.2020**
(21) Application number: 15727804.5
(22) Date of filing: 22.05.2015
(51) Int. Cl.: C12Q 1/68

(54) **MIT BIOMARKERS AND METHODS USING THE SAME**
MIT-BIOMARKER UND VERFAHREN MIT VERWENDUNG DAVON
BIOMARQUEURS MIT ET LEURS MÉTHODES D'UTILISATION

(30) Priority: 23.05.2014 US 201462002612 P; 03.10.2014 US 201462059362 P; 30.01.2015 US 201562109775 P
(43) Date of publication of application: 29.03.2017
(73) Proprietor: F. Hoffmann-La Roche AG, 4070 Basel (CH)
(72) Inventor: MODRUSAN, Zora, South San Francisco, California 94080 (US); SESHAGIRI, Somasekar, South San Francisco, California 94080 (US); JAISWAL, Bijay, South San Francisco, California 94080 (US); ZHANG, Na, South San Francisco, California 94080 (US); DURINCK, Steffen, South San Francisco, California 94080 (US); STAWISKI, Eric, South San Francisco, California 94080 (US)
(74) Representative: Winkler, Thomas
(86) International application number: PCT/US2015/032294
(87) International publication number: WO 2015/179835

(56) References cited:
- WO-A1-98/06871
- WO-A1-2009/059142
- US-A1- 2012 095 078
- G MARTIGNONI ET AL: "Cathepsin-K immunoreactivity distinguishes MiTF/TFE family renal translocation carcinomas from other renal carcinomas", MODERN PATHOLOGY, vol. 22, no. 8, 24 April 2009 (2009-04-24) , pages 1016-1022, XP055211297, ISSN: 0893-3952, DOI: 10.1038/modpathol.2009.58
- ANDREW J. WAGNER ET AL: "Tivantinib (ARQ 197), a selective inhibitor of MET, in patients with microphthalmia transcription factor-associated tumors", CANCER, vol. 118, no. 23, 1 December 2012 (2012-12-01), pages 5894-5902, XP055211454, ISSN: 0008-543X, DOI: 10.1002/cncr.27582
- STEPHAN MACHER-GOEPPINGER ET AL: "Molecular heterogeneity of TFE3 activation in renal cell carcinomas", MODERN PATHOLOGY, 28 October 2011 (2011-10-28), XP055211453, ISSN: 0893-3952, DOI: 10.1038/modpathol.2011.169
- LIQING ZHUANG ET AL: "Mcl-1, Bcl-XL and Stat3 expression are associated with progression of melanoma whereas Bcl-2, AP-2 and MITF levels decrease during progression of melanoma", MODERN PATHOLOGY, vol. 20, no. 4, 1 April 2007 (2007-04-01), pages 416-426, XP055212025, ISSN: 0893-3952, DOI: 10.1038/modpathol.3800750
- RACHEL KOBOS ET AL: "Combining integrated genomics and functional genomics to dissect the biology of a cancer-associated, aberrant transcription factor, the ASPSCR1-TFE3 fusion oncoprotein", THE JOURNAL OF PATHOLOGY, vol. 229, no. 5, 5 March 2013 (2013-03-05) , pages 743-754, XP055211456, ISSN: 0022-3417, DOI: 10.1002/path.4158
- G. G. MALOUF ET AL: "Next-Generation Sequencing of Translocation Renal Cell Carcinoma Reveals Novel RNA Splicing Partners and Frequent Mutations of Chromatin-Remodeling Genes", CLINICAL CANCER RESEARCH, vol. 20, no. 15, 4 June 2014 (2014-06-04), pages 4129-4140, XP055211294, ISSN: 1078-0432, DOI: 10.1158/1078-0432.CCR-13-3036

## Description

### FIELD

Provided are therapies related to the treatment of pathological conditions, such as cancer.

### BACKGROUND

Kidney cancer accounts for ∼60,000 new cases and ∼13,000 deaths annually in the United States (Siegel et al., 2013). About 85% of kidney cancers are renal cell carcinoma (RCC), which arise from the renal epithelium. Clear cell RCC (ccRCC), which constitutes 75% of RCCs, is the best characterized kidney cancer subtype (Pena-Llopis et al., 2012; Sato et al., 2013; TCGA, 2013). The remaining 25% of RCCs broadly classified as non-clear cell RCCs (nccRCCs) represent distinct tumor subtypes, including papillary (pRCC; 10-15%) and chromophobe (chRCC; 4-5%) (Osunkoya, 2010; Picken, 2010; Young, 2010; Yusenko, 2010a, b). In needle core biopsies, chRCC is at times difficult to distinguish from renal oncocytoma (RO), a benign kidney epithelial tumor with an incidence rate of ∼5% (Osunkoya, 2010; Picken, 2010; Young, 2010; Yusenko, 2010a, b). Their diagnosis remains a challenge and is compounded by the presence of mixed tumors that show features of both RO and chRCC (Osunkoya, 2010; Picken, 2010; Young, 2010; Yusenko, 2010a, b). Other nccRCC types include collecting duct (<1%), translocation (tRCC; rare) and medullary (rare). About 4-5% of tumors remain unclassified(Bellmunt and Dutcher, 2013). While infrequent, tRCC tend to affect adolescents and young adults and are particularly devastating. Once nccRCCs metastasize, the disease generally remains incurable. While several drugs have recently been approved for metastatic RCC, registration trials involved almost exclusively patients with ccRCC, and there are no treatments with demonstrated efficacy in nccRCC subtypes (Bellmunt and Dutcher, 2013).

WO2009/059142 describes a translocation of TFE3 in renal cell carcinoma, the translocatioin is detected using a probe. WO98/06871 describes fusions of TFE3 with other genes in renal cancer samples.

Martignoni et al. (2009) Modern Pathology, Vol. 22, No. 8, pp 1016-1022 describe TFE3 and TFEB translocations and fusions in renal cancer samples and analyses their samples for the expression of TFE3, TFEB, and MITF. Wagner et al. (2012) Cancer, Vol 118, No. 23 pp 5894-5902 describe the treatment of patients with MiT translocations which lead to overexpression of TFE3 and TFEB with Trivantimib which inhibits MET.

There remains a need to better understand the pathogenesis of cancers, in particular, human renal cell carcinomas and also to identify new therapeutic targets.

### SUMMARY

Methods are provided for determining MiT biomarker expression (determining presence of MiT biomarker), comprising the step of determining whether a sample from an individual expresses MiT biomarker. In some embodiments, MiT is MITF. In some aspects of the disclosure, MiT is TFEB. In some aspects of the disclosure, MiT is TFEC. In some aspects of the disclosure, MiT is TFE3. In some aspects of the dislcosure MiT is SBNO2.

In some aspects of the disclosure of any of the methods of the invention, presence of biomarker is indicated by the presence of elevated biomarker expression level (e.g., compared to reference expression level). In some embodiments, one or more biomarker comprises a translocation or inversion (e.g., rearrangement and/or fusion) of MITF. In some embodiments, translocation is a MITF translocation. In some embodiments, the MITF translocation comprises ACTG1 and MITF. In some embodiments, the MITF translocation comprises ACTG1 exon 3. In some embodiments, the MITF translocation comprises ACTG1 exon 3 and MITF exon 3. In some embodiments, MITF translocation comprises SEQ ID NO:13 and/or 30. In some embodiments, MITF translocation comprises SEQ ID NO: 30. In some embodiments, the MITF translocation is detectable by primers which consist of or comprise SEQ ID NO:11 and/or 12. In some embodiments, the MITF translocation is detectable by primers which consist of or comprise SEQ ID NO:9, 10, 11 and/or 12. In some embodiments, the MITF translocation is driven by the ACTG1 promoter. In some embodiments, the MITF translocation comprises AP3S1 and MITF. In some embodiments, the MITF translocation comprises AP3S1 exon 3. In some embodiments, the MITF translocation comprises ACTG1 exon 3 and MITF exon 3. In some embodiments, wherein the MITF translocation is driven by the AP3S1 promoter. In some aspects of the disclosure , the translocation is a TFEB translocation. In some aspects of the disclosure, the TFEB translocation comprises CLTC and TFEB. In some aspects of the disclosure, the TFEB translocation comprises CLTC exon 17. In some aspects of the disclosure, the TFEB translocation comprises CLTC exon 17 and TFEB exon 6. In some aspects of the disclosure, the TFEB translocation comprises SEQ ID NO:19. In some aspects of the disclosure, theTFEB translocation is detectable by primers which consist of or comprise SEQ ID NO:17 and/or 18. In some aspects of the disclosure, the TFEB translocation is detectable by primers which consist of or comprise SEQ ID NO:15, 16, 17 and/or 18. In some aspects of the disclosure, TFEB translocation is driven by the CLTC promoter. In some aspects of the disclosure, the translocation is a SBNO2 inversion. In some aspects of the disclosure, SBNO2 translocation inversion comprises MIDN and SBNO2. In some aspects of the disclosure, SBNO2 inversion comprises MIDN promoter. In some aspects of the disclosure, the SBNO2 inversion comprises MIDN promoter and SBNO2 exon 1. In some aspects of the disclosure , the SBNO2 inversion comprises SEQ ID NO:25. In some aspects of the disclosure , the SBNO2 inversion is detectable by primers which consist of or comprise SEQ ID NO:23 and/or 24. In some aspects of the disclosure, the SBNO2 inversion is detectable by primers which consist of or comprise SEQ ID NO:21, 22, 23, and/or 25. In some aspects of the disclosure, SBNO2 inversion is driven by the CLTC promoter.

In some embodiments of any of the methods of the invention, the MiT translocation results in elevated expression levels of MET (e.g., compared to a reference without the MiT translocation).

In some embodiments of any of the methods of the invention, the MiT translocation (e.g., rearrangement and/or fusion) results in elevated activity and/or activation of MET (e.g., compared to a reference without the MiT translocation).

In some embodiments of any of the methods of the invention, the MITF translocation (e.g., rearrangement and/or fusion) results in elevated expression levels of BIRC7 (e.g., compared to a reference without the MiT translocation).

In some embodiments of any of the methods of the invention, the MiT translocation (e.g., rearrangement and/or fusion) results in elevated activity and/or activation of BIRC7 (e.g., compared to a reference without the MiT translocation).

In some embodiments of any of the methods of the invention, the translocation is a somatic translocation.

In some embodiments of any of the methods of the invention, the translocation is an intra-chromosomal translocation.

In some embodiments of any of the methods of the invention, the translocation is an inter-chromosomal translocation.

In some embodiments of any of the methods of the invention, the translocation is an inversion

In some embodiments of any of the methods of the invention, the translocation is a deletion.

In some embodiments of any of the methods of the invention, the translocation is a translocation fusion polynucleotide (e.g., functional MiT-translocation fusion polynucleotide) and/or functional translocation fusion polypeptide (e.g., functional MiT-translocation fusion polypeptide).

In some embodiments of any of the methods of the invention, the sample is a cancer sample.

In some embodiments of any of the methods of the invention, the cancer is squamous cell cancer (e.g., epithelial squamous cell cancer), lung cancer including small-cell lung cancer (SCLC), non-small cell lung cancer (NSCLC), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer (including metastatic breast cancer), colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, tumors of the biliary tract, as well as head and neck cancer.

In some embodiments of any of the methods of the invention, the cancer is renal cell carcinoma (RCC). In some embodiments, the RCC is non-clear cell renal cell carcinoma (nccRCC) or translocation RCC (tRCC). In some embodiments, the RCC is nccRCC.

Described are methods of treating cancer in an individual comprising administering to the individual an effective amount of a MiT antagonist, wherein treatment is based upon the individual having cancer comprising MiT overexpression. In some embodiments, the cancer comprises a MiT translocation, the method comprising providing an effective amount of a MiT antagonist.

Described herein are methods of treating cancer in an individual provided that the individual has been found to have cancer comprising a MiT translocation, the method comprising administering to the individual an effective amount of a MiT antagonist.

Decribed herein are methods of treating cancer in an individual, the method comprising: determining that a sample obtained from the individual comprises a MiT translocation, and administering an effective amount of an anti-cancer therapy comprising a MiT antagonist to the individual, whereby the cancer is treated.

Described herein are methods of treating cancer, comprising: (a) selecting an individual having cancer, wherein the cancer comprising a MiT translocation; and (b) administering to the individual thus selected an effective amount of a MiT antagonist, whereby the cancer is treated.

Disclosed herein are methods of identifying an individual with cancer who is more or less likely to exhibit benefit from treatment with an anti-cancer therapy comprising a MiT antagonist, the method comprising: determining presence or absence of a MiT translocation in a sample obtained from the individual, wherein presence of the MiT translocation in the sample indicates that the individual is more likely to exhibit benefit from treatment with the anti-cancer therapy comprising the MiT antagonist or absence of the MiT translocation indicates that the individual is less likely to exhibit benefit from treatment with the anti-cancer therapy comprising the MiT antagonist.

Disclosed herein are methods of predicting whether an individual with cancer is more or less likely to respond effectively to treatment with an anti-cancer therapy comprising a MiT antagonist, the method comprising determining a MiT translocation, whereby presence of the MiT translocation indicates that the individual is more likely to respond effectively to treatment with the MiT antagonist and absence of the MiT translocation indicates that the individual is less likely to respond effectively to treatment with the MiT antagonist.

Disclosed herein are methods of predicting the response or lack of response of an individual with cancer to an anti-cancer therapy comprising a MiT antagonist comprising detecting in a sample obtained from the individual presence or absence of a MiT translocation, wherein presence of the MiT translocation is predictive of response of the individual to the anti-cancer therapy comprising the MiT antagonist and absence of the MiT translocation is predictive of lack of response of the individual to the anti-cancer therapy comprising the MiT antagonist.

In some aspects of the disclosure of any of the methods described, the methods further comprises administering to the individual an effective amount of a MiT antagonist.

Disclosed herein are methods of inhibiting proliferation of a nccRCC cancer cell comprising contacting the cancer cell with an effective amount of a MiT-translocation antagonist.

Disclosed herein are methods of treating nccRCC in an individual comprising administering to the individual an effective amount of a MiT antagonist. In some aspects of the disclosure, the MiT antagonist is a MiT-translocation antagonist.

In some aspects of the disclosure of any of the methods described, the cancer or cancer cell comprises MiT translocation.

In some aspects of the disclosure of any of the methods described, the cancer or cancer cell comprises MiT overexpression.

In some aspects of the disclosure of any of the methods described, the cancer or cancer cell comprises BIRC7 overexpression.

In some aspects of the disclosure of any of the methods described, MiT is MITF.

In some aspects of the disclosure of any of the methods described, MiT is TFEB.

In some aspects of the disclosure of any of the methods described, MiT is TFEC.

In some aspects of the disclosure of any of the methods described, MiT is TFE3.

In some aspects of the disclosure of any of the methods described, MiT is SBNO2.

In some aspects of the disclosure of any of the methods described, MiT translocation is a MITF translocation.

In some aspects of the disclosure of any of the methods described, MiT translocation is a TFEB translocation.

In some aspects of the disclosure of any of the methods described, MiT translocation is a TFEC translocation.

In some aspects of the disclosure of any of the methods described, MiT translocation is a TFE3 translocation.

In some aspects of the disclosure, the MiT translocation is detected using any of the methods of determining MiT translocation (detecting presence of MiT translocation) disclosed herein.

In some aspects of the disclosure of any of the methods described, the cancer or cancer is squamous cell cancer (e.g., epithelial squamous cell cancer), lung cancer including small-cell lung cancer (SCLC), non-small cell lung cancer (NSCLC), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer (including metastatic breast cancer), colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, tumors of the biliary tract, as well as head and neck cancer.

In some aspects of the disclosureof any of the methods of the invention, the cancer or cancer is renal cell carcinoma (RCC). In some aspects of the disclosure, the RCC is non-clear cell renal cell carcinoma (nccRCC) or translocation RCC (tRCC). In some aspects of the disclosure, the RCC is nccRCC.

In some aspects of the disclosure of any of the methods described, the MiT antagonist is an antibody, binding polypeptide, small molecule, or polynucleotide.

In some aspects of the disclosure of any of the methods described, the MiT antagonist is a MET antagonist.

In some aspects of the disclosure of any of the methods described, the MiT antagonist is a BIRC7 antagonist.

In some aspects of the disclosure of any of the methods described, the MiT antagonist is a MITF antagonist.

In some aspects of the disclosure of any of the methods decribed, the MiT antagonist is a TFEB antagonist.

In some aspects of the disclosure of any of the methods described, the MiT antagonist is a TFEC antagonist.

In some aspects of the disclosure of any of the methods decribed, the MiT antagonist is a TFE3 antagonist.

In some aspects of the disclosure of any of the methods described, the MiT antagonist binds MITF translocation. In some aspects of the disclosure, the MITF translocation comprises ACTG1 and MITF. In some aspects of the disclosure, MITF translocation comprises ACTG1 exon 3. In some aspects of the disclosure , MITF translocation comprises ACTG1 exon 3 and MITF exon 3. In some aspects of the disclosure, the MITF translocation comprises SEQ ID NO:13 and/or 30. In some aspects of the disclosure, the MITF translocation comprises SEQ ID NO: 30. In some aspects of the disclosure, the MITF translocation is driven by the ACTG1 promoter. In some embodiments, the MITF translocation comprises AP3S1 and MITF. In some aspects of the disclosure, MITF translocation comprises AP3S1 exon 3. In some aspects of the disclosure, the MITF translocation comprises ACTG1 exon 3 and MITF exon 3. In some aspects of the disclosure, the MITF translocation is driven by the AP3S1 promoter. In some aspects of the disclosure, MiT antagonist binds TFEB translocation. In some aspects of the disclosure, the TFEB translocation comprises CLTC and TFEB. In some aspects of the disclosure, the TFEB translocation comprises CLTC exon 17. In some aspects of the disclosure, the TFEB translocation comprises CLTC exon 17and TFEB exon 6. In some aspects of the disclosure, TFEB translocation comprises SEQ ID NO:19. In some aspects of the disclosure, TFEB translocation is driven by the CLTC promoter. In some aspects of the disclosure, the MiT antagonist binds a SBNO2 translocation. In some aspects of the disclosure, the SBNO2 translocation is an inversion. In some aspects of the disclosure, the SBNO2 inversion comprises MIDN and SBNO2. In some aspects of the disclosure, SBNO2 inversion comprises MIDN promoter. In some aspects of the disclosure, the SBNO2 inversion comprises MIDN promoter and SBNO2 exon 1. In some aspects of the disclosure, the SBNO2 inversion comprises SEQ ID NO:25. In some aspects of the disclosure, the SBNO2 inversion is driven by the CLTC promoter.

In some aspects of the disclosure of any of the methods described, the methods further comprises administering an additional therapeutic agent.

### BRIEF DESCRIPTION OF THE FIGURES

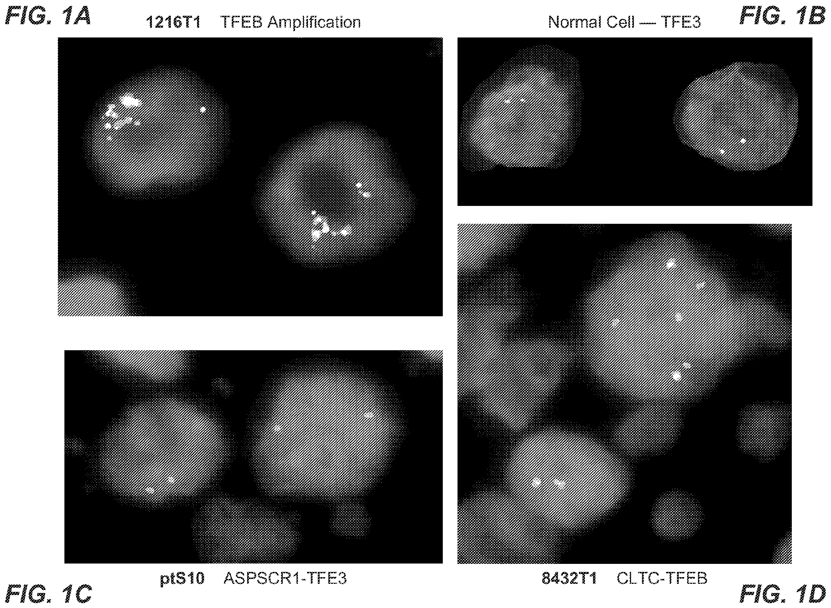

**Figures 1a, b, c, d****.** FISH (fluorescence in situ hybridization) image showing (**a**) TFEB amplification in tumor cells, (**b**) TFE3 in a normal cells, (**c-d**) TFE3 (c) and TFEB (d) gene rearrangement in tumor cells. DNA probe set (Agilent Technologies, CA) that hybridized to 5' or 3' side of TFE3 gene in chromosome band Xp11.2 or TFEB gene in chromosome band 6p21 were used. TFE3 probes used: TFE3 5' Xp11 labeled red and TFE3 3' Xp11 labeled green. TFEB probes used: TFEB 5' 6p21.1 labeled red and TFEB 3' 6p21.1 labeled green. An intact copy of the gene produced an yellow (Y) signal from the merge of red (R) and green (G) labels (panel b). In panel c, consistent with TFE3 translocation (fusion) we observed patterns involving separate red and green signals (seen in more than 80% of the cells) in a male patient. Similarly in panel d, consistent with TFEB translocation we observe patterns involving clearly separate red and green signals. In panel a, 90% of the cells showed polysomy of the TFEB gene and ∼25% showed extra copy of the 3'TFEB signal consistent with the amplification detected on the SNP array.

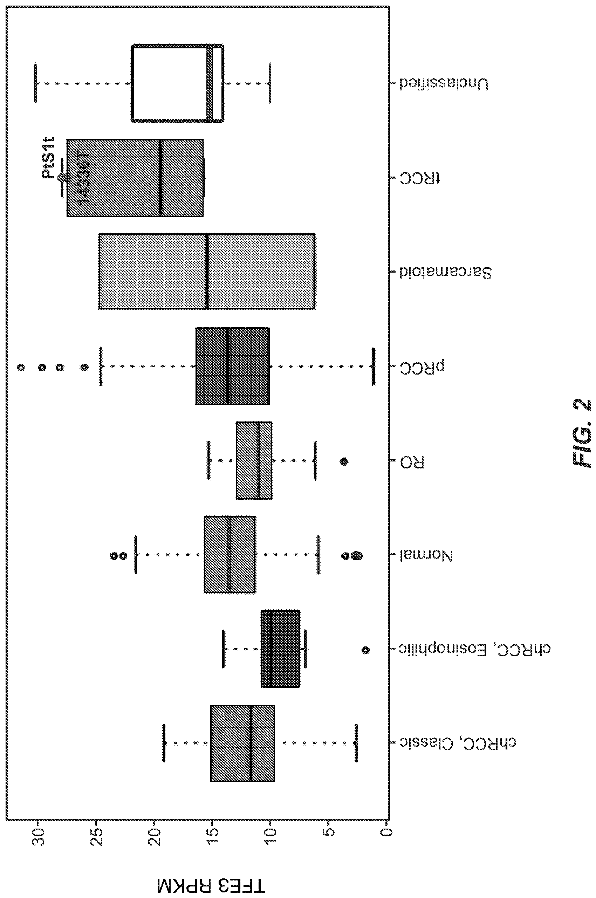

**Figure 2****.** Boxplot of *TFE3* expression in the indicated nccRCC subtypes.

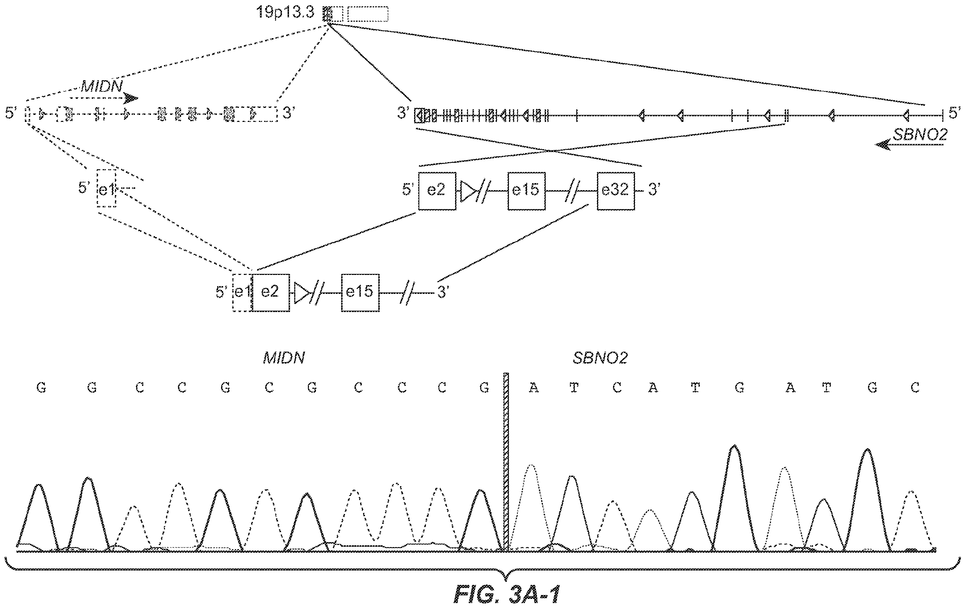

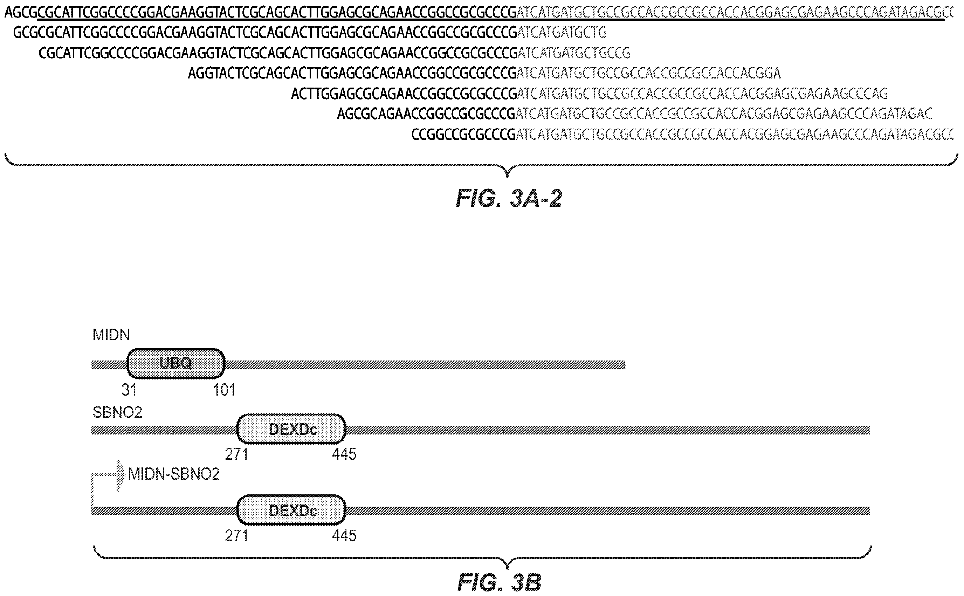

**Figures 3a****,** **b****.** *MIDN-SBNO2* gene fusion (**a**) Cartoon depicting the location, orientation and exon-intron architecture of *MIDN-SBNO2* fusion on the genome. The read evidence for *MIDN(e1)-SBNO2(e2)* fusion identified using RNA-seq data are shown. Representative Sanger sequencing chromatogram of the RT-PCR derived products confirming the *MIDN(e1)-SBNO2*(e2) fusion junction, (**b**) Schematic of the resulting MIDN-SBNO2 fusion protein.

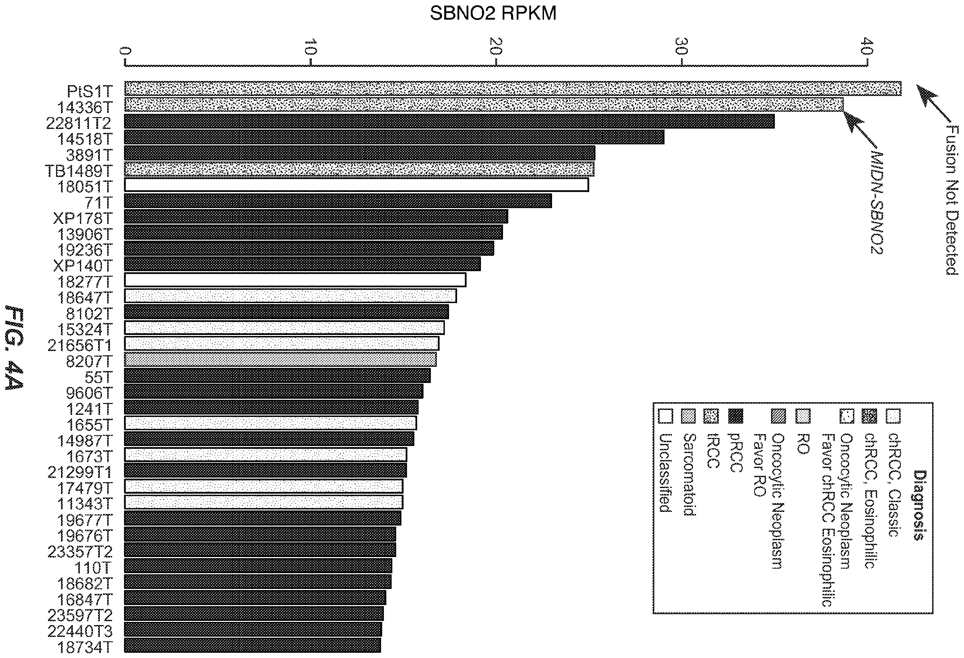

**Figure 4a****-d.** Barplot of *SBNO2* expression in tumors as measured by RNA-seq.

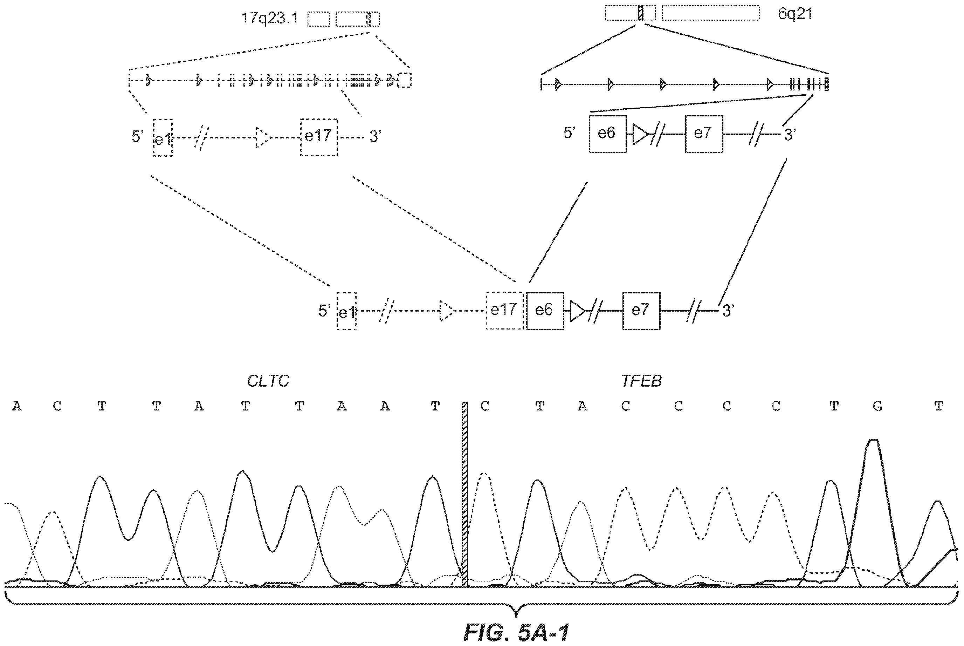

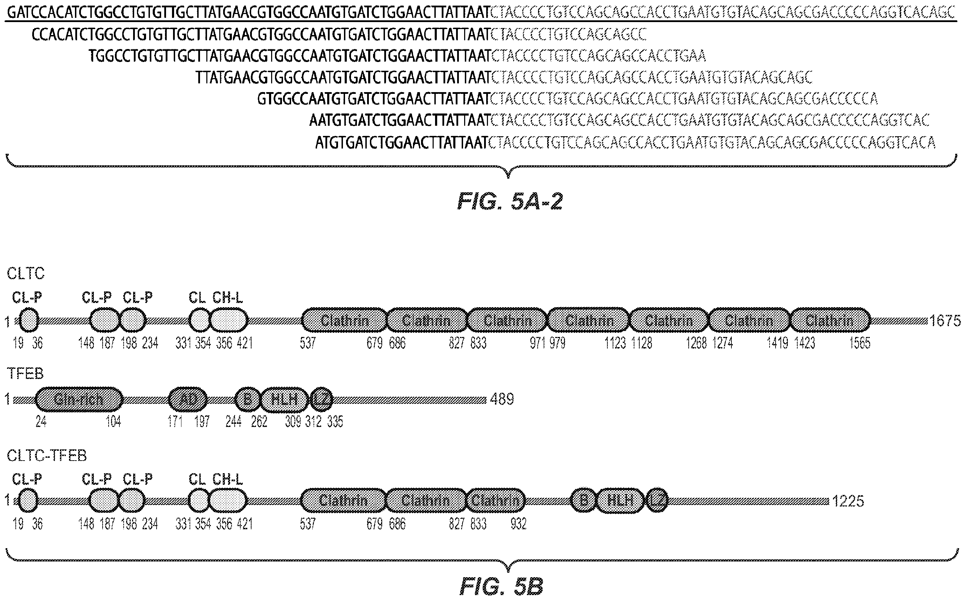

**Figures 5a****,** **b****. *CLTC-TFEB* gene fusion.** (**a**) Cartoon depicting the location, orientation and exon-intron architecture of *CLTC-TFEB* fusion on the genome. The read evidence for *CLTC(e17)-TFEB(e6)* fusion identified using RNA-seq data are shown. Representative Sanger sequencing chromatogram of the RT-PCR derived products confirming the *CLTC(e17)-TFEB(e6)* fusion junction, (**b**) Schematic of the resulting CLTC-TFEB fusion protein. CL-P - clathrin_propel; CL- clathrin-link; CH-L - clathrin_H_link; Gln-rich - glycine rich; AD - activation domain; B - basic; HLH- helix-loop-helix & LZ- leucine zipper.

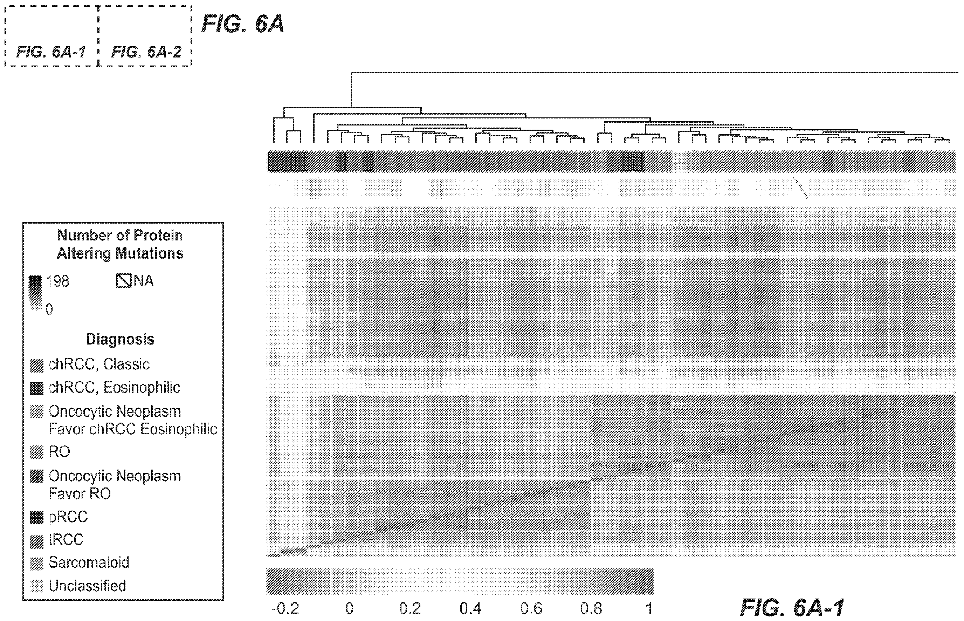

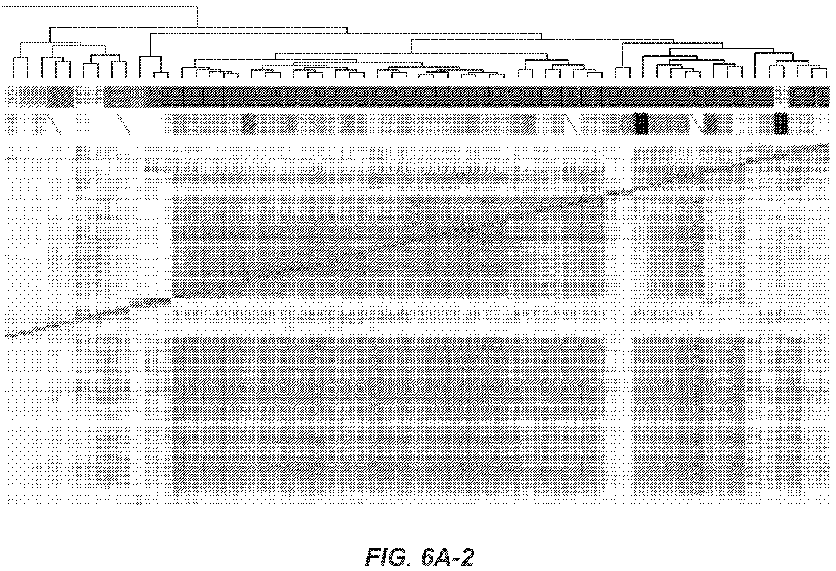

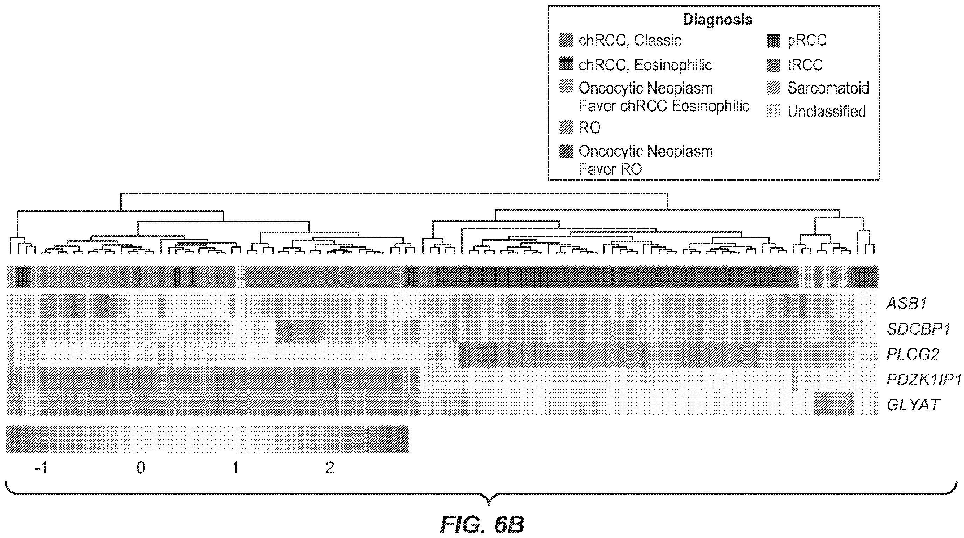

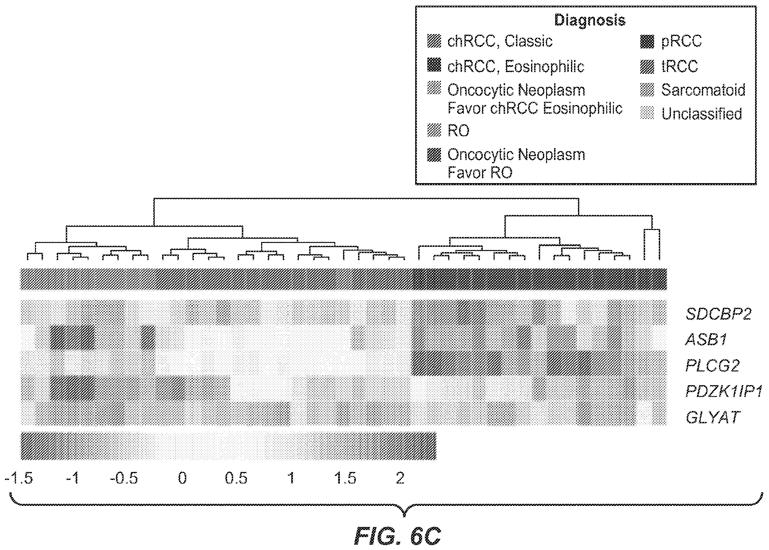

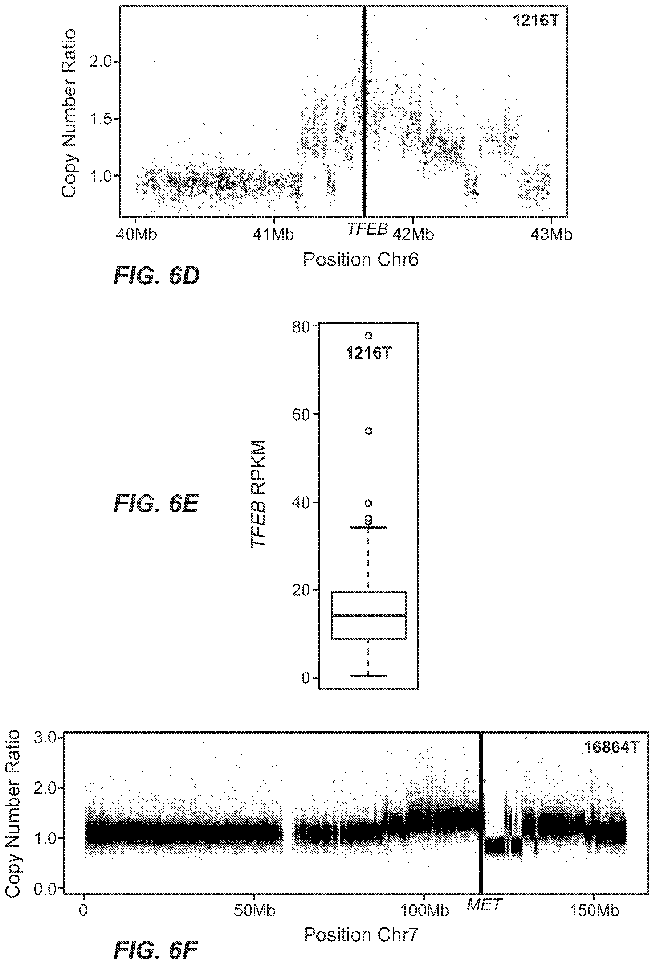

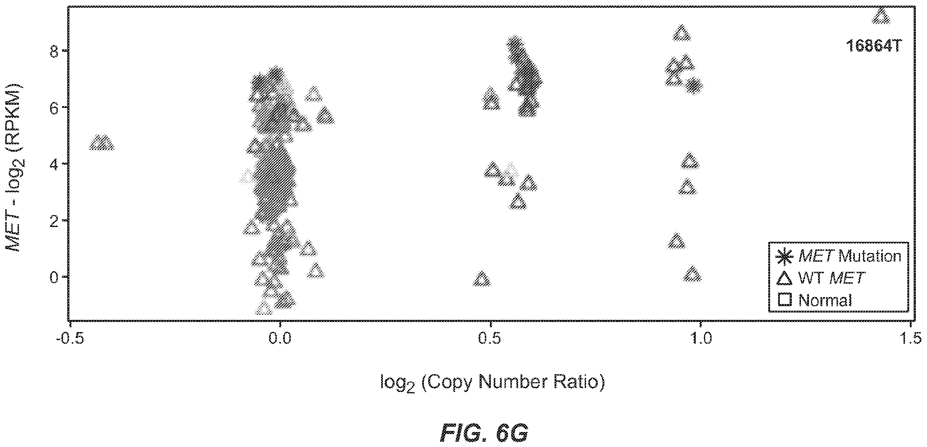

**Figures 6a****-g. RNA-seq based classification of nccRCC.** (**a**) Copy number ratio plot depicting a focal *TFEB* amplification in sample 1216T (b) Boxplot of *TFEB* expression in tumors show a high level of *TFEB* expression in sample 1216T.

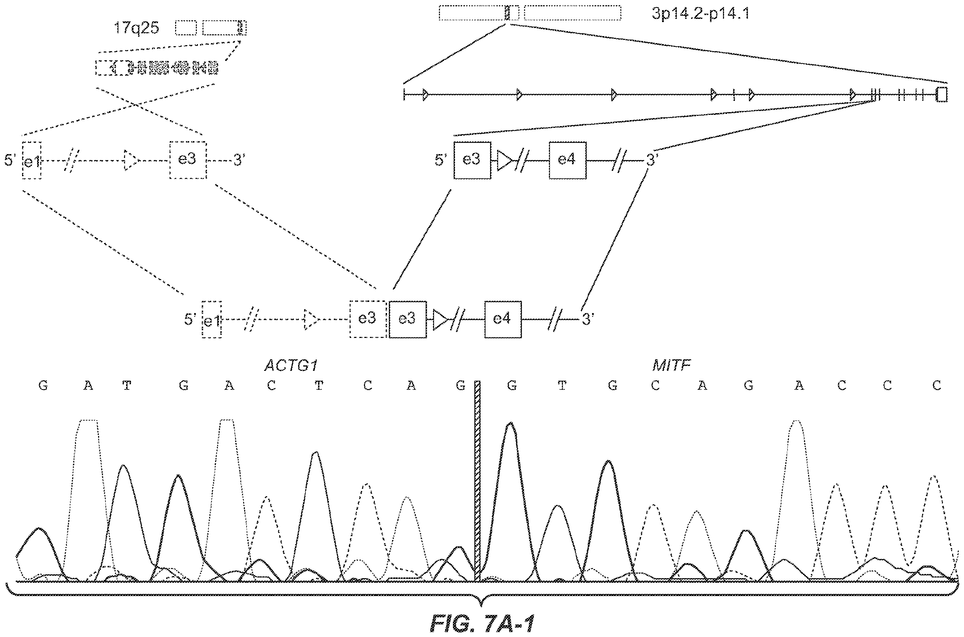

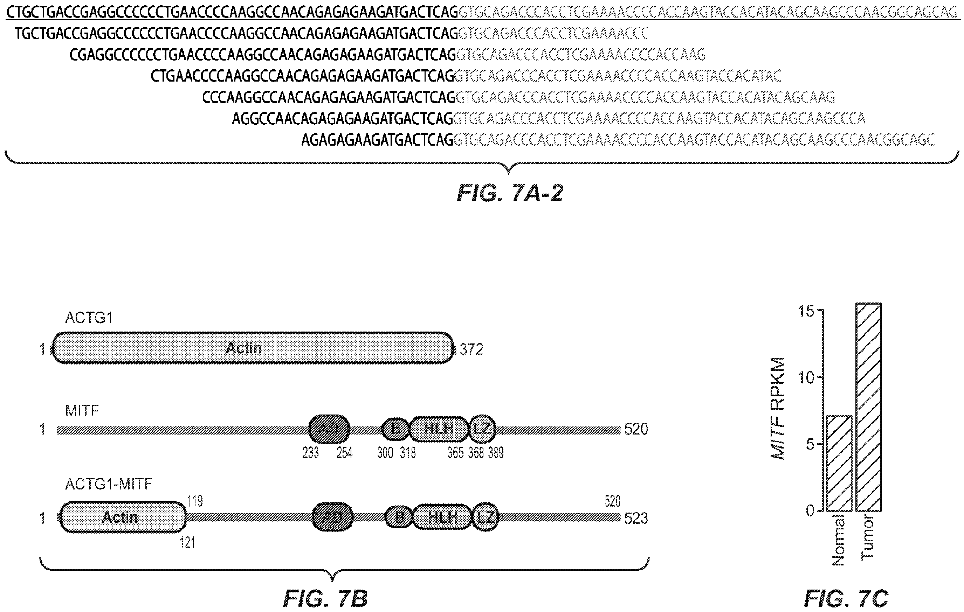

**Figures 7a****,** **b, c****. MITF gene fusion.** (**a**) Cartoon depicting the location, orientation, exon-intron architecture of ACTG1-*MITF* fusion on the genome, the read evidence for *ACTG1(e3)-MITF(e3)* fusion identified using RNA-seq data and a representative Sanger sequencing chromatogram of RT-PCR derived product confirming the *ACTG1(e3)-MITF(e3)* fusion junction are shown (**b**) Schematic of the ACTG1-MITF fusion protein (**c**) *MITF* expression in tumor harboring the MITF fusion AD - activation domain; B - basic; HLH- helix-loop-helix & LZ- leucine zipper.

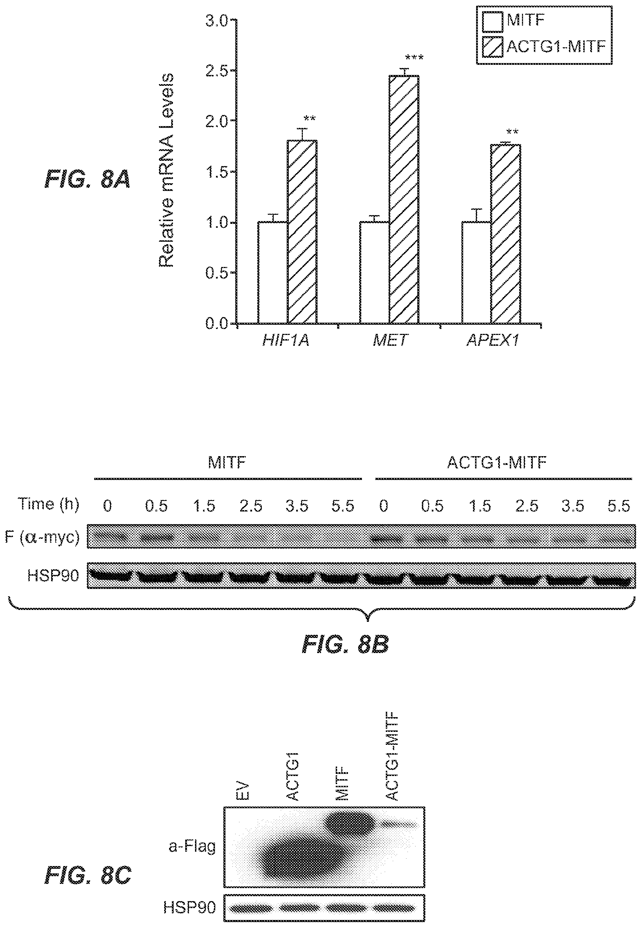

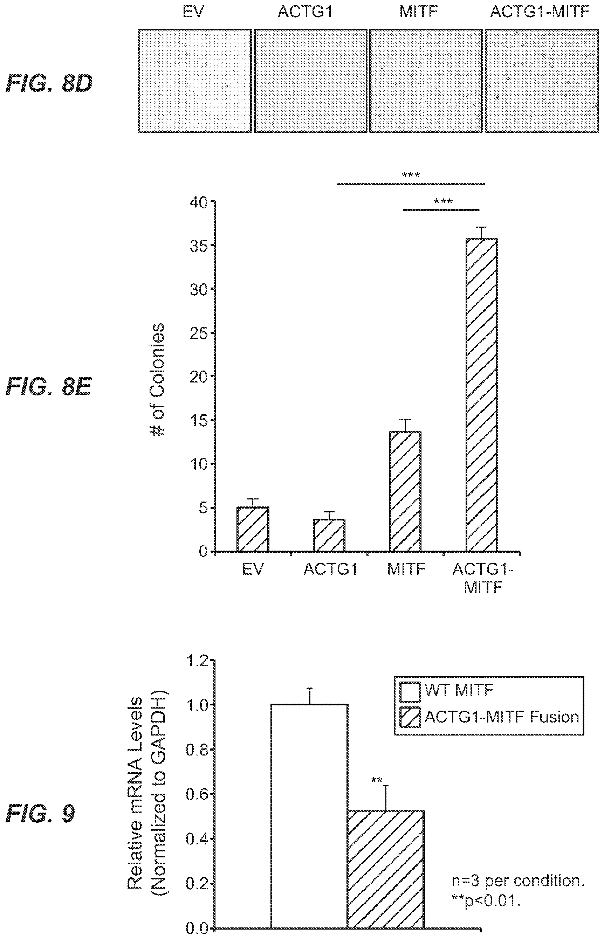

**Figures 8a, b, c****,** **d, e****. ACTG1-MITF gene fusion promotes anchorage independent growth.** (**a**) Expression of MITF target genes in HEK293T cells transfected with MITF WT or fusion constructs. The values shown are from three replicates. (error bar represents SEM; **p<0.01; ***p<0.001). (**b**) Stability of MITF fusion protein overtime in HEK293T cells transfected with indicated constructs following cycloheximide treatment, assessed using Western blot. (**c**) Western blot showing the expression of Flag-tagged ACTG1, MITF and ACTG1-MITF fusion proteins in NIH3T3 cell expressing the indicated constructs. Hsp90 was used as a loading control, (**d**) Representative images depicting the colony formation by NIH3T3 cells stably expressing the indicated constructs (EV = Empty Vector). (**e**) Quantification of the number of colonies (>300uM diameter) shown in panel (d). Data shown are mean ± SEM (n=3, ***p<0.001).
**Figure 9****.** Expression levels of WT MITF and ACTG1-MITF in transiently transfected 293T cells. This was used to normalize the expression of target genes show in Fig 8a.

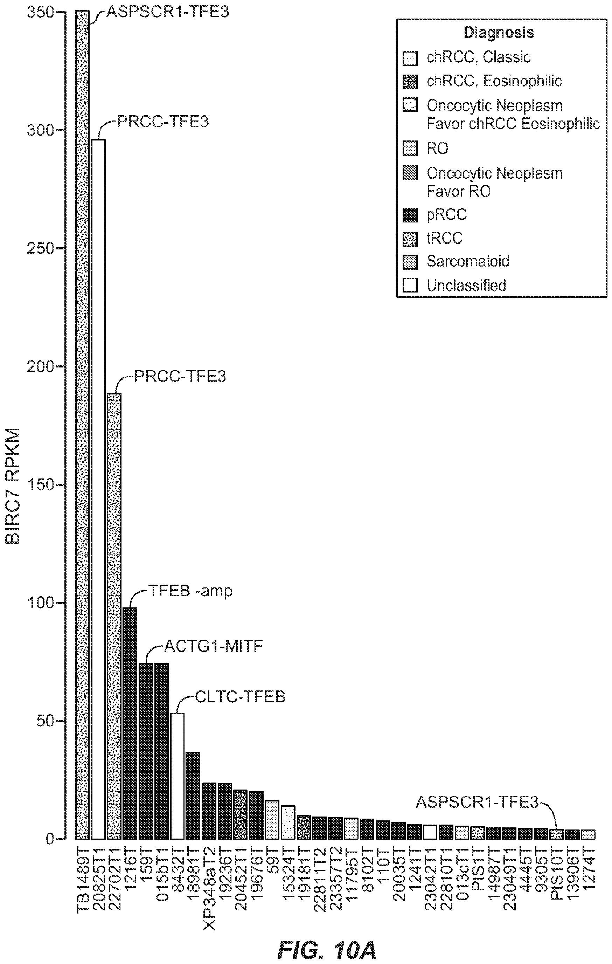

**Figure 10a****-b.** Expression of *BIRC7* in tumors with MITF/TFE translocation or fusion compared to samples without a translocation. Expression of *BIRC7* was significant in samples with MITF/TFE event (t-test p-value 0.002308467).

### DETAILED DESCRIPTION

### I. Definitions

The term "MITF" refers herein to a native MITF from any vertebrate source, including mammals such as primates (*e.g.,* humans) and rodents (*e.g.,* mice and rats), unless otherwise indicated. The term encompasses "full-length," unprocessed MITF as well as any form of MITF that results from processing in the cell. The term also encompasses naturally occurring variants of MITF, *e*.*g*., splice variants or allelic variants. The sequence of an exemplary human MITF nucleic acid sequence is SEQ ID NO:2.

"MITF variant" or variations thereof, means a MITF polypeptide or polynucleotide, generally being or encoding an active MITF polypeptide, as defined herein having at least about 80% amino acid sequence identity with any of the MITF as disclosed herein. Such MITF variants include, for instance, MITF wherein one or more nucleic acid or amino acid residues are added or deleted. Ordinarily, an MITF variant will have at least about 80% sequence identity, alternatively at least about 81%, 82%, 83%, 84%, 85%, 86%, 87%, 88%, 89%, 90%, 91%, 92%, 93%, 94%, 95%, 96%, 97%, 98%, or 99% sequence identity, to MITF as disclosed herein. Ordinarily, MITF variant are at least about 10 residues in length, alternatively at least about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 in length, or more. Optionally, MITF variant will have or encode a sequence having no more than one conservative amino acid substitution as compared to MITF, alternatively no more than 2, 3, 4, 5, 6, 7, 8, 9, or 10 conservative amino acid substitutions as compared to MITF.

The term "TFEB" refers herein to a native TFEB from any vertebrate source, including mammals such as primates (*e.g.,* humans) and rodents (*e.g.,* mice and rats), unless otherwise indicated. The term encompasses "full-length," unprocessed TFEB as well as any form of TFEB that results from processing in the cell. The term also encompasses naturally occurring variants of TFEB, *e*.*g*., splice variants or allelic variants. The sequence of an exemplary human TFEB nucleic acid sequence is SEQ ID NO:4.

"TFEB variant" or variations thereof, means a TFEB polypeptide or polynucleotide, generally being or encoding an active TFEB polypeptide, as defined herein having at least about 80% amino acid sequence identity with any of the TFEB as disclosed herein. Such TFEB variants include, for instance, TFEB wherein one or more nucleic acid or amino acid residues are added or deleted. Ordinarily, an TFEB variant will have at least about 80% sequence identity, alternatively at least about 81%, 82%, 83%, 84%, 85%, 86%, 87%, 88%, 89%, 90%, 91%, 92%, 93%, 94%, 95%, 96%, 97%, 98%, or 99% sequence identity, to TFEB as disclosed herein. Ordinarily, TFEB variant are at least about 10 residues in length, alternatively at least about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 in length, or more. Optionally, TFEB variant will have or encode a sequence having no more than one conservative amino acid substitution as compared to TFEB, alternatively no more than 2, 3, 4, 5, 6, 7, 8, 9, or 10 conservative amino acid substitutions as compared to TFEB.

The term "TFE3" refers herein to a native TFE3 from any vertebrate source, including mammals such as primates (*e.g.,* humans) and rodents (*e.g.,* mice and rats), unless otherwise indicated. The term encompasses "full-length," unprocessed TFE3 as well as any form of TFE3 that results from processing in the cell. The term also encompasses naturally occurring variants of TFE3, *e.g.,* splice variants or allelic variants. The sequence of an exemplary human TFE3 nucleic acid sequence is SEQ ID NO:6.

"TFE3 variant" or variations thereof, means a TFE3 polypeptide or polynucleotide, generally being or encoding an active TFE3 polypeptide, as defined herein having at least about 80% amino acid sequence identity with any of the TFE3 as disclosed herein. Such TFE3 variants include, for instance, TFE3 wherein one or more nucleic acid or amino acid residues are added or deleted. Ordinarily, an TFE3 variant will have at least about 80% sequence identity, alternatively at least about 81%, 82%, 83%, 84%, 85%, 86%, 87%, 88%, 89%, 90%, 91%, 92%, 93%, 94%, 95%, 96%, 97%, 98%, or 99% sequence identity, to TFE3 as disclosed herein. Ordinarily, TFE3 variant are at least about 10 residues in length, alternatively at least about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 in length, or more. Optionally, TFE3 variant will have or encode a sequence having no more than one conservative amino acid substitution as compared to TFE3, alternatively no more than 2, 3, 4, 5, 6, 7, 8, 9, or 10 conservative amino acid substitutions as compared to TFE3.

The term "TFEC" refers herein to a native TFEC from any vertebrate source, including mammals such as primates (*e.g.,* humans) and rodents (*e.g.,* mice and rats), unless otherwise indicated. The term encompasses "full-length," unprocessed TFEC as well as any form of TFEC that results from processing in the cell. The term also encompasses naturally occurring variants of TFEC, *e.g.,* splice variants or allelic variants. The sequence of an exemplary human TFEC nucleic acid sequence is SEQ ID NO:8.

"TFEC variant" or variations thereof, means a TFEC polypeptide or polynucleotide, generally being or encoding an active TFEC polypeptide, as defined herein having at least about 80% amino acid sequence identity with any of the TFEC as disclosed herein. Such TFEC variants include, for instance, TFEC wherein one or more nucleic acid or amino acid residues are added or deleted. Ordinarily, an TFEC variant will have at least about 80% sequence identity, alternatively at least about 81%, 82%, 83%, 84%, 85%, 86%, 87%, 88%, 89%, 90%, 91%, 92%, 93%, 94%, 95%, 96%, 97%, 98%, or 99% sequence identity, to TFEC as disclosed herein. Ordinarily, TFEC variant are at least about 10 residues in length, alternatively at least about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 in length, or more. Optionally, TFEC variant will have or encode a sequence having no more than one conservative amino acid substitution as compared to TFEC, alternatively no more than 2, 3, 4, 5, 6, 7, 8, 9, or 10 conservative amino acid substitutions as compared to TFEC.

The term "MiT" refers to the proteins MITF, TFEB, TFE3, TFEC, and SBNO2.

The term "MiT translocation" refers herein to a MiT wherein a portion of a broken chromosome including, for example, polynucleotide encoding MiT, variant, or fragment thereof or a second gene, variant, or fragment thereof, reattaches in a different chromosome location, for example, a chromosome location different from MiT native location or a chromosome location in and/or around the MiT native location which is different from the second gene's native location. The MiT translocation may be a MITF translocation, TFEB translocation, TFE3 translocation, TFEC translocation and/or SBNO2 translocation.

The term "MITF translocation" refers herein to a MITF wherein a portion of a broken chromosome including, for example, polynucleotide encoding MITF, variant, or fragment thereof or a second gene, variant, or fragment thereof, reattaches in a different chromosome location, for example, a chromosome location different from MITF native location or a chromosome location in and/or around the MITF native location which is different from the second gene's native location.

The term "TFEB translocation" refers herein to a TFEB wherein a portion of a broken chromosome including, for example, polynucleotide encoding TFEB, variant, or fragment thereof or a second gene, variant, or fragment thereof, reattaches in a different chromosome location, for example, a chromosome location different from TFEB native location or a chromosome location in and/or around the TFEB native location which is different from the second gene's native location.

The term "TFE3 translocation" refers herein to a TFE3 wherein a portion of a broken chromosome including, for example, polynucleotide encoding TFE3, variant, or fragment thereof or a second gene, variant, or fragment thereof, reattaches in a different chromosome location, for example, a chromosome location different from TFE3 native location or a chromosome location in and/or around the TFE3 native location which is different from the second gene's native location.

The term "TFEC translocation" refers herein to a TFEC wherein a portion of a broken chromosome including, for example, polynucleotide encoding TFEC, variant, or fragment thereof or a second gene, variant, or fragment thereof, reattaches in a different chromosome location, for example, a chromosome location different from TFEC native location or a chromosome location in and/or around the TFEC native location which is different from the second gene's native location.

The term "SBNO2 translocation" refers herein to a SBNO2 wherein a portion of a broken chromosome including, for example, polynucleotide encoding SBNO2, variant, or fragment thereof or a second gene, variant, or fragment thereof, reattaches in a different chromosome location, for example, a chromosome location different from SBNO2 native location or a chromosome location in and/or around the SBNO2 native location which is different from the second gene's native location.

The term "MiT-translocation fusion polynucleotide" refers herein to the nucleic acid sequence of a MiT translocation gene product or fusion polynucleotide. The MiT -translocation fusion polynucleotide may be a MITF-translocation fusion polynucleotide, TFEB-translocation fusion polynucleotide, TFE3-translocation fusion polynucleotide, TFEC-translocation fusion polynucleotide and/or SBNO2-translocation fusion polynucleotide. The term " MiT-translocation fusion polypeptide" refers herein to the amino acid sequence of a MiT translocation gene product or fusion polynucleotide. The MiT-translocation fusion polypeptide may be a MITF-translocation fusion polypeptide, TFEB-translocation fusion polypeptide, TFE3-translocation fusion polypeptide, TFEC-translocation fusion polypeptide and/or SBNO2-translocation fusion polypeptide.

The term "MiT-translocation antagonist" as defined herein is any molecule that partially or fully blocks, inhibits, or neutralizes a biological activity mediated by a MiT-translocation fusion polypeptide. In some aspects of the disclosure such antagonist binds to MiT-translocation fusion polypeptide. According to one aspect of the disclosure, the antagonist is a polypeptide. According to another aspect of the disclosure, the antagonist is a small molecule antagonist. According to another aspect of the disclosure , the antagonist is a polynucleotide antagonist. The MiT translocation may be a MITF-translocation antagonist, TFEB-translocation antagonist, TFE3-translocation antagonist, TFEC-translocation antagonist and/or SBNO2-translocation antagonist.

The term "MITF-translocation antagonist" as defined herein is any molecule that partially or fully blocks, inhibits, or neutralizes a biological activity mediated by a MITF - translocation fusion polypeptide. In some aspects of the disclosure such antagonist binds to MITF - translocation fusion polypeptide. According to one aspect of the disclosure, the antagonist is a polypeptide. According to another aspect of the disclosure, the antagonist is a small molecule antagonist. According to another aspect of the disclosure, the antagonist is a polynucleotide antagonist.

The term "TFEB-translocation antagonist" as defined herein is any molecule that partially or fully blocks, inhibits, or neutralizes a biological activity mediated by a TFEB-translocation fusion polypeptide. In some aspects of the disclosure such antagonist binds to TFEB-translocation fusion polypeptide. According to one aspect of the disclosure, the antagonist is a polypeptide. According to another aspect of the disclosure, the antagonist is a small molecule antagonist. According to another aspect of the disclosure, the antagonist is a polynucleotide antagonist.

The term "TFE3-translocation antagonist" as defined herein is any molecule that partially or fully blocks, inhibits, or neutralizes a biological activity mediated by a TFE3-translocation fusion polypeptide. In some aspects of the disclosure such antagonist binds to TFE3-translocation fusion polypeptide. According to one aspect of the disclosure, the antagonist is a polypeptide. According to another aspect of the disclosure, the antagonist is a small molecule antagonist. According to another aspect of the disclosure, the antagonist is a polynucleotide antagonist.

The term "TFEC-translocation antagonist" as defined herein is any molecule that partially or fully blocks, inhibits, or neutralizes a biological activity mediated by a TFEC-translocation fusion polypeptide. In some aspects of the disclosure such antagonist binds to TFEC-translocation fusion polypeptide. According to one aspect of the disclosure, the antagonist is a polypeptide. According to another aspect of the disclosure, the antagonist is a small molecule antagonist. According to another aspect of the disclosure, the antagonist is a polynucleotide antagonist.

The term "SBNO2-translocation antagonist" as defined herein is any molecule that partially or fully blocks, inhibits, or neutralizes a biological activity mediated by a SBNO2-translocation fusion polypeptide. In some aspects of the disclosure such antagonist binds to SBNO2-translocation fusion polypeptide. According to one aspect of the disclosure, the antagonist is a polypeptide. According to another aspect of the disclosure, the antagonist is a small molecule antagonist. According to another aspect of the disclosure, the antagonist is a polynucleotide antagonist.

The term "MET pathway antagonist" as defined herein is any molecule that partially or fully blocks, inhibits, or neutralizes a biological activity mediated by the MET pathway. In some aspects of the disclosure such antagonist binds to a MET pathway polypeptide. According to one aspect of the disclosure, the antagonist is a polypeptide. According to another aspect of the disclosure, the antagonist is an antibody antagonist. According to another aspect of the disclosure, the antagonist is a small molecule antagonist. According to another aspect of the disclosure, the antagonist is a polynucleotide antagonist.

The term "BIRC7 pathway antagonist" as defined herein is any molecule that partially or fully blocks, inhibits, or neutralizes a biological activity mediated by the BIRC7 pathway. In some aspects of the disclosure such antagonist binds to a BIRC7 pathway polypeptide. According to one aspect of the disclosure, the antagonist is a polypeptide. According to another aspect of the disclosure, the antagonist is an antibody antagonist. According to another aspect of the disclosure, the antagonist is a small molecule antagonist. According to another aspect of the disclosure, the antagonist is a polynucleotide antagonist.

"Polynucleotide" or "nucleic acid" as used interchangeably herein, refers to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. A sequence of nucleotides may be interrupted by non-nucleotide components. A polynucleotide may comprise modification(s) made after synthesis, such as conjugation to a label. Other types of modifications include, for example, "caps," substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, aspects of the disclosure wherein phosphate is replaced by P(O)S ("thioate"), P(S)S ("dithioate"), (O)NR₂ ("amidate"), P(O)R, P(O)OR', CO, or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, refers to generally single-stranded, synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

The term "primer" refers to a single stranded polynucleotide that is capable of hybridizing to a nucleic acid and following polymerization of a complementary nucleic acid, generally by providing a free 3'-OH group.

The term "small molecule" refers to any molecule with a molecular weight of about 2000 Daltons or less, preferably of about 500 Daltons or less.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

An "isolated" antibody is one which has been separated from a component of its natural environment. In some aspects of the disclosure, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, *see, e.g.,* Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g., scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The terms "full length antibody," "intact antibody," and "whole antibody" are used herein interchangeably to refer to an antibody having a structure substantially similar to a native antibody structure or having heavy chains that contain an Fc region as defined herein.

The term "monoclonal antibody" as used herein refers to an antibody obtained from a population of substantially homogeneous antibodies, *i.e.,* the individual antibodies comprising the population are identical and/or bind the same epitope, except for possible variant antibodies, *e*.*g*., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

"Native antibodies" refer to naturally occurring immunoglobulin molecules with varying structures. For example, native IgG antibodies are heterotetrameric glycoproteins of about 150,000 Daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CHI, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "chimeric" antibody refers to an antibody in which a portion of the heavy and/or light chain is derived from a particular source or species, while the remainder of the heavy and/or light chain is derived from a different source or species.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "humanized" antibody refers to a chimeric antibody comprising amino acid residues from non-human HVRs and amino acid residues from human FRs. In certain aspects of the disclosure, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, *e*.*g*., a non-human antibody, refers to an antibody that has undergone humanization.

The "class" of an antibody refers to the type of constant domain or constant region possessed by its heavy chain. There are five major classes of antibodies: IgA, IgD, IgE, IgG, and IgM, and several of these may be further divided into subclasses (isotypes), *e*.*g*., IgG₁, IgG₂, IgG₃, IgG₄, IgA₁, and IgA₂. The heavy chain constant domains that correspond to the different classes of immunoglobulins are called α, δ, ε, γ, and µ, respectively.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g., B cell receptor); and B cell activation.

The term "Fc region" herein is used to define a C-terminal region of an immunoglobulin heavy chain that contains at least a portion of the constant region. The term includes native sequence Fc regions and variant Fc regions. In one aspect of the disclosure, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one aspect of the disclosure, for the VL, the subgroup is subgroup kappa I as in Kabat *et al., supra.* In one aspect of the disclosure, for the VH, the subgroup is subgroup III as in Kabat *et al., supra.*

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some aspects of the disclosure, the number of amino acid changes are 10 or less, 9 or less, 8 or less, 7 or less, 6 or less, 5 or less, 4 or less, 3 or less, or 2 or less. In some aspects of the disclosure, the VL acceptor human framework is identical in sequence to the VL human immunoglobulin framework sequence or human consensus framework sequence.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (*See, e.g.,* Kindt et al., Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. *See, e.g.,* Portolano et al., J. Immunol. 150:880-887 (1993); Clarkson et al., Nature 352:624-628 (1991).

The term "hypervariable region" or "HVR," as used herein, refers to each of the regions of an antibody variable domain which are hypervariable in sequence and/or form structurally defined loops ("hypervariable loops"). Generally, native four-chain antibodies comprise six HVRs; three in the VH (HI, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (HI), 53-55 (H2), and 96-101 (H3). (Chothia and Lesk, J. Mol. Biol. 196:901-917 (1987).) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (*See* Almagro and Fransson, Front. Biosci. 13:1619-1633 (2008).) Unless otherwise indicated, HVR residues and other residues in the variable domain (e.g., FR residues) are numbered herein according to Kabat *et al., supra.*

"Affinity" refers to the strength of the sum total of noncovalent interactions between a single binding site of a molecule (*e.g.,* an antibody) and its binding partner (*e.g.,* an antigen). Unless indicated otherwise, as used herein, "binding affinity" refers to intrinsic binding affinity which reflects a 1:1 interaction between members of a binding pair (*e.g*., antibody and antigen). The affinity of a molecule X for its partner Y can generally be represented by the dissociation constant (Kd). Affinity can be measured by common methods known in the art, including those described herein. Specific illustrative and exemplary aspects of the disclosure for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The terms "anti-MiT antibody" and "an antibody that binds to MiT" refer to an antibody that is capable of binding MiT polypeptide with sufficient affinity such that the antibody is useful as a diagnostic and/or therapeutic agent in targeting MiT. In one aspect of the disclosure, the extent of binding of an anti-MiT antibody to an unrelated, non-MiT polypeptide is less than about 10% of the binding of the antibody to MiT-translocation fusion polypeptides measured, *e*.*g*., by a radioimmunoassay (RIA). In certain aspects of the disclosure, an antibody that binds to MiT has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (*e.g.,* 10⁻⁸ M or less, *e.g.,* from 10⁻⁸ M to 10⁻¹³ M, *e.g.,* from 10⁻⁹ M to 10⁻¹³ M). MiT may be a MITF translocation, TFEB translocation, TFE3 translocation, TFEC translocation and/or SBNO2 translocation.

The terms "anti-MiT-translocation antibody" and "an antibody that binds to MiT - translocation fusion polypeptide" refer to an antibody that is capable of binding MiT -translocation fusion polypeptide with sufficient affinity such that the antibody is useful as a diagnostic and/or therapeutic agent in targeting MiT translocation. In one aspect of the disclosure, the extent of binding of an anti-MiT translocation antibody to an unrelated, non-MiT-translocation fusion polypeptide, and/or nontranslocated-MiT polypeptide is less than about 10% of the binding of the antibody to R-spondin-translocation fusion polypeptides measured, *e*.*g*., by a radioimmunoassay (RIA). In certain aspects of the disclosure, an antibody that binds to MiT-translocation fusion polypeptide has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (*e.g.,* 10⁻⁸ M or less, *e.g.,* from 10⁻⁸ M to 10⁻¹³ M, *e.g.,* from 10⁻⁹ M to 10⁻¹³ M). In certain aspects of the disclosure, an anti- MiT translocation antibody binds to an epitope of MiT translocation that is unique among MiT translocations. MiT translocation may be a MITF translocation, TFEB translocation, TFE3 translocation, TFEC translocation and/or SBNO2 translocation.

A "blocking" antibody or an "antagonist" antibody is one which inhibits or reduces biological activity of the antigen it binds. Preferred blocking antibodies or antagonist antibodies substantially or completely inhibit the biological activity of the antigen.

A "naked antibody" refers to an antibody that is not conjugated to a heterologous moiety (e.g., a cytotoxic moiety) or radiolabel. The naked antibody may be present in a pharmaceutical formulation.

An "immunoconjugate" is an antibody conjugated to one or more heterologous molecule(s), including but not limited to a cytotoxic agent.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "detection" includes any means of detecting, including direct and indirect detection.

The term "biomarker" as used herein refers to an indicator, *e*.*g*., predictive, diagnostic, and/or prognostic, which can be detected in a sample. The biomarker may serve as an indicator of a particular subtype of a disease or disorder (e.g., cancer) characterized by certain, molecular, pathological, histological, and/or clinical features. In some aspects of the disclosure, the biomarker is a gene. In some aspects of the disclosure, the biomarker is a variation (e.g., mutation and/or polymorphism) of a gene. In some embodiments, the biomarker is a translocation. Biomarkers include, but are not limited to, polynucleotides (e.g., DNA, and/or RNA), polypeptides, polypeptide and polynucleotide modifications (e.g., posttranslational modifications), carbohydrates, and/or glycolipid-based molecular markers.

The "presence," "amount," or "level" of a biomarker associated with an increased clinical benefit to an individual is a detectable level in a biological sample. These can be measured by methods known to one skilled in the art and also disclosed herein. The expression level or amount of biomarker assessed can be used to determine the response to the treatment.

The terms "level of expression" or "expression level" in general are used interchangeably and generally refer to the amount of a biomarker in a biological sample. "Expression" generally refers to the process by which information (e.g., gene-encoded and/or epigenetic) is converted into the structures present and operating in the cell. Therefore, as used herein, "expression" may refer to transcription into a polynucleotide, translation into a polypeptide, or even polynucleotide and/or polypeptide modifications (e.g., posttranslational modification of a polypeptide). Fragments of the transcribed polynucleotide, the translated polypeptide, or polynucleotide and/or polypeptide modifications (e.g., posttranslational modification of a polypeptide) shall also be regarded as expressed whether they originate from a transcript generated by alternative splicing or a degraded transcript, or from a post-translational processing of the polypeptide, *e*.*g*., by proteolysis. "Expressed genes" include those that are transcribed into a polynucleotide as mRNA and then translated into a polypeptide, and also those that are transcribed into RNA but not translated into a polypeptide (for example, transfer and ribosomal RNAs).

"Elevated expression," "elevated expression levels," or "elevated levels" refers to an increased expression or increased levels of a biomarker in an individual relative to a control, such as an individual or individuals who are not suffering from the disease or disorder (*e*.*g*., cancer) or an internal control (*e*.*g*., housekeeping biomarker).

"Reduced expression," "reduced expression levels," or "reduced levels" refers to a decrease expression or decreased levels of a biomarker in an individual relative to a control, such as an individual or individuals who are not suffering from the disease or disorder (*e*.*g*., cancer) or an internal control (*e*.*g*., housekeeping biomarker).

The term "housekeeping biomarker" refers to a biomarker or group of biomarkers (e.g., polynucleotides and/or polypeptides) which are typically similarly present in all cell types. In some aspects of the disclosure, the housekeeping biomarker is a "housekeeping gene." A "housekeeping gene" refers herein to a gene or group of genes which encode proteins whose activities are essential for the maintenance of cell function and which are typically similarly present in all cell types.

"Amplification," as used herein generally refers to the process of producing multiple copies of a desired sequence. "Multiple copies" mean at least two copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

The term "multiplex-PCR" refers to a single PCR reaction carried out on nucleic acid obtained from a single source (e.g., an individual) using more than one primer set for the purpose of amplifying two or more DNA sequences in a single reaction.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, *see* Ausubel et al., Current Protocols in Molecular Biology, Wiley Interscience Publishers, (1995).

"Stringent conditions" or "high stringency conditions", as defined herein, can be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) overnight hybridization in a solution that employs 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with a 10 minute wash at 42°C in 0.2 x SSC (sodium chloride/sodium citrate) followed by a 10 minute high-stringency wash consisting of 0.1 x SSC containing EDTA at 55°C.

"Moderately stringent conditions" can be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "diagnosis" is used herein to refer to the identification or classification of a molecular or pathological state, disease or condition (*e.g*., cancer). For example, "diagnosis" may refer to identification of a particular type of cancer. "Diagnosis" may also refer to the classification of a particular subtype of cancer, *e.g.,* by histopathological criteria, or by molecular features (*e.g.,* a subtype characterized by expression of one or a combination of biomarkers (*e.g*., particular genes or proteins encoded by said genes)).

The term "aiding diagnosis" is used herein to refer to methods that assist in making a clinical determination regarding the presence, or nature, of a particular type of symptom or condition of a disease or disorder (*e.g*., cancer). For example, a method of aiding diagnosis of a disease or condition (*e.g*., cancer) can comprise detecting certain biomarkers in a biological sample from an individual.

The term "sample," as used herein, refers to a composition that is obtained or derived from a subject and/or individual of interest that contains a cellular and/or other molecular entity that is to be characterized and/or identified, for example based on physical, biochemical, chemical and/or physiological characteristics. For example, the phrase "disease sample" and variations thereof refers to any sample obtained from a subject of interest that would be expected or is known to contain the cellular and/or molecular entity that is to be characterized. Samples include, but are not limited to, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, and combinations thereof.

By "tissue sample" or "cell sample" is meant a collection of similar cells obtained from a tissue of a subject or individual. The source of the tissue or cell sample may be solid tissue as from a fresh, frozen and/or preserved organ, tissue sample, biopsy, and/or aspirate; blood or any blood constituents such as plasma; bodily fluids such as cerebral spinal fluid, amniotic fluid, peritoneal fluid, or interstitial fluid; cells from any time in gestation or development of the subject. The tissue sample may also be primary or cultured cells or cell lines. Optionally, the tissue or cell sample is obtained from a disease tissue/organ. The tissue sample may contain compounds which are not naturally intermixed with the tissue in nature such as preservatives, anticoagulants, buffers, fixatives, nutrients, antibiotics, or the like.

A "reference sample", "reference cell", "reference tissue", "control sample", "control cell", or "control tissue", as used herein, refers to a sample, cell, tissue, standard, or level that is used for comparison purposes. In one aspect of the disclosure, a reference sample, reference cell, reference tissue, control sample, control cell, or control tissue is obtained from a healthy and/or non-diseased part of the body (*e.g*., tissue or cells) of the same subject or individual. For example, healthy and/or non-diseased cells or tissue adjacent to the diseased cells or tissue (*e.g*., cells or tissue adjacent to a tumor). In another aspect of the disclosure, a reference sample is obtained from an untreated tissue and/or cell of the body of the same subject or individual. In yet another aspect of the disclosure, a reference sample, reference cell, reference tissue, control sample, control cell, or control tissue is obtained from a healthy and/or non-diseased part of the body (*e.g*., tissues or cells) of an individual who is not the subject or individual. In even another aspect of the disclosure, a reference sample, reference cell, reference tissue, control sample, control cell, or control tissue is obtained from an untreated tissue and/or cell of the body of an individual who is not the subject or individual.

For the purposes herein a "section" of a tissue sample is meant a single part or piece of a tissue sample, *e.g.,* a thin slice of tissue or cells cut from a tissue sample. It is understood that multiple sections of tissue samples may be taken and subjected to analysis, provided that it is understood that the same section of tissue sample may be analyzed at both morphological and molecular levels, or analyzed with respect to both polypeptides and polynucleotides.

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to the aspect of the disclosure of polynucleotide analysis or protocol, one may use the results of the polynucleotide expression analysis or protocol to determine whether a specific therapeutic regimen should be performed.

"Individual response" or "response" can be assessed using any endpoint indicating a benefit to the individual, including, without limitation, (1) inhibition, to some extent, of disease progression (*e.g*., cancer progression), including slowing down and complete arrest; (2) a reduction in tumor size; (3) inhibition (*i.e.,* reduction, slowing down or complete stopping) of cancer cell infiltration into adjacent peripheral organs and/or tissues; (4) inhibition (*i.e.* reduction, slowing down or complete stopping) of metasisis; (5) relief, to some extent, of one or more symptoms associated with the disease or disorder (*e.g*., cancer); (6) increase in the length of progression free survival; and/or (9) decreased mortality at a given point of time following treatment.

The phrase "substantially similar," as used herein, refers to a sufficiently high degree of similarity between two numeric values (generally one associated with a molecule and the other associated with a reference/comparator molecule) such that one of skill in the art would consider the difference between the two values to not be of statistical significance within the context of the biological characteristic measured by said values (e.g., Kd values). The difference between said two values may be, for example, less than about 20%, less than about 10%, and/or less than about 5% as a function of the reference/comparator value. The phrase "substantially normal" refers to substantially similar to a reference (*e.g*., normal reference).

The phrase "substantially different," refers to a sufficiently high degree of difference between two numeric values (generally one associated with a molecule and the other associated with a reference/comparator molecule) such that one of skill in the art would consider the difference between the two values to be of statistical significance within the context of the biological characteristic measured by said values (*e.g*., Kd values). The difference between said two values may be, for example, greater than about 10%, greater than about 20%, greater than about 30%, greater than about 40%, and/or greater than about 50% as a function of the value for the reference/comparator molecule.

The word "label" when used herein refers to a detectable compound or composition. The label is typically conjugated or fused directly or indirectly to a reagent, such as a polynucleotide probe or an antibody, and facilitates detection of the reagent to which it is conjugated or fused. The label may itself be detectable (*e.g*., radioisotope labels or fluorescent labels) or, in the case of an enzymatic label, may catalyze chemical alteration of a substrate compound or composition which results in a detectable product.

An "effective amount" of an agent refers to an amount effective, at dosages and for periods of time necessary, to achieve the desired therapeutic or prophylactic result.

A "therapeutically effective amount" of a substance/molecule of the invention, agonist or antagonist may vary according to factors such as the disease state, age, sex, and weight of the individual, and the ability of the substance/molecule, agonist or antagonist to elicit a desired response in the individual. A therapeutically effective amount is also one in which any toxic or detrimental effects of the substance/molecule, agonist or antagonist are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some aspects of the disclosure, antibodies of the invention are used to delay development of a disease or to slow the progression of a disease.

The terms "cancer" and "cancerous" refer to or describe the physiological condition in mammals that is typically characterized by unregulated cell growth/proliferation. Examples of cancer include, but are not limited to, carcinoma, lymphoma (e.g., Hodgkin's and non-Hodgkin's lymphoma), blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer. In some embodiments, the cancer is renal cell carcinoma (e.g., non-clear cell renal cell carcinoma (nccRCC), translocation RCC (tRCC)), melanoma, perivascular epithelioid tumor, alveolar soft part sarcoma, clear cell sarcoma, angiomyolipoma, lymphangioleiomyomatoma, clear cell sugar lung tumor, pancreatic, uterine, schwannoma, cellular blue nevi, neurofibroma, germ cell tumor, lymphoma, liposarcoma.

The term "anti-cancer therapy" refers to a therapy useful in treating cancer. Examples of anti-cancer therapeutic agents include, but are limited to, *e*.*g*., chemotherapeutic agents, growth inhibitory agents, cytotoxic agents, agents used in radiation therapy, anti-angiogenesis agents, apoptotic agents, anti-tubulin agents, and other agents to treat cancer , anti-CD20 antibodies, platelet derived growth factor inhibitors (*e.g.*, Gleevec™ (Imatinib Mesylate)), a COX-2 inhibitor *(e.g.,* celecoxib), interferons, cytokines, antagonists *(e.g.,* neutralizing antibodies) that bind to one or more of the following targets PDGFR-beta, BlyS, APRIL, BCMA receptor(s), TRAIL/Apo2, and other bioactive and organic chemical agents, etc. Combinations thereof are also included in the invention.

The term "cytotoxic agent" as used herein refers to a substance that inhibits or prevents a cellular function and/or causes cell death or destruction. Cytotoxic agents include, but are not limited to, radioactive isotopes (*e*.*g*., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (*e.g*., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

A "chemotherapeutic agent" refers to a chemical compound useful in the treatment of cancer. Examples of chemotherapeutic agents include alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegaI1 (*see, e.g.,* Nicolaou et al., Angew. Chem Intl. Ed. Engl., 33: 183-186 (1994)); CDP323, an oral alpha-4 integrin inhibitor; dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXIL®), liposomal doxorubicin TLC D-99 (MYOCET®), pegylated liposomal doxorubicin (CAELYX®), and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2'-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoid, *e*.*g*., paclitaxel (TAXOL®), albumin-engineered nanoparticle formulation of paclitaxel (ABRAXANE™), and docetaxel (TAXOTERE®); chloranbucil; 6-thioguanine; mercaptopurine; methotrexate; platinum agents such as cisplatin, oxaliplatin (*e.g*., ELOXATIN®), and carboplatin; vincas, which prevent tubulin polymerization from forming microtubules, including vinblastine (VELBAN®), vincristine (ONCOVIN®), vindesine (ELDISINE®, FILDESIN®), and vinorelbine (NAVELBINE®); etoposide (VP-16); ifosfamide; mitoxantrone; leucovorin; novantrone; edatrexate; daunomycin; aminopterin; ibandronate; topoisomerase inhibitor RFS 2000; difluoromethylornithine (DMFO); retinoids such as retinoic acid, including bexarotene (TARGRETIN®); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; topoisomerase 1 inhibitor *(e.g.,* LURTOTECAN®); rmRH (*e.g.,* ABARELIX®); BAY439006 (sorafenib; Bayer); SU-11248 (sunitinib, SUTENT®, Pfizer); perifosine, COX-2 inhibitor (*e.g.,* celecoxib or etoricoxib), proteosome inhibitor (*e.g.,* PS341); bortezomib (VELCADE®); CCI-779; tipifarnib (R11577); orafenib, ABT510; Bcl-2 inhibitor such as oblimersen sodium (GENASENSE®); pixantrone; EGFR inhibitors (*see* definition below); tyrosine kinase inhibitors (*see* definition below); serine-threonine kinase inhibitors such as rapamycin (sirolimus, RAPAMUNE®); farnesyltransferase inhibitors such as lonafarnib (SCH 6636, SARASAR™); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN™) combined with 5-FU and leucovorin.

Chemotherapeutic agents as defined herein include "anti-hormonal agents" or "endocrine therapeutics" which act to regulate, reduce, block, or inhibit the effects of hormones that can promote the growth of cancer. They may be hormones themselves, including, but not limited to: anti-estrogens with mixed agonist/antagonist profile, including, tamoxifen (NOLVADEX®), 4-hydroxytamoxifen, toremifene (FARESTON®), idoxifene, droloxifene, raloxifene (EVISTA®), trioxifene, keoxifene, and selective estrogen receptor modulators (SERMs) such as SERM3; pure anti-estrogens without agonist properties, such as fulvestrant (FASLODEX®), and EM800 (such agents may block estrogen receptor (ER) dimerization, inhibit DNA binding, increase ER turnover, and/or suppress ER levels); aromatase inhibitors, including steroidal aromatase inhibitors such as formestane and exemestane (AROMASIN®), and nonsteroidal aromatase inhibitors such as anastrazole (ARIMIDEX®), letrozole (FEMARA®) and aminoglutethimide, and other aromatase inhibitors include vorozole (RIVISOR®), megestrol acetate (MEGASE®), fadrozole, and 4(5)-imidazoles; lutenizing hormone-releaseing hormone agonists, including leuprolide (LUPRON® and ELIGARD®), goserelin, buserelin, and tripterelin; sex steroids, including progestines such as megestrol acetate and medroxyprogesterone acetate, estrogens such as diethylstilbestrol and premarin, and androgens/retinoids such as fluoxymesterone, all transretionic acid and fenretinide; onapristone; anti-progesterones; estrogen receptor down-regulators (ERDs); anti-androgens such as flutamide, nilutamide and bicalutamide; and pharmaceutically acceptable salts, acids or derivatives of any of the above; as well as combinations of two or more of the above.

The term "prodrug" as used in this application refers to a precursor or derivative form of a pharmaceutically active substance that is less cytotoxic to tumor cells compared to the parent drug and is capable of being enzymatically activated or converted into the more active parent form. *See, e.g.,* Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

A "growth inhibitory agent" when used herein refers to a compound or composition which inhibits growth of a cell (*e.g.,* a cell whose growth is dependent upon a MiT gene and/or MITF translocation expression either *in vitro* or *in vivo*)*.* Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al., (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one time administration and typical dosages range from 10 to 200 units (Grays) per day.

An "individual" or "subject" is a mammal. Mammals include, but are not limited to, domesticated animals (*e.g.,* cows, sheep, cats, dogs, and horses), primates (*e.g.,* humans and non-human primates such as monkeys), rabbits, and rodents (*e.g*., mice and rats). In certain aspects of the disclosure, the individual or subject is a human.

The term "concurrently" is used herein to refer to administration of two or more therapeutic agents, where at least part of the administration overlaps in time. Accordingly, concurrent administration includes a dosing regimen when the administration of one or more agent(s) continues after discontinuing the administration of one or more other agent(s).

By "reduce" or "inhibit" is meant the ability to cause an overall decrease of 20%, 30%, 40%, 50%, 60%, 70%, 75%, 80%, 85%, 90%, 95%, or greater. Reduce or inhibit can refer to the symptoms of the disorder being treated, the presence or size of metastases, or the size of the primary tumor.

The term "package insert" is used to refer to instructions customarily included in commercial packages of therapeutic products, that contain information about the indications, usage, dosage, administration, combination therapy, contraindications and/or warnings concerning the use of such therapeutic products.

An "article of manufacture" is any manufacture (*e.g*., a package or container) or kit comprising at least one reagent, *e.g.,* a medicament for treatment of a disease or disorder (*e.g.,* cancer), or a probe for specifically detecting a biomarker described herein. In certain aspects of the disclosure, the manufacture or kit is promoted, distributed, or sold as a unit for performing the methods described herein.

A "target audience" is a group of people or an institution to whom or to which a particular medicament is being promoted or intended to be promoted, as by marketing or advertising, especially for particular uses, treatments, or indications, such as individuals, populations, readers of newspapers, medical literature, and magazines, television or internet viewers, radio or internet listeners, physicians, drug companies, etc.

As is understood by one skilled in the art, reference to "about" a value or parameter herein includes (and describes) aspects of the disclosure that are directed to that value or parameter per se. For example, description referring to "about X" includes description of "X".

It is understood that aspect and embodiments of the invention described herein include "consisting" and/or "consisting essentially of" aspects and embodiments. As used herein, the singular form "a", "an", and "the" includes plural references unless indicated otherwise.

### II. Methods and Uses

Described herein are methods utilizing a MiT antagonist. In particular, described herein are methods utilizing a MiT-translocation antagonist. For example, described herein are methods of inhibiting cell proliferation of a cancer cell comprising contacting the cancer cell with an effective amount of a MiT-translocation antagonist. Also described herein are methods of treating cancer in an individual comprising administering to the individual an effective amount of a MiT - translocation antagonist. In some embodiments, the cancer or cancer comprises a MiT translocation.

Also described herein are methods of treating cancer in an individual comprising administering to the individual an effective amount of an anti-cancer therapy, wherein treatment is based upon the individual having cancer comprising one or more biomarkers. In some aspects of the disclosure, the anti-cancer therapy comprises a MiT antagonist. For example, described are methods of treating cancer in an individual comprising administering to the individual an effective amount of a MiT antagonist, wherein treatment is based upon the individual having cancer comprising MiT overexpression and/or a MiT translocation. For example, described are methods of treating cancer in an individual comprising administering to the individual an effective amount of a MiT antagonist, wherein treatment is based upon the individual having cancer comprising MET overexpression and/or BIRC7 overexpression. In some aspects of the disclosure, the MiT antagonist is MITF, TFEB, TFE3, TFEC and/or SBNO2 antagonist). In some aspects of the disclosure, the MiT antagonist is a MiT-translocation antagonist (e.g., a MITF-, TFEB-, TFE3-, TFEC- and/or SBNO2-translocation antagonist). In some aspects of the disclosure, the MiT antagonist is a MET pathway antagonist. In some aspects of the disclosure, the MiT antagonist is a BIRC7 pathway antagonist.

Also described herein are methods of treating nccRCC in an individual comprising administering to the individual an effective amount of an anti-cancer therapy, wherein treatment is based upon the individual nccRCC cancer comprising one or more biomarkers. In some aspects of the disclosure, the anti-cancer therapy comprises a MiT antagonist. For example, described are methods of treating nccRCC in an individual comprising administering to the individual an effective amount of a MiT antagonist, wherein treatment is based upon the individual having cancer comprising MiT overexpression and/or a MiT translocation. For example, described are methods of treating nccRCC in an individual comprising administering to the individual an effective amount of a MiT antagonist, wherein treatment is based upon the individual having cancer comprising MET overexpression and/or BIRC7 overexpression. In some aspects of the disclosure, the MiT antagonist is MITF, TFEB, TFE3, TFEC and/or SBNO2 antagonist). In some aspects of the disclosure, the MiT antagonist is a MiT-translocation antagonist (e.g., a MITF-, TFEB-, TFE3-, TFEC- and/or SBNO2-translocation antagonist). In some aspects of the disclosure, the MiT antagonist is a MET pathway antagonist. In some aspects of the disclosure, the MiT antagonist is a BIRC7 pathway antagonist.

Further described herein are methods of treating cancer in an individual described that the individual has been found to have cancer comprising one or more biomarkers, the treatment comprising administering to the individual an effective amount of an anti-cancer therapy. In some aspects of the disclosure, the anti-cancer therapy comprises a MiT antagonist. For example, described herein are methods of treating cancer in an individual described that the individual has been found to have cancer comprising a MiT translocation, the treatment comprising administering to the individual an effective amount of a MiT antagonist. For example, described herein are methods of treating cancer in an individual described that the individual has been found to have cancer comprising MET overexpression and/or BIRC7 overexpression, the treatment comprising administering to the individual an effective amount of a MiT antagonist. In some aspects of the disclosure, the MiT antagonist is a MiT-translocation antagonist. In some aspects of the disclosure, the MiT antagonist is a MET pathway antagonist. In some aspects of the disclosure, the MiT antagonist is a BIRC7 pathway antagonist.

Further described herein are methods of treating nccRCC in an individual described that the individual has been found to have nccRCC comprising one or more biomarkers, the treatment comprising administering to the individual an effective amount of an anti-cancer therapy. In some aspects of the disclosure, the anti-cancer therapy comprises a MiT antagonist. For example, described herein are methods of treating nccRCC in an individual described that the individual has been found to have nccRCC comprising a MiT translocation, the treatment comprising administering to the individual an effective amount of a MiT antagonist. For example, described herein are methods of treating nccRCC in an individual described that the individual has been found to have nccRCC comprising MET overexpression and/or BIRC7 overexpression, the treatment comprising administering to the individual an effective amount of a MiT antagonist. n some aspects of the disclosure, the MiT antagonist is a MiT-translocation antagonist. In some aspects of the disclosure, the MiT antagonist is a MET pathway antagonist. In some aspects of the disclosure, the MiT antagonist is a BIRC7 pathway antagonist.

Described herein are methods of treating a cancer cell, wherein the cancer cell comprises one or more biomarkers, the method comprising providing an effective amount of a MiT antagonist. For example, described herein are methods of treating a cancer cell, wherein the cancer cell comprises an MiT translocation, the method comprising providing an effective amount of a MiT antagonist. For example, described herein are methods of treating a cancer cell, wherein the cancer cell comprises MET overexpression and/or BIRC7 overexpression, the method comprising providing an effective amount of a MiT antagonist. In some aspects of the disclosure, the MiT antagonist is a MiT-translocation antagonist. In some aspects of the disclosure, the MiT antagonist is a MET pathway antagonist. In some aspects of the disclosure, the MiT antagonist is a BIRC7 pathway antagonist.

Described herein are methods of treating a nccRCC cell, wherein the cancer cell comprises one or more biomarkers, the method comprising providing an effective amount of a MiT antagonist. For example, described herein are methods of treating a nccRCC cancer cell, wherein the cancer cell comprises an MiT translocation, the method comprising providing an effective amount of a MiT antagonist. For example, described herein are methods of treating a nccRCC cancer cell, wherein the cancer cell comprises MET overexpression and/or BIRC7 overexpression, the method comprising providing an effective amount of a MiT antagonist. In some aspects of the disclosure, the MiT antagonist is a MiT-translocation antagonist. In some aspects of the disclosure, the MiT antagonist is a MET pathway antagonist. In some aspects of the disclosure, the MiT antagonist is a BIRC7 pathway antagonist.

Described herein are methods for treating cancer in an individual, the method comprising: determining that a sample obtained from the individual comprises one or more biomarkers, and administering an effective amount of an anti-cancer therapy comprising a MiT antagonist to the individual, whereby the cancer is treated. For example, described herein are methods for treating cancer in an individual, the method comprising: determining that a sample obtained from the individual comprises MiT overexpression and/or MiT translocation, and administering an effective amount of an anti-cancer therapy comprising a MiT antagonist to the individual, whereby the cancer is treated.

In some aspects of the disclosure, the MiT antagonist is a MiT-translocation antagonist. In some aspects of the disclosure, the MiT antagonist is a MET pathway antagonist. In some aspects of the disclosure, the MiT antagonist is a BIRC7 pathway antagonist.

Described herein are methods for treating nccRCC in an individual, the method comprising: determining that a sample obtained from the individual comprises one or more biomarkers, and administering an effective amount of an anti-cancer therapy comprising a MiT antagonist to the individual, whereby the cancer is treated. For example, described herein are methods for treating nccRCC in an individual, the method comprising: determining that a sample obtained from the individual comprises MiT overexpression and/or MiT translocation, and administering an effective amount of an anti-cancer therapy comprising a MiT antagonist to the individual, whereby the nccRCC is treated. For example, described herein are methods for treating nccRCC in an individual, the method comprising: determining that a sample obtained from the individual comprises MET overexpression and/or BIRC7 overexpression and administering an effective amount of an anti-cancer therapy comprising a MiT antagonist to the individual, whereby the nccRCC is treated. In some aspects of the disclosure, the MiT antagonist is a MiT-translocation antagonist. In some aspects of the disclosures, the MiT antagonist is a MET pathway antagonist. In some aspects of the disclosure, the MiT antagonist is a BIRC7 pathway antagonist.

Described herein are also methods of treating cancer, comprising: (a) selecting an individual having cancer, wherein the cancer comprises one or more biomarkers; and (b) administering to the individual thus selected an effective amount of a MiT antagonist, whereby the cancer is treated. For example, described herein are also methods of treating cancer, comprising: (a) selecting an individual having cancer, wherein the cancer comprises a MiT translocation; and (b) administering to the individual thus selected an effective amount of a MiT antagonist, whereby the cancer is treated. In some aspects of the disclosure, the MiT antagonist is a MiT-translocation antagonist. In some aspects of the disclosure, the MiT antagonist is a MET pathway antagonist. In some aspects of the disclosure, the MiT antagonist is a BIRC7 pathway antagonist.

Described herein are also methods of treating nccRCC, comprising: (a) selecting an individual having nccRCC, wherein the nccRCC comprises one or more biomarkers; and (b) administering to the individual thus selected an effective amount of a MiT antagonist, whereby the nccRCC is treated. For example, described herein are also methods of treating nccRCC, comprising: (a) selecting an individual having cancer, wherein the nccRCC comprises a MiT translocation; and (b) administering to the individual thus selected an effective amount of a MiT antagonist, whereby the nccRCC is treated. In some aspects of the disclosure, the MiT antagonist is a MiT-translocation antagonist. In some aspects of the disclosure, the MiT antagonist is a MET pathway antagonist. In some aspects of the disclosure, the MiT antagonist is a BIRC7 pathway antagonist. For example, described herein are also methods of treating nccRCC, comprising: (a) selecting an individual having cancer, wherein the nccRCC comprises MET overexpression and/or BIRC7 overexpression; and (b) administering to the individual thus selected an effective amount of a MiT antagonist, whereby the nccRCC is treated. In some aspects of the disclosure, the MiT antagonist is a MiT-translocation antagonist. In some aspects of the disclosure, the MiT antagonist is a MET pathway antagonist. In some aspects of the disclosure, the MiT antagonist is a BIRC7 pathway antagonist.

Further described herein are methods of identifying an individual with cancer who is more or less likely to exhibit benefit from treatment with an anti-cancer therapy, the method comprising: determining presence or absence of one or more biomarkers in a sample obtained from the individual, wherein presence of the one or more biomarkers in the sample indicates that the individual is more likely to exhibit benefit from treatment with the anti-cancer therapy or absence of the one or more biomarkers indicates that the individual is less likely to exhibit benefit from treatment with the anti-cancer therapy. In some aspects of the disclosure, the anti-cancer therapy comprises a MiT antagonist. For example, described herein are methods of identifying an individual with cancer who is more or less likely to exhibit benefit from treatment with an anti-cancer therapy comprising a MiT antagonist, the method comprising: determining presence or absence of MiT overexpression and/or MiT translocation in a sample obtained from the individual, wherein presence of the MiT overexpression and/or MiT translocation in the sample indicates that the individual is more likely to exhibit benefit from treatment with the anti-cancer therapy comprising the MiT antagonist or absence of the MiT overexpression and/or MiT translocation indicates that the individual is less likely to exhibit benefit from treatment with the anti-cancer therapy comprising the MiT antagonist. In some aspects of the disclosure, the method further comprises administering an effective amount of a MiT antagonist. In some aspects of the disclosure, the MiT antagonist is a MiT-translocation antagonist. In some aspects of the disclosure, the MiT antagonist is a MET pathway antagonist. In some aspects of the disclosure, the MiT antagonist is a BIRC7 pathway antagonist.

Further described herein are methods of identifying an individual with nccRCC who is more or less likely to exhibit benefit from treatment with an anti-cancer therapy, the method comprising: determining presence or absence of one or more biomarkers in a sample obtained from the individual, wherein presence of the one or more biomarkers in the sample indicates that the individual is more likely to exhibit benefit from treatment with the anti-cancer therapy or absence of the one or more biomarkers indicates that the individual is less likely to exhibit benefit from treatment with the anti-cancer therapy. In some aspects of the disclosure, the anti-cancer therapy comprises a MiT antagonist. For example, described herein are methods of identifying an individual with nccRCC who is more or less likely to exhibit benefit from treatment with an anti-cancer therapy comprising a MiT antagonist, the method comprising: determining presence or absence of MiT overexpression and/or MiT translocation in a sample obtained from the individual, wherein presence of the MiT overexpression and/or MiT translocation in the sample indicates that the individual is more likely to exhibit benefit from treatment with the anti-cancer therapy comprising the MiT antagonist or absence of the MiT overexpression and/or MiT translocation indicates that the individual is less likely to exhibit benefit from treatment with the anti-cancer therapy comprising the MiT antagonist. For example, described herein are methods of identifying an individual with nccRCC who is more or less likely to exhibit benefit from treatment with an anti-cancer therapy comprising a MiT antagonist, the method comprising: determining presence or absence of MET overexpression and/or BIRC7 overexpression in a sample obtained from the individual, wherein presence of the MiT overexpression and/or MiT translocation in the sample indicates that the individual is more likely to exhibit benefit from treatment with the anti-cancer therapy comprising the MiT antagonist or absence of the MiT overexpression and/or MiT translocation indicates that the individual is less likely to exhibit benefit from treatment with the anti-cancer therapy comprising the MiT antagonist. In some aspects of the disclosure, the method further comprises administering an effective amount of a MiT antagonist. In some aspects of the disclosure, the MiT antagonist is a MiT-translocation antagonist. In some aspects of the disclosure, the MiT antagonist is a MET pathway antagonist. In some aspects of the disclosure, the MiT antagonist is a BIRC7 pathway antagonist.

Described herein are methods for predicting whether an individual with cancer is more or less likely to respond effectively to treatment with an anti-cancer therapy comprising a MiT antagonist, the method comprising determining one or more biomarkers, whereby presence of the one or more biomarkers indicates that the individual is more likely to respond effectively to treatment with the MiT antagonist and absence of the one or more biomarkers indicates that the individual is less likely to respond effectively to treatment with the MiT antagonist. For example, described herein are methods for predicting whether an individual with cancer is more or less likely to respond effectively to treatment with an anti-cancer therapy comprising a MiT antagonist, the method comprising determining a MiT overexpression and/or MiT translocation, whereby presence of the MiT overexpression and/or MiT translocation indicates that the individual is more likely to respond effectively to treatment with the MiT antagonist and absence of the MiT overexpression and/or MiT translocation indicates that the individual is less likely to respond effectively to treatment with the MiT antagonist. In some aspects of the disclosure, the method further comprises administering an effective amount of a MiT antagonist. In some aspects of the disclosure, the MiT antagonist is a MiT-translocation antagonist. In some aspects of the disclosure, the MiT antagonist is a MET pathway antagonist. In some aspects of the disclosure, the MiT antagonist is a BIRC7 pathway antagonist.

Described herein are methods for predicting whether an individual with nccRCC is more or less likely to respond effectively to treatment with an anti-cancer therapy comprising a MiT antagonist, the method comprising determining one or more biomarkers, whereby presence of the one or more biomarkers indicates that the individual is more likely to respond effectively to treatment with the MiT antagonist and absence of the one or more biomarkers indicates that the individual is less likely to respond effectively to treatment with the MiT antagonist. For example, described herein are methods for predicting whether an individual with nccRCC is more or less likely to respond effectively to treatment with an anti-cancer therapy comprising a MiT antagonist, the method comprising determining a MiT overexpression and/or MiT translocation, whereby presence of the MiT overexpression and/or MiT translocation indicates that the individual is more likely to respond effectively to treatment with the MiT antagonist and absence of the MiT overexpression and/or MiT translocation indicates that the individual is less likely to respond effectively to treatment with the MiT antagonist. For example, described herein are methods for predicting whether an individual with nccRCC is more or less likely to respond effectively to treatment with an anti-cancer therapy comprising a MiT antagonist, the method comprising determining MET overexpression and/or BIRC7 overexpression, whereby presence of the MET overexpression and/or BIRC7 overexpression indicates that the individual is more likely to respond effectively to treatment with the MiT antagonist and absence of the MiT overexpression and/or MiT translocation indicates that the individual is less likely to respond effectively to treatment with the MiT antagonist. In some aspects of the disclosure, the method further comprises administering an effective amount of a MiT antagonist. In some aspects of the disclosure, the MiT antagonist is a MiT-translocation antagonist. In some embodiments, the MiT antagonist is a MET pathway antagonist. In some aspects of the disclosure, the MiT antagonist is a BIRC7 pathway antagonist.

Disclosed herein are methods of predicting the response or lack of response of an individual with cancer to an anti-cancer therapy comprising a MiT antagonist comprising detecting in a sample obtained from the individual presence or absence of one or more biomarkers, wherein presence of the one or more biomarkers is predictive of response of the individual to the anti-cancer therapy comprising the MiT antagonist and absence of the one or more biomarkers is predictive of lack of response of the individual to the anti-cancer therapy comprising the MiT antagonist. For example, provided herein are methods of predicting the response or lack of response of an individual with cancer to an anti-cancer therapy comprising a MiT antagonist comprising detecting in a sample obtained from the individual presence or absence of MiT overexpression and/or MiT translocation, wherein presence of the MiT overexpression and/or MiT translocation is predictive of response of the individual to the anti-cancer therapy comprising the MiT antagonist and absence of the MiT overexpression and/or MiT translocation is predictive of lack of response of the individual to the anti-cancer therapy comprising the MiT antagonist. In some aspects of the disclosure, the method further comprises administering an effective amount of a MiT antagonist. In some aspects of the disclosure, the MiT antagonist is a MiT-translocation antagonist. In some aspects of the disclosure, the MiT antagonist is a MET pathway antagonist. In some aspects of the disclosure, the MiT antagonist is a BIRC7 pathway antagonist.

Disclosed herein are methods of predicting the response or lack of response of an individual with nccRCC to an anti-cancer therapy comprising a MiT antagonist comprising detecting in a sample obtained from the individual presence or absence of one or more biomarkers, wherein presence of the one or more biomarkers is predictive of response of the individual to the anti-cancer therapy comprising the MiT antagonist and absence of the one or more biomarkers is predictive of lack of response of the individual to the anti-cancer therapy comprising the MiT antagonist. For example, provided herein are methods of predicting the response or lack of response of an individual with nccRCC to an anti-cancer therapy comprising a MiT antagonist comprising detecting in a sample obtained from the individual presence or absence of MiT overexpression and/or MiT translocation, wherein presence of the MiT overexpression and/or MiT translocation is predictive of response of the individual to the anti-cancer therapy comprising the MiT antagonist and absence of the MiT overexpression and/or MiT translocation is predictive of lack of response of the individual to the anti-cancer therapy comprising the MiT antagonist. For example, provided herein are methods of predicting the response or lack of response of an individual with nccRCC to an anti-cancer therapy comprising a MiT antagonist comprising detecting in a sample obtained from the individual presence or absence of MET expression and/or BIRC7 expression, wherein presence of the MET expression and/or BIRC7 expression is predictive of response of the individual to the anti-cancer therapy comprising the MiT antagonist and absence of the MET expression and/or BIRC7 expression is predictive of lack of response of the individual to the anti-cancer therapy comprising the MiT antagonist. In some aspects of the disclosure, the method further comprises administering an effective amount of a MiT antagonist. In some aspects of the disclosure, the MiT antagonist is a MiT-translocation antagonist. In some aspects of the disclosure, the MiT antagonist is a MET pathway antagonist. In some aspects of the disclosure, the MiT antagonist is a BIRC7 pathway antagonist.

In some aspects of the disclosure of any of the methods, the one or more biomarkers comprise one or more genes selected from the group consisting of MET, HIF1A, APEX1, and BIRC7. In some aspects of the disclosure, the presence of one or more biomarkers comprises the presence of a variation (*e.g*., polymorphism or mutation) of one or more genes selected from the group consisting of MET, and BIRC7. In some aspects of the disclosure, the variation (*e.g*., polymorphism or mutation) is a somatic variation (*e.g*., polymorphism or mutation).

In some aspects of the disclosure of any of the methods, the one or more biomarkers comprise one or more MiT (e.g., MITF, TFEB, TFE3, TFEC, and/or SBNO2). In some aspects of the disclosure, presence of the one or more biomarkers is indicated by the presence of elevated expression levels (*e.g*., compared to reference) of one or more MiT (e.g., MITF, TFEB, TFE3, TFEC, and/or SBNO2). In some aspects of the disclosures expression is polypeptide expression. In some aspects of the disclosure, expression is nucleic acid expression. In some aspects of the disclosure, the one or more biomarkers comprises MITF. In some aspects of the disclosure, the one or more biomarkers comprises TFEB. In some aspects of the disclosure, the one or more biomarkers comprises TFE3. In some aspects of the disclosure, the one or more biomarkers comprises TFEC. In some aspects of the disclosure, the one or more biomarkers comprises SBNO2.

In some aspects of the disclosure of any of the methods, the one or more biomarkers comprise MET and/or BIRC7. In some aspects of the disclosure, presence of the one or more biomarkers is indicated by the presence of elevated expression levels (*e.g*., compared to reference) of MET and/or BIRC7. In some aspects of the disclosure, expression is polypeptide expression. In some aspects of the disclosure, expression is nucleic acid expression. In some aspects of the disclosure, the one or more biomarkers comprises MET. In some aspects of the disclosure, the one or more biomarkers comprises BIRC7.

In some aspects of the disclosure of any of the methods, the one or more biomarkers comprise one or more of MITF, TFEB, TFE3, TFEC, SBNO2, MET and/or BIRC7. In some aspects of the disclosure, presence of the one or more biomarkers is indicated by the presence of elevated expression levels (e.g., compared to reference) of one or more of MITF, TFEB, TFE3, TFEC, SBNO2, MET and/or BIRC7.

In some aspects of the disclosure of any of the methods, the one or more biomarkers comprise a translocation (*e.g*., inversion, rearrangement and/or fusion) of one or more genes selected from MITF, TFEB, TFE3, TFEC, and/or SBNO2. In some aspects of the disclosure, the presence of one or more biomarkers comprises the presence of a translocation (*e.g*., rearrangement and/or fusion) of one or more genes selected from MITF, TFEB, TFE3, TFEC, and/or SBNO2.

In some aspects of the disclosure of any of the methods, the one or more biomarkers comprise a translocation (*e.g*., rearrangement and/or fusion) of one or more genes selected from MITF, TFEB, TFE3, TFEC, and/or SBNO2, and one or more of MET and/or BIRC7. In some aspects of the disclosure, the presence of one or more biomarkers comprises the presence of a translocation (*e.g*., rearrangement and/or fusion) of one or more genes selected from MITF, TFEB, TFE3, TFEC, and/or SBNO2, and overexpression of one or more of MET and/or BIRC7.

In some aspects of the disclosure of any of the methods, the translocation (*e.g*., rearrangement and/or fusion) is a MITF translocation (*e.g*., rearrangement and/or fusion). In some aspects of the disclosure, the MITF translocation (*e.g*., rearrangement and/or fusion) comprises *ACTG1* and *MITF.* In some aspects of the disclosure, the MITF translocation *(e.g.,* rearrangement and/or fusion) comprises *ACTG1* exon 3. In some aspects of the disclosure, the MITF translocation *(e.g.,* rearrangement and/or fusion) comprises *ACTG1* exon 3 and *MITF* exon 3. In some aspects of the disclosure, the MITF translocation (*e.g*., rearrangement and/or fusion) comprises SEQ ID NO:13 and/or 30. In some aspects of the disclosure, the MITF translocation (*e.g*., rearrangement and/or fusion) comprises SEQ ID NO: 30. In some aspects of the disclosure, the MITF translocation (*e.g*., rearrangement and/or fusion) is detectable by primers which include SEQ ID NO:11 and/or 12. In some aspects of the disclosure, the MITF translocation (*e.g*., rearrangement and/or fusion) is detectable by primers which include SEQ ID NO:9, 10, 11 and/or 12. In some aspects of the disclosure, the MITF translocation (*e.g.,* rearrangement and/or fusion) is driven by the *ACTG1* promoter. In some aspects of the disclosure, the MITF translocation (*e.g*., rearrangement and/or fusion) comprises *AP3S1* and *MITF.* In some aspects of the disclosure, the MITF translocation (*e.g.,* rearrangement and/or fusion) comprises *AP3S1* exon 3. In some aspects of the disclosure, the MITF translocation (*e.g.,* rearrangement and/or fusion) comprises *ACTG1* exon 3 and *MITF* exon 3. In some aspects of the disclosure, the MITF translocation (*e.g*., rearrangement and/or fusion) is driven by the *AP3S1* promoter. In some aspects of the disclosure, the ASP3S1-MITF translocation is present in a clear cell RCC.

In some aspects of the disclosure of any of the methods, the translocation (*e.g*., rearrangement and/or fusion) is a TFEB translocation (*e.g*., rearrangement and/or fusion). In some aspects of the disclosure, the TFEB translocation (*e.g*., rearrangement and/or fusion) comprises *CLTC* and TFEB. In some aspects of the disclosure, the TFEB translocation (*e.g.,* rearrangement and/or fusion) comprises *CLTC* exon 17. In some aspects of the disclosure, the TFEB translocation (*e.g.,* rearrangement and/or fusion) comprises *CLTC* exon 17and *TFEB* exon 6. In some aspects of the disclosure, the TFEB translocation (*e.g*., rearrangement and/or fusion) comprises SEQ ID NO:19. In some aspects of the disclosure, the TFEB translocation (*e.g*., rearrangement and/or fusion) is detectable by primers which include SEQ ID NO:17 and/or 18. In some aspects of the disclosure, the TFEB translocation (*e.g*., rearrangement and/or fusion) is detectable by primers which include SEQ ID NO:15, 16, 17 and/or 18. In some aspects of the disclosure, the TFEB translocation *(e.g.,* rearrangement and/or fusion) is driven by the *CLTC* promoter.

In some aspects of the disclosure of any of the methods, the translocation (*e.g*., rearrangement and/or fusion) is a SBNO2 translocation (*e.g*., rearrangement and/or fusion). In some aspects of the disclosure, the SBNO2 translocation (*e.g*., rearrangement and/or fusion) comprises *MIDN* and *SBNO2.* In some aspects of the disclosure, the SBNO2 (*e.g.,* rearrangement and/or fusion) comprises *MIDN* promoter. In some aspects of the disclosure, the SBNO2 translocation (*e.g.,* rearrangement and/or fusion) comprises *MIDN* promoter and *SBNO2* exon 1. In some aspects of the disclosure, the SBNO2 translocation (*e.g*., rearrangement and/or fusion) comprises SEQ ID NO:25. In some aspects of the disclosure, the SBNO2 translocation (*e.g*., rearrangement and/or fusion) is detectable by primers which include SEQ ID NO:23 and/or 24. In some aspects of the disclosure, the SBNO2 translocation (*e.g*., rearrangement and/or fusion) is detectable by primers which include SEQ ID NO:21, 22, 23, and/or 25. In some aspects of the disclosure, the SBNO2 translocation (*e.g.,* rearrangement and/or fusion) is driven by the *CLTC* promoter.

In some aspects of the disclosure, the MiT (e.g., MITF) translocation (e.g., rearrangement and/or fusion) results in elevated expression levels of MiT (*e.g*., compared to a reference without the MiT translocation). In some aspects of the disclosure, the MiT translocation (*e.g.,* rearrangement and/or fusion) results in elevated activity and/or activation of MiT (*e.g.,* compared to a reference without the MiT translocation).

In some aspects of the disclosure, the MiT (e.g., MITF) translocation (*e.g*., rearrangement and/or fusion) results in elevated expression levels of MET (*e.g*., compared to a reference without the MiT translocation). In some aspects of the disclosure, the MiT translocation (*e.g.,* rearrangement and/or fusion) results in elevated activity and/or activation of MET (*e.g.,* compared to a reference without the MiT translocation).

In some aspects of the disclosure, the MiT (e.g., MITF) translocation (*e.g*., rearrangement and/or fusion) results in elevated expression levels of BIRC7 (*e.g*., compared to a reference without the MiT translocation). In some aspects of the disclosure, the MiT translocation (*e.g.,* rearrangement and/or fusion) results in elevated activity and/or activation of BIRC7 (*e.g.,* compared to a reference without the MiT translocation).

In some aspects of the disclosure, a MiT (e.g., MITF) mutation results in elevated expression levels of MiT (*e.g*., compared to a reference without the MiT translocation). In some aspects of the disclosure, the MiT translocation mutation results in elevated activity and/or activation of MiT (*e.g*., compared to a reference without the MiT translocation). In some aspects of the disclosure, the MITF mutation is one or more of the following: G259A, A260G, A260G, T403G, G426C, A/T (-3), G712A, G1120A. In some aspects of the disclosure, the MITF mutation is one or more of the following: E318K, I212M and E213D 4TΔ2B, L135V, L142F, G244R, D380N.

In some aspects of the disclosure, the MiT (e.g., MITF) mutation results in elevated expression levels of MET (*e.g*., compared to a reference without the MiT translocation). In some aspects of the disclosure, the MiT (e.g., MITF) mutation results in elevated activity and/or activation of MET (*e.g*., compared to a reference without the MiT translocation).

In some aspects of the disclosure, the MiT (e.g., MITF) mutation results in elevated expression levels of BIRC7 (*e.g*., compared to a reference without the MiT translocation). In some aspects of the disclosure, the MiT (e.g., MITF) mutation results in elevated activity and/or activation of BIRC7 (*e.g*., compared to a reference without the MiT translocation).

In some aspects of the disclosure of any of the translocation (*e.g*., rearrangement and/or fusion), the translocation (*e.g.,* rearrangement and/or fusion) is a somatic translocation (*e.g.,* rearrangement and/or fusion). In some aspects of the disclosure, the translocation (*e.g*., rearrangement and/or fusion) is an intra-chromosomal translocation (*e.g*., rearrangement and/or fusion). In some aspects of the disclosure, the translocation (*e.g*., rearrangement and/or fusion) is an inter-chromosomal translocation (*e.g*., rearrangement and/or fusion). In some aspects of the disclosure, the translocation (*e.g*., rearrangement and/or fusion) is an inversion. In some aspects of the disclosure, the translocation (*e.g*., rearrangement and/or fusion) is a deletion. In some aspects of the disclosure, the translocation (*e.g*., rearrangement and/or fusion) is a functional translocation fusion polynucleotide (*e.g*., functional MiT-translocation fusion polynucleotide) and/or functional translocation fusion polypeptide (*e.g*., functional MiT-translocation fusion polypeptide). In some aspects of the disclosure, the functional translocation fusion polypeptide (*e.g*., functional MiT-translocation fusion polypeptide) activates a pathway known to be modulated by one of the translocated genes (*e.g.*, BIRC7 pathway). In some aspects of the disclosure, the methods of determining pathway activation are known in the art and include luciferase reporter assays as described herein.

Examples of cancers and cancer cells include, but are not limited to, carcinoma, lymphoma, blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, melanoma, and leukemia or lymphoid malignancies. More particular examples of such cancers include The method of claim 81, wherein the MiT antagonist binds.

Presence and/or expression levels/amount of a biomarker (e.g., MiT translocation or MiT mutation) can be determined qualitatively and/or quantitatively based on any suitable criterion known in the art, including but not limited to DNA, mRNA, cDNA, proteins, protein fragments and/or gene copy number. In certain aspects of the disclosure, presence and/or expression levels/amount of a biomarker in a first sample is increased as compared to presence/absence and/or expression levels/amount in a second sample. In certain aspects of the disclosure, presence/absence and/or expression levels/amount of a biomarker in a first sample is decreased as compared to presence and/or expression levels/amount in a second sample. In certain aspects of the disclosure, the second sample is a reference sample, reference cell, reference tissue, control sample, control cell, or control tissue. Additional disclosures for determining presence/absence and/or expression levels/amount of a gene are described herein.

In some aspects of the disclosure of any of the methods, elevated expression refers to an overall increase of about any of 10%, 20%, 30%, 40%, 50%, 60%, 70%, 80%, 90%, 95%, 96%, 97%, 98%, 99% or greater, in the level of biomarker (*e.g.,* protein or nucleic acid (*e.g.,* gene or mRNA)), detected by standard art known methods such as those described herein, as compared to a reference sample, reference cell, reference tissue, control sample, control cell, or control tissue. In certain aspects of the disclosure, the elevated expression refers to the increase in expression level/amount of a biomarker in the sample wherein the increase is at least about any of 1.5X, 1.75X, 2X, 3X, 4X, 5X, 6X, 7X, 8X, 9X, 10X, 25X, 50X, 75X, or 100X the expression level/amount of the respective biomarker in a reference sample, reference cell, reference tissue, control sample, control cell, or control tissue. In some aspects of the disclosure, elevated expression refers to an overall increase of greater than about 1.5 fold, about 1.75 fold, about 2 fold, about 2.25 fold, about 2.5 fold, about 2.75 fold, about 3.0 fold, or about 3.25 fold as compared to a reference sample, reference cell, reference tissue, control sample, control cell, control tissue, or internal control (*e.g*., housekeeping gene).

In some aspects of the disclosure of any of the methods, reduced expression refers to an overall reduction of about any of 10%, 20%, 30%, 40%, 50%, 60%, 70%, 80%, 90%, 95%, 96%, 97%, 98%, 99% or greater, in the level of biomarker (*e.g.,* protein or nucleic acid (*e.g.,* gene or mRNA)), detected by standard art known methods such as those described herein, as compared to a reference sample, reference cell, reference tissue, control sample, control cell, or control tissue. In certain aspects of the disclosure, reduced expression refers to the decrease in expression level/amount of a biomarker in the sample wherein the decrease is at least about any of 0.9X, 0.8X, 0.7X, 0.6X, 0.5X, 0.4X, 0.3X, 0.2X, 0.1X, 0.05X, or 0.01X the expression level/amount of the respective biomarker in a reference sample, reference cell, reference tissue, control sample, control cell, or control tissue.

In one aspect, provided are methods for determining MiT translocation expression (determining presence of MiT translocation), comprising the step of determining whether a sample from a patient (e.g., from a patient's cancer) has MiT translocation.

In some aspects of the disclosure of any of the methods, the one or more biomarkers comprise one or more MiT (e.g., MITF, TFEB, TFE3, TFEC, and/or SBNO2). In some aspects of the disclosure, presence of the one or more biomarkers is indicated by the presence of elevated expression levels (e.g., compared to reference) of one or more MiT (e.g., MITF). In some aspects of the disclosure, expression is polypeptide expression. In some aspects of the disclosure, expression is nucleic acid expression. In some aspects of the disclosure, the one or more biomarkers comprises MITF. In some aspects of the disclosure, the one or more biomarkers comprises TFEB. In some aspects of the disclosure, the one or more biomarkers comprises TFE3. In some aspects of the disclosure, the one or more biomarkers comprises TFEC. In some aspects of the disclosure, the one or more biomarkers comprises SBNO2.

In some aspects of the disclosure of any of the methods, the one or more biomarkers comprise MET and/or BIRC7. In some aspects of the disclosure, presence of the one or more biomarkers is indicated by the presence of elevated expression levels (e.g., compared to reference) of MET and/or BIRC7. In some aspects of the disclosure, expression is polypeptide expression. In some aspects of the disclosure, expression is nucleic acid expression. In some aspects of the disclosure, the one or more biomarkers comprises MET. In some aspects of the disclosure, the one or more biomarkers comprises BIRC7.

In some aspects of the disclosure of any of the methods, the one or more biomarkers comprise one or more of MITF, TFEB, TFE3, TFEC, SBNO2, MET and/or BIRC7. In some aspects of the disclosure, presence of the one or more biomarkers is indicated by the presence of elevated expression levels (e.g., compared to reference) of one or more of MITF, TFEB, TFE3, TFEC, SBNO2, MET and/or BIRC7.

In some aspects of the disclosure of any of the methods, the one or more biomarkers comprise a translocation (e.g., rearrangement and/or fusion) of one or more genes selected from MITF, TFEB, TFE3, TFEC, and/or SBNO2. In some aspects of the disclosure, the presence of one or more biomarkers comprises the presence of a translocation (e.g., rearrangement and/or fusion) of one or more genes selected from MITF, TFEB, TFE3, TFEC, and/or SBNO2.

In some aspects of the disclosure of any of the methods, the one or more biomarkers comprise a translocation (e.g., rearrangement and/or fusion) of one or more genes selected from MITF, TFEB, TFE3, TFEC, and/or SBNO2, and one or more of MET and/or BIRC7. In some aspects of the disclosure, the presence of one or more biomarkers comprises the presence of a translocation (e.g., rearrangement and/or fusion) of one or more genes selected from MITF, TFEB, TFE3, TFEC, and/or SBNO2, and overexpression of one or more of MET and/or BIRC7.

In some aspects of the disclosure of any of the methods, the translocation (e.g., rearrangement and/or fusion) is a MITF translocation (e.g., rearrangement and/or fusion). In some aspects of the disclosure, the MITF translocation (e.g., rearrangement and/or fusion) comprises ACTG1 and MITF. In some aspects of the disclosure, the MITF translocation (e.g., rearrangement and/or fusion) comprises ACTG1 exon 3. In some aspects of the disclosure, the MITF translocation (e.g., rearrangement and/or fusion) comprises ACTG1 exon 3 and MITF exon 3. In some aspects of the disclosure, the MITF translocation (e.g., rearrangement and/or fusion) comprises SEQ ID NO:13 and/or 30. In some aspects of the disclosure, the MITF translocation (e.g., rearrangement and/or fusion) comprises SEQ ID NO: 30. In some aspects of the disclosure, the MITF translocation (e.g., rearrangement and/or fusion) is detectable by primers which include SEQ ID NO:11 and/or 12. In some aspects of the disclosure, the MITF translocation (e.g., rearrangement and/or fusion) is detectable by primers which include SEQ ID NO:9, 10, 11 and/or 12. In some aspects of the disclosure, the MITF translocation (e.g., rearrangement and/or fusion) is driven by the ACTG1 promoter. In some aspects of the disclosure, the MITF translocation (e.g., rearrangement and/or fusion) comprises AP3S1 and MITF. In some aspects of the disclosure, the MITF translocation (e.g., rearrangement and/or fusion) comprises AP3S1 exon 3. In some aspects of the disclosure, the MITF translocation (e.g., rearrangement and/or fusion) comprises ACTG1 exon 3 and MITF exon 3. In some aspects of the disclosure, the MITF translocation (e.g., rearrangement and/or fusion) is driven by the AP3S1 promoter. In some aspects of the disclosure, the ASP3S1-MITF translocation is present in a clear cell RCC.

In some aspects of the disclosure of any of the methods, the translocation (e.g., rearrangement and/or fusion) is a TFEB translocation (e.g., rearrangement and/or fusion). In some aspects of the disclosure, the TFEB translocation (e.g., rearrangement and/or fusion) comprises CLTC and TFEB. In some aspects of the disclosure, the TFEB translocation (e.g., rearrangement and/or fusion) comprises CLTC exon 17. In some aspects of the disclosure, the TFEB translocation (e.g., rearrangement and/or fusion) comprises CLTC exon 17and TFEB exon 6. In some aspects of the disclosure, the TFEB translocation (e.g., rearrangement and/or fusion) comprises SEQ ID NO:19. In some aspects of the disclosure, the TFEB translocation (e.g., rearrangement and/or fusion) is detectable by primers which include SEQ ID NO:17 and/or 18. In some aspects of the disclosure, the TFEB translocation (e.g., rearrangement and/or fusion) is detectable by primers which include SEQ ID NO:15, 16, 17 and/or 18. In some aspects of the disclosure, the TFEB translocation (e.g., rearrangement and/or fusion) is driven by the CLTC promoter.

In some aspects of the disclosure of any of the methods, the translocation (e.g., rearrangement and/or fusion) is a SBNO2 translocation (e.g., rearrangement and/or fusion). In some aspects of the disclosure, the SBNO2 translocation (e.g., rearrangement and/or fusion) comprises MIDN and SBNO2. In some aspects of the disclosure, the SBNO2 translocation (e.g., rearrangement and/or fusion) comprises MIDN promoter. In some aspects of the disclosure, the SBNO2 translocation (e.g., rearrangement and/or fusion) comprises MIDN promoter and SBNO2 exon 1. In some aspects of the disclosure, the SBNO2 translocation (e.g., rearrangement and/or fusion) comprises SEQ ID NO:25. In some aspects of the disclosure, the SBNO2 translocation (e.g., rearrangement and/or fusion) is detectable by primers which include SEQ ID NO:23 and/or 24. In some aspects of the disclosure, the SBNO2 translocation (e.g., rearrangement and/or fusion) is detectable by primers which include SEQ ID NO:21, 22, 23, and/or 25. In some aspects of the disclosure, the SBNO2 translocation (e.g., rearrangement and/or fusion) is driven by the CLTC promoter.

In some aspects of the disclosure, the MiT (e.g., MITF) translocation (e.g., rearrangement and/or fusion) results in elevated expression levels of MiT (e.g., compared to a reference without the MiT translocation). In some aspects of the disclosure, the MiT translocation (e.g., rearrangement and/or fusion) results in elevated activity and/or activation of MiT (e.g., compared to a reference without the MiT translocation).

In some aspects of the disclosure, the MiT (e.g., MITF) translocation (e.g., rearrangement and/or fusion) results in elevated expression levels of MET (e.g., compared to a reference without the MiT translocation). In some aspects of the disclosure, the MiT translocation (e.g., rearrangement and/or fusion) results in elevated activity and/or activation of MET (e.g., compared to a reference without the MiT translocation).

In some aspects of the disclosure, the MiT (e.g., MITF) translocation (e.g., rearrangement and/or fusion) results in elevated expression levels of BIRC7 (e.g., compared to a reference without the MiT translocation). In some aspects of the disclosure, the MiT translocation (e.g., rearrangement and/or fusion) results in elevated activity and/or activation of BIRC7 (e.g., compared to a reference without the MiT translocation).

In some aspects of the disclosure, a MiT (e.g., MITF) mutation results in elevated expression levels of MiT (e.g., compared to a reference without the MiT translocation). In some aspects of the disclosure, the MiT translocation mutation results in elevated activity and/or activation of MiT (e.g., compared to a reference without the MiT translocation). In some aspects of the disclosure, the MITF mutation is one or more of the following: G259A, A260G, A260G, T403G, G426C, A/T (-3), G712A, G1120A. In some aspects of the disclosure, the MITF mutation is one or more of the following: E318K, I212M and E213D 4TΔ2B, L135V, L142F, G244R, D380N.

In some aspects of the disclosure, the MiT (e.g., MITF, TFEB, TFE3, TFEC, and/or SBNO2) mutation results in elevated expression levels of MET (e.g., compared to a reference without the MiT translocation). In some aspects of the disclosure, the MiT (e.g., MITF, TFEB, TFE3, TFEC, and/or SBNO2) mutation results in elevated activity and/or activation of MET (e.g., compared to a reference without the MiT translocation).

In some aspects of the disclosure, the MiT (e.g., MITF, TFEB, TFE3, TFEC, and/or SBNO2) mutation results in elevated expression levels of BIRC7 (e.g., compared to a reference without the MiT translocation). In some aspects of the disclosure, the MiT (e.g., MITF, TFEB, TFE3, TFEC, and/or SBNO2) mutation results in elevated activity and/or activation of BIRC7 (e.g., compared to a reference without the MiT translocation).

In some aspects of the disclosure of any of the translocation (e.g., rearrangement and/or fusion), the translocation (e.g., rearrangement and/or fusion) is a somatic translocation (e.g., rearrangement and/or fusion). In some aspects of the disclosure, the translocation (e.g., rearrangement and/or fusion) is an intra-chromosomal translocation (e.g., rearrangement and/or fusion). In some aspects of the disclosure, the translocation (e.g., rearrangement and/or fusion) is an inter-chromosomal translocation (e.g., rearrangement and/or fusion). In some aspects of the disclosure, the translocation (e.g., rearrangement and/or fusion) is an inversion. In some aspects of the disclosure, the translocation (e.g., rearrangement and/or fusion) is a deletion. In some aspects of the disclosure, the translocation (e.g., rearrangement and/or fusion) is a functional translocation fusion polynucleotide (e.g., functional MiT-translocation fusion polynucleotide) and/or functional translocation fusion polypeptide (e.g., functional MiT-translocation fusion polypeptide). In some aspects of the disclosure, the functional translocation fusion polypeptide (e.g., functional MiT-translocation fusion polypeptide) activates a pathway known to be modulated by one of the translocated genes (e.g., BIRC7 pathway). In some aspects of the disclosure, the methods of determining pathway activation are known in the art and include luciferase reporter assays as described herein.

Presence and/or expression level/amount of various biomarkers in a sample can be analyzed by a number of methodologies, many of which are known in the art and understood by the skilled artisan, including, but not limited to, immunohistochemical ("IHC"), Western blot analysis, immunoprecipitation, molecular binding assays, ELISA, ELIFA, fluorescence activated cell sorting ("FACS"), MassARRAY, proteomics, quantitative blood based assays (as for example Serum ELISA), biochemical enzymatic activity assays, in situ hybridization, Southern analysis, Northern analysis, whole genome sequencing, polymerase chain reaction ("PCR") including quantitative real time PCR ("qRT-PCR") and other amplification type detection methods, such as, for example, branched DNA, SISBA, TMA and the like), RNA-Seq, FISH, microarray analysis, gene expression profiling, and/or serial analysis of gene expression ("SAGE"), as well as any one of the wide variety of assays that can be performed by protein, gene, and/or tissue array analysis. Typical protocols for evaluating the status of genes and gene products are found, for example in Ausubel et al., eds., 1995, Current Protocols In Molecular Biology, Units 2 (Northern Blotting), 4 (Southern Blotting), 15 (Immunoblotting) and 18 (PCR Analysis). Multiplexed immunoassays such as those available from Rules Based Medicine or Meso Scale Discovery ("MSD") may also be used.

In some embodiments, presence and/or expression level/amount of a biomarker is determined using a method comprising: (a) performing gene expression profiling, PCR (such as rtPCR), RNA-seq, microarray analysis, SAGE, MassARRAY technique, or FISH on a sample (such as a subject cancer sample); and b) determining presence and/or expression level/amount of a biomarker in the sample. In some aspects of the disclosure, the microarray method comprises the use of a microarray chip having one or more nucleic acid molecules that can hybridize under stringent conditions to a nucleic acid molecule encoding a gene mentioned above or having one or more polypeptides (such as peptides or antibodies) that can bind to one or more of the proteins encoded by the genes mentioned above. In one aspect of the disclosure, the PCR method is qRT-PCR. In one aspect of the disclosure, the PCR method is multiplex-PCR. In some embodiments, gene expression is measured by microarray. In some aspects of the disclosure, gene expression is measured by qRT-PCR. In some aspects of the disclosure, expression is measured by multiplex-PCR.

Methods for the evaluation of mRNAs in cells are well known and include, for example, hybridization assays using complementary DNA probes (such as in situ hybridization using labeled riboprobes specific for the one or more genes, Northern blot and related techniques) and various nucleic acid amplification assays (such as RT-PCR using complementary primers specific for one or more of the genes, and other amplification type detection methods, such as, for example, branched DNA, SISBA, TMA and the like).

Samples from mammals can be conveniently assayed for mRNAs using Northern, dot blot or PCR analysis. In addition, such methods can include one or more steps that allow one to determine the levels of target mRNA in a biological sample (*e.g*., by simultaneously examining the levels a comparative control mRNA sequence of a "housekeeping" gene such as an actin family member). Optionally, the sequence of the amplified target cDNA can be determined.

Optional methods of the invention include protocols which examine or detect mRNAs, such as target mRNAs, in a tissue or cell sample by microarray technologies. Using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. For example, a selection of genes whose expression correlates with increased or reduced clinical benefit of anti-angiogenic therapy may be arrayed on a solid support. Hybridization of a labeled probe with a particular array member indicates that the sample from which the probe was derived expresses that gene.

According to some embodiments, presence and/or expression level/amount is measured by observing protein expression levels of an aforementioned gene. In certain aspects of the disclosure, the method comprises contacting the biological sample with antibodies to a biomarker (e.g., anti-MiT antibody, anti-MiT-translocation antibodies) described herein under conditions permissive for binding of the biomarker, and detecting whether a complex is formed between the antibodies and biomarker. Such method may be an in vitro or in vivo method. In one aspect of the disclosure, an antibody is used to select subjects eligible for therapy with MiT antagonist, in particular MiT-translocation antagonist, *e.g*., a biomarker for selection of individuals.

In certain aspects of the disclosure, the presence and/or expression level/amount of biomarker proteins in a sample is examined using IHC and staining protocols. IHC staining of tissue sections has been shown to be a reliable method of determining or detecting presence of proteins in a sample. In one aspect, expression level of biomarker is determined using a method comprising: (a) performing IHC analysis of a sample (such as a subject cancer sample) with an antibody; and b) determining expression level of a biomarker in the sample. In some aspects of the disclosure, IHC staining intensity is determined relative to a reference value.

IHC may be performed in combination with additional techniques such as morphological staining and/or fluorescence in-situ hybridization. Two general methods of IHC are available; direct and indirect assays. According to the first assay, binding of antibody to the target antigen is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromogenic or fluorogenic substrate is added to provide visualization of the antigen. Signal amplification occurs because several secondary antibodies may react with different epitopes on the primary antibody.

The primary and/or secondary antibody used for IHC typically will be labeled with a detectable moiety. Numerous labels are available which can be generally grouped into the following categories: (a) Radioisotopes, such as 35S, 14C, 1251, 3H, and 1311; (b) colloidal gold particles; (c) fluorescent labels including, but are not limited to, rare earth chelates (europium chelates), Texas Red, rhodamine, fluorescein, dansyl, Lissamine, umbelliferone, phycocrytherin, phycocyanin, or commercially available fluorophores such SPECTRUM ORANGE7 and SPECTRUM GREEN7 and/or derivatives of any one or more of the above; (d) various enzyme-substrate labels are available and U.S. Patent No. 4,275,149 provides a review of some of these. Examples of enzymatic labels include luciferases (*e.g*., firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (*e.g*., glucose oxidase, galactose oxidase, and glucose-6-phosphate dehydrogenase), heterocyclic oxidases (such as uricase and xanthine oxidase), lactoperoxidase, microperoxidase, and the like.

Examples of enzyme-substrate combinations include, for example, horseradish peroxidase (HRPO) with hydrogen peroxidase as a substrate; alkaline phosphatase (AP) with para-Nitrophenyl phosphate as chromogenic substrate; and β-D-galactosidase (β-D-Gal) with a chromogenic substrate (*e.g.*, p-nitrophenyl-β-D-galactosidase) or fluorogenic substrate (*e.g.*, 4-methylumbelliferyl-β-D-galactosidase). For a general review of these, *see* U.S. Patent Nos. 4,275,149 and 4,318,980.

Specimens thus prepared may be mounted and coverslipped. Slide evaluation is then determined, *e.g*., using a microscope, and staining intensity criteria, routinely used in the art, may be employed. In some aspects of the disclosure, a staining pattern score of about 1+ or higher is diagnostic and/or prognostic. In certain aspects of the disclosure, a staining pattern score of about 2+ or higher in an IHC assay is diagnostic and/or prognostic. In other aspects of the disclosure, a staining pattern score of about 3 or higher is diagnostic and/or prognostic. In one aspect of the disclosure, it is understood that when cells and/or tissue from a tumor or colon adenoma are examined using IHC, staining is generally determined or assessed in tumor cell and/or tissue (as opposed to stromal or surrounding tissue that may be present in the sample).

In alternative methods, the sample may be contacted with an antibody specific for said biomarker (*e.g*., anti-MiT antibody, anti-MiT- translocation antibody) under conditions sufficient for an antibody-biomarker complex to form, and then detecting said complex. The presence of the biomarker may be detected in a number of ways, such as by Western blotting and ELISA procedures for assaying a wide variety of tissues and samples, including plasma or serum. A wide range of immunoassay techniques using such an assay format are available, *see*, *e.g.*, U.S. Pat. Nos. 4,016,043, 4,424,279 and 4,018,653. These include both single-site and two-site or "sandwich" assays of the non-competitive types, as well as in the traditional competitive binding assays. These assays also include direct binding of a labeled antibody to a target biomarker.

Exemplary MITF antibodies include c5 (abcam), D5 (abeam), HPA003259 (Sigma), and anti-MITF antibodies are further described and exemplified herein. Exemplary TFEB antibodies include ab113372 (abeam), ab113372 (abeam), LS-B5907 (LSBio), and further anti-TFEB antibodies are described and exemplified herein. Exemplary TFEC antibodies include 13547-1-AP (proteinTech), and further anti-TFEC antibodies are described and exemplified herein. Exemplary TFE3 antibodies include MRQ-37 (Ventana), HPA023881 (Sigma), and further anti-TFE3 antibodies are described and exemplified herein. Exemplary MET antibodies include SP44 (Ventana), Met4. Exemplary BIRC7 antibodies include 1D12 (OriGene).

Presence and/or expression level/amount of a selected biomarker in a tissue or cell sample may also be examined by way of functional or activity-based assays. For instance, if the biomarker is an enzyme, one may conduct assays known in the art to determine or detect the presence of the given enzymatic activity in the tissue or cell sample.

In certain aspects of the disclosure, the samples are normalized for both differences in the amount of the biomarker assayed and variability in the quality of the samples used, and variability between assay runs. Such normalization may be accomplished by detecting and incorporating the expression of certain normalizing biomarkers, including well known housekeeping genes, such as ACTB. Alternatively, normalization can be based on the mean or median signal of all of the assayed genes or a large subset thereof (global normalization approach). On a gene-by-gene basis, measured normalized amount of a subject tumor mRNA or protein is compared to the amount found in a reference set. Normalized expression levels for each mRNA or protein per tested tumor per subject can be expressed as a percentage of the expression level measured in the reference set. The presence and/or expression level/amount measured in a particular subject sample to be analyzed will fall at some percentile within this range, which can be determined by methods well known in the art.

In certain aspects of the disclosure, relative expression level of a gene is determined as follows:

Relative expression gene1 sample1 = 2 exp (Ct housekeeping gene - Ct gene1) with Ct determined in a sample.

Relative expression gene1 reference RNA = 2 exp (Ct housekeeping gene - Ct gene1) with Ct determined in the reference sample.

Normalized relative expression gene1 sample1 = (relative expression gene1 sample1 / relative expression gene1 reference RNA) x 100

Ct is the threshold cycle. The Ct is the cycle number at which the fluorescence generated within a reaction crosses the threshold line.

All experiments are normalized to a reference RNA, which is a comprehensive mix of RNA from various tissue sources (*e.g*., reference RNA #636538 from Clontech, Mountain View, CA). Identical reference RNA is included in each qRT-PCR run, allowing comparison of results between different experimental runs.

In one embodiment, the sample is a clinical sample. In another embodiment, the sample is used in a diagnostic assay. In some embodiments, the sample is obtained from a primary or metastatic tumor. Tissue biopsy is often used to obtain a representative piece of tumor tissue. Alternatively, tumor cells can be obtained indirectly in the form of tissues or fluids that are known or thought to contain the tumor cells of interest. For instance, samples of lung cancer lesions may be obtained by resection, bronchoscopy, fine needle aspiration, bronchial brushings, or from sputum, pleural fluid or blood. Genes or gene products can be detected from cancer or tumor tissue or from other body samples such as urine, sputum, serum or plasma. The same techniques discussed above for detection of target genes or gene products in cancerous samples can be applied to other body samples. Cancer cells may be sloughed off from cancer lesions and appear in such body samples. By screening such body samples, a simple early diagnosis can be achieved for these cancers. In addition, the progress of therapy can be monitored more easily by testing such body samples for target genes or gene products.

In certain aspects of the disclosure, a reference sample, reference cell, reference tissue, control sample, control cell, or control tissue is a single sample or combined multiple samples from the same subject or individual that are obtained at one or more different time points than when the test sample is obtained. For example, a reference sample, reference cell, reference tissue, control sample, control cell, or control tissue is obtained at an earlier time point from the same subject or individual than when the test sample is obtained. Such reference sample, reference cell, reference tissue, control sample, control cell, or control tissue may be useful if the reference sample is obtained during initial diagnosis of cancer and the test sample is later obtained when the cancer becomes metastatic.

In certain aspects of the disclosure, a reference sample, reference cell, reference tissue, control sample, control cell, or control tissue is a combined multiple samples from one or more healthy individuals who are not the subject or individual. In certain aspects of the disclosure, a reference sample, reference cell, reference tissue, control sample, control cell, or control tissue is a combined multiple samples from one or more individuals with a disease or disorder (*e.g*., cancer) who are not the subject or individual. In certain aspects of the disclosure, a reference sample, reference cell, reference tissue, control sample, control cell, or control tissue is pooled RNA samples from normal tissues or pooled plasma or serum samples from one or more individuals who are not the subject or individual. In certain aspects of the disclosure, a reference sample, reference cell, reference tissue, control sample, control cell, or control tissue is pooled RNA samples from tumor tissues or pooled plasma or serum samples from one or more individuals with a disease or disorder (*e.g.*, cancer) who are not the subject or individual.

In some aspects of the disclosure of any of the methods, the MiT antagonist is a MiT-translocation antagonist. In some aspects of the disclosure of any of the methods, the MiT antagonist in particular MiT-translocation antagonist is an antibody, binding polypeptide, binding small molecule, or polynucleotide. In some aspects of the disclosure, the MiT antagonist in particular MiT-translocation antagonist is an antibody. In some aspects of the disclosure, the antibody is a monoclonal antibody. In some aspects of the disclosure, the antibody is a human, humanized, or chimeric antibody. In some aspects of the disclosure, the antibody is an antibody fragment and the antibody fragment binds MiT polypeptide in particular MiT antagonist and/or MiT-translocation fusion polypeptide.

In some aspects of the disclosure of any of the methods, the individual according to any of the above aspects of the disclosure may be a human.

In some aspects of the disclosure of any of the methods, the method comprises administering to an individual having such cancer an effective amount of a MiT antagonist in particular MiT -translocation antagonist. In one such aspect of the disclosure, the method further comprises administering to the individual an effective amount of at least one additional therapeutic agent, as described below. In some aspects of the disclosure, the individual may be a human.

The MiT antagonist, in particular MiT-translocation antagonist, described herein can be used either alone or in combination with other agents in a therapy. For instance, a MiT antagonist, in particular MiT-translocation antagonist, described herein may be co-administered with at least one additional therapeutic agent including another MiT antagonist. In certain aspects of the disclosure, an additional therapeutic agent is a chemotherapeutic agent.

Such combination therapies noted above encompass combined administration (where two or more therapeutic agents are included in the same or separate formulations), and separate administration, in which case, administration of the MiT antagonist, in particular MiT-translocation antagonist, can occur prior to, simultaneously, and/or following, administration of the additional therapeutic agent and/or adjuvant. MiT antagonist, in particular MiT-translocation antagonist, can also be used in combination with radiation therapy.

A MiT antagonist, in particular MiT-translocation antagonist (*e.g*., an antibody, binding polypeptide, and/or small molecule) described herein (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, *e.g*., by injections, such as intravenous or subcutaneous injections, depending in part on whether the administration is brief or chronic. Various dosing schedules including but not limited to single or multiple administrations over various time-points, bolus administration, and pulse infusion are contemplated herein.

MiT antagonist, in particular MiT antagonist (*e.g*., an antibody, binding polypeptide, and/or small molecule) described herein may be formulated, dosed, and administered in a fashion consistent with good medical practice. Factors for consideration in this context include the particular disorder being treated, the particular mammal being treated, the clinical condition of the individual, the cause of the disorder, the site of delivery of the agent, the method of administration, the scheduling of administration, and other factors known to medical practitioners. The MiT antagonist, in particular MiT antagonist, need not be, but is optionally formulated with one or more agents currently used to prevent or treat the disorder in question. The effective amount of such other agents depends on the amount of the MiT antagonist, in particular MiT antagonist, present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of a MiT antagonist, in particular MiT antagonist, described herein (when used alone or in combination with one or more other additional therapeutic agents) will depend on the type of disease to be treated, the severity and course of the disease, whether the MiT antagonist, in particular MiT antagonist, is administered for preventive or therapeutic purposes, previous therapy, the subject's clinical history and response to the MiT antagonist, and the discretion of the attending physician. The MiT antagonist, in particular MiT antagonist, is suitably administered to the individual at one time or over a series of treatments. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. Such doses may be administered intermittently, *e.g*., every week or every three weeks (*e.g*., such that the individual receives from about two to about twenty, or *e.g*., about six doses of the MiT antagonist). An initial higher loading dose, followed by one or more lower doses may be administered. An exemplary dosing regimen comprises administering. However, other dosage regimens may be useful. The progress of this therapy is easily monitored by conventional techniques and assays.

It is understood that any of the above formulations or therapeutic methods may be carried out using an immunoconjugate of the invention in place of or in addition to the MiT antagonist, in particular MiT antagonist.

### III. Therapeutic Compositions

Provided herein are MiT antagonists useful in the methods described herein. In some aspects of the disclosure, the MiT antagonists are an antibody, binding polypeptide, binding small molecule, and/or polynucleotide. In some aspects of the disclosure, the MiT antagonists are MET pathway antagonists. In some aspects of the disclosure, the MiT antagonists are BIRC7 antagonists.

### Antibody antagonists

In some aspects of the disclosure, the MET pathway antagonist is an antibody. In one aspect of the disclosure, the medicament is an antibody, including but not limited to an antibody which binds to human c-met. In some aspects of the disclosure, the antibody interferes with (e.g., blocks) c-met binding to hepatocyte growth factor (HGF). In some aspects of the disclosure, the antibody binds to c-met. In some aspects of the disclosure, the antibody binds to HGF. In one aspect of the disclosure, the anti-c-met antibody is onartuzumab. Other anti-c-met antibodies suitable for use in the methods of the invention are described herein and known in the art. For example, anti-c-met antibodies disclosed in WO05/016382 (including but not limited to antibodies 13.3.2, 9.1.2, 8.70.2, 8.90.3); an anti-c-met antibodies produced by the hybridoma cell line deposited with ICLC number PD 03001 at the CBA in Genoa, or that recognizes an epitope on the extracellular domain of the β chain of the HGF receptor, and said epitope is the same as that recognized by the monoclonal antibody); anti-c-met antibodies disclosed in WO2007/126799 (including but not limited to 04536, 05087, 05088, 05091, 05092, 04687, 05097, 05098, 05100, 05101, 04541, 05093, 05094, 04537, 05102, 05105, 04696, 04682); anti c-met antibodies disclosed in WO2009/007427 (including but not limited to an antibody deposited at CNCM, Institut Pasteur, Paris, France, on March 14, 2007 under the number I-3731, on March 14, 2007 under the number 1-3732, on July 6, 2007 under the number I-3786, on March 14, 2007 under the number I-3724; an anti-c-met antibody disclosed in 20110129481; an anti-c-met antibody disclosed in US20110104176; an anti-c-met antibody disclosed in WO2009/134776; an anti-c-met antibody disclosed in WO2010/059654; an anti-c-met antibody disclosed in WO2011020925 (including but not limited to an antibody secreted from a hybridoma deposited at the CNCM, Institut Pasteur, Paris, France, on March 12, 2008 under the number I-3949 and the hybridoma deposited on January 14, 2010 under the number I-4273).

In some aspects of the disclosure, the MET pathway antagonist is an anti-hepatocyte growth factor (HGF) antibody, including but not limited to, humanized anti-HGF antibody TAK701, rilotumumab, Ficlatuzumab, and/or humanized antibody 2B8 described in WO2007/143090. In some aspects of the disclosure, the anti-HGF antibody is an anti-HGF antibody described in US7718174B2.

In some aspects of the disclosure, the BIRC7 antagonist is an anti-BIRC7 antibody. Exemplary antibodies are known in the art.

In a further aspect, antibody aspects of the disclosure, in particular, according to any of the above aspects of the disclosure, may incorporate any of the features, singly or in combination, as described in Sections below:

### Antibody Affinity

In certain aspects of the disclosure, an antibody provided herein has a dissociation constant (Kd) of ≤ 1µM. In one aspect of the disclosure, Kd is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (*see*, *e.g*., Chen et al., J. Mol. Biol. 293:865-881(1999)). To establish conditions for the assay, MICROTITER® multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [¹²⁵I]-antigen are mixed with serial dilutions of a Fab of interest (*e.g*., consistent with assessment of the anti-VEGF antibody, Fab-12, in Presta et al., Cancer Res. 57:4593-4599 (1997)). The Fab of interest is then incubated overnight; however, the incubation may continue for a longer period (*e.g*., about 65 hours) to ensure that equilibrium is reached. Thereafter, the mixtures are transferred to the capture plate for incubation at room temperature (*e.g*., for one hour). The solution is then removed and the plate washed eight times with 0.1% polysorbate 20 (TWEEN-20®) in PBS. When the plates have dried, 150 µl/well of scintillant (MICROSCINT-20™; Packard) is added, and the plates are counted on a TOPCOUNT™ gamma counter (Packard) for ten minutes. Concentrations of each Fab that give less than or equal to 20% of maximal binding are chosen for use in competitive binding assays.

According to another aspect of the disclosure, Kd is measured using surface plasmon resonance assays using a BIACORE®-2000 or a BIACORE®-3000 (BIAcore, Inc., Piscataway, NJ) at 25°C with immobilized antigen CM5 chips at ∼10 response units (RU). Briefly, carboxymethylated dextran biosensor chips (CM5, BIACORE, Inc.) are activated with *N*-ethyl-*N'-*(3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 µg/ml (∼0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 µl/min. Association rates (kₒₙ) and dissociation rates (k_{off}) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio k_{off}/kₒₙ. *See*, *e.g*., Chen et al., J. Mol. Biol. 293:865-881 (1999). If the on-rate exceeds 10⁶M⁻¹s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO™ spectrophotometer (ThermoSpectronic) with a stirred cuvette.

### Antibody Fragments

In certain aspects of the disclosure, an antibody provided herein is an antibody fragment. Antibody fragments include, but are not limited to, Fab, Fab', Fab'-SH, F(ab')₂, Fv, and scFv fragments, and other fragments described below. For a review of certain antibody fragments, *see* Hudson et al., Nat. Med. 9:129-134 (2003). For a review of scFv fragments, *see*, *e.g*., Pluckthün, in The Pharmacology of Monoclonal Antibodies, vol. 113, Rosenburg and Moore eds., (Springer-Verlag, New York), pp. 269-315 (1994); *see* also WO 93/16185; and U.S. Patent Nos. 5,571,894 and 5,587,458. For discussion of Fab and F(ab')₂ fragments comprising salvage receptor binding epitope residues and having increased *in vivo* half-life, *see* U.S. Patent No. 5,869,046.

Diabodies are antibody fragments with two antigen-binding sites that may be bivalent or bispecific. *See*, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain aspects of the disclosure, a single-domain antibody is a human single-domain antibody (Domantis, Inc., Waltham, MA; *see*, *e.g.*, U.S. Patent No. 6,248,516 B1).

Antibody fragments can be made by various techniques, including but not limited to proteolytic digestion of an intact antibody as well as production by recombinant host cells (*e.g.*, *E. coli* or phage), as described herein.

### Chimeric and Humanized Antibodies

In certain aspects of the disclosure, an antibody provided herein is a chimeric antibody. Certain chimeric antibodies are described, *e.g*., in U.S. Patent No. 4,816,567; and Morrison et al., Proc. Natl. Acad. Sci. USA, 81:6851-6855 (1984)). In one example, a chimeric antibody comprises a non-human variable region (*e.g.*, a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain aspects of the disclosure, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, *e.g.*, CDRs, (or portions thereof) are derived from a non-human antibody, and FRs (or portions thereof) are derived from human antibody sequences. A humanized antibody optionally will also comprise at least a portion of a human constant region. In some aspects of the disclosure, some FR residues in a humanized antibody are substituted with corresponding residues from a non-human antibody (*e.g*., the antibody from which the HVR residues are derived), *e.g*., to restore or improve antibody specificity or affinity.

Humanized antibodies and methods of making them are reviewed, *e.g*., in Almagro and Fransson, Front. Biosci. 13:1619-1633 (2008), and are further described, *e.g.*, in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (*see*, *e.g*., Sims et al., J. Immunol. 151:2296 (1993)); framework regions derived from the consensus sequence of human antibodies of a particular subgroup of light or heavy chain variable regions (*see, e.g.*, Carter et al., Proc. Natl. Acad. Sci. USA, 89:4285 (1992); and Presta et al., J. Immunol., 151:2623 (1993)); human mature (somatically mutated) framework regions or human germline framework regions (*see, e.g.*, Almagro and Fransson, Front. Biosci. 13:1619-1633 (2008)); and framework regions derived from screening FR libraries (*see*, *e.g.*, Baca et al., J. Biol. Chem. 272:10678-10684 (1997) and Rosok et al., J. Biol. Chem. 271:22611-22618 (1996)).

### Human Antibodies

In certain aspects of the disclosure, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, *see* Lonberg, Nat. Biotech. 23:1117-1125 (2005). *See also*, *e.g*., U.S. Patent Nos. 6,075,181 and 6,150,584 describing XENOMOUSE™ technology; U.S. Patent No. 5,770,429 describing HuMab® technology; U.S. Patent No. 7,041,870 describing K-M MOUSE® technology, and U.S. Patent Application Publication No. US 2007/0061900, describing VelociMouse® technology). Human variable regions from intact antibodies generated by such animals may be further modified, *e.g*., by combining with a different human constant region.

Human antibodies can also be made by hybridoma-based methods. Human myeloma and mouse-human heteromyeloma cell lines for the production of human monoclonal antibodies have been described. (*See*, *e.g*., Kozbor J. Immunol., 133: 3001 (1984); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clin. Pharma., 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### Library-Derived Antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, *e.g.*, in Hoogenboom et al., in METHODS IN MOL. BIOL. 178:1-37 (O'Brien et al., ed., Human Press, Totowa, NJ, 2001) and further described, *e.g.*, in the McCafferty et al., Nature 348:552-554; Clackson et al., Nature 352: 624-628 (1991); Marks et a/., J. Mol. Biol. 222: 581-597 (1992); Marks and Bradbury, in METHODS IN MOL. BIOL. 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (*e.g*., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement in vitro, as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain aspects of the disclosure, an antibody provided herein is a multispecific antibody, *e.g*., a bispecific antibody. Multispecific antibodies are monoclonal antibodies that have binding specificities for at least two different sites. In certain aspects of the disclosure, one of the binding specificities is for MiT polypeptide such as an R-spondin-translocation fusion polypeptide and the other is for any other antigen. In certain aspects of the disclosure, bispecific antibodies may bind to two different epitopes of MiT polypeptide such as an R-spondin-translocation fusion polypeptide. Bispecific antibodies may also be used to localize cytotoxic agents to cells which express MiT polypeptide such as an R-spondin-translocation fusion polypeptide. Bispecific antibodies can be prepared as full length antibodies or antibody fragments.

Techniques for making multispecific antibodies include, but are not limited to, recombinant co-expression of two immunoglobulin heavy chain-light chain pairs having different specificities (*see* Milstein and Cuello, Nature 305: 537 (1983)), WO 93/08829, and Traunecker et al., EMBO J. 10: 3655 (1991)), and "knob-in-hole" engineering (*see*, *e.g.*, U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (*see*, *e.g.*, US Patent No. 4,676,980, and Brennan et al., Science, 229: 81 (1985)); using leucine zippers to produce bi-specific antibodies (*see, e.g.*, Kostelny et al., J. Immunol., 148(5):1547-1553 (1992)); using "diabody" technology for making bispecific antibody fragments (*see*, *e.g.*, Hollinger et al., Proc. Natl. Acad. Sci. USA, 90:6444-6448 (1993)); and using single-chain Fv (sFv) dimers (*see*, *e.g.*, Gruber et al., J. Immunol., 152:5368 (1994)); and preparing trispecific antibodies as described, *e.g.*, in Tutt et al., J. Immunol. 147: 60 (1991).

Engineered antibodies with three or more functional antigen binding sites, including "Octopus antibodies," are also included herein (*see*, *e.g.*, US 2006/0025576).

The antibody or fragment herein also includes a "Dual Acting FAb" or "DAF" comprising an antigen binding site that binds to a MiT polypeptide such as an R-spondin-translocation fusion polypeptide as well as another, different antigen (*see*, US 2008/0069820, for example).

### 7. Antibody Variants

### Glycosylation variants

In certain aspects of the disclosure, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. *See*, *e.g.*, Wright et al., TIBTECH 15:26-32 (1997). The oligosaccharide may include various carbohydrates, *e.g*., mannose, N-acetyl glucosamine (GlcNAc), galactose, and sialic acid, as well as a fucose attached to a GlcNAc in the "stem" of the biantennary oligosaccharide structure. In some aspects of the disclosure, modifications of the oligosaccharide in an antibody of the invention may be made in order to create antibody variants with certain improved properties.

In one aspect of the disclosure, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e.g., complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, *i.e*., between positions 294 and 300, due to minor sequence variations in antibodies. Such fucosylation variants may have improved ADCC function. *See*, *e.g.*, US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al., J. Mol. Biol. 336:1239-1249 (2004); Yamane-Ohnuki et al., Biotech. Bioeng. 87: 614 (2004). Examples of cell lines capable of producing defucosylated antibodies include Lec13 CHO cells deficient in protein fucosylation (Ripka et al., Arch. Biochem. Biophys. 249:533-545 (1986); US 2003/0157108, Presta, L; and WO 2004/056312, Adams et al.*,* especially at Example 11), and knockout cell lines, such as alpha-1,6-fucosyltransferase gene, FUT8, knockout CHO cells (*see*, *e.g.*, Yamane-Ohnuki et al., Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, *e.g*., in which a biantennary oligosaccharide attached to the Fc region of the antibody is bisected by GlcNAc. Such antibody variants may have reduced fucosylation and/or improved ADCC function. Examples of such antibody variants are described, *e.g.*, in WO 2003/011878 (Jean-Mairet et al.); US Patent No. 6,602,684 (Umana et al.); and US 2005/0123546 (Umana et al.)*.* Antibody variants with at least one galactose residue in the oligosaccharide attached to the Fc region are also provided. Such antibody variants may have improved CDC function. Such antibody variants are described, *e.g.,* in WO 1997/30087 (Patel et al.); WO 1998/58964 (Raju, S.); and WO 1999/22764 (Raju, S.).

### Fc region variants

In certain aspects of the disclosure, one or more amino acid modifications may be introduced into the Fc region of an antibody provided herein, thereby generating an Fc region variant. The Fc region variant may comprise a human Fc region sequence (*e.g*., a human IgG1, IgG2, IgG3 or IgG4 Fc region) comprising an amino acid modification (*e.g*., a substitution) at one or more amino acid positions.

In certain aspects of the disclosure, the invention contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express Fc(RIII only, whereas monocytes express Fc(RI, Fc(RII and Fc(RIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (*see*, *e.g*., Hellstrom, I. et al., Proc. Nat'l Acad. Sci. USA 83:7059-7063 (1986)) and Hellstrom, I et al., Proc. Nat'l Acad. Sci. USA 82:1499-1502 (1985); 5,821,337 (*see* Bruggemann, M. et al., J. Exp. Med. 166:1351-1361 (1987)). Alternatively, non-radioactive assays methods maybe employed (*see*, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo*, *e.g*., in a animal model such as that disclosed in Clynes et al., Proc. Natl. Acad. Sci. USA 95:652-656 (1998). C1q binding assays may also be carried out to confirm that the antibody is unable to bind C1q and hence lacks CDC activity. *See*, *e.g*., C1q and C3c binding ELISA in WO 2006/029879 and WO 2005/100402. To assess complement activation, a CDC assay may be performed (*see*, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (*see, e.g.*, Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (*See, e.g*., U.S. Patent No. 6,737,056; WO 2004/056312, and Shields et al., J. Biol. Chem. 9(2): 6591-6604 (2001).) In certain aspects of the disclosure, an antibody variant comprises an Fc region with one or more amino acid substitutions which improve ADCC, *e.g*., substitutions at positions 298, 333, and/or 334 of the Fc region (EU numbering of residues). In some aspects of the disclosure, alterations are made in the Fc region that result in altered (*i.e*., either improved or diminished) C1q binding and/or Complement Dependent Cytotoxicity (CDC), *e.g.*, as described in US Patent No. 6,194,551, WO 99/51642, and Idusogie et al., J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, *e.g*., substitution of Fc region residue 434 (US Patent No. 7,371,826). *See also* Duncan & Winter, Nature 322:738-40 (1988); U.S. Patent No. 5,648,260; U.S. Patent No. 5,624,821; and WO 94/29351 concerning other examples of Fc region variants.

### Cysteine engineered antibody variants

In certain aspects of the disclosure, it may be desirable to create cysteine engineered antibodies, *e.g*., "thioMAbs," in which one or more residues of an antibody are substituted with cysteine residues. In particular aspects of the disclosure, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain aspects of the disclosure, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies maybe generated as described, *e.g*., in U.S. Patent No. 7,521,541.

### Immunoconjugates

Further provided herein are immunoconjugates comprising an anti-MiT antibody such as an R-spondin-translocation fusion polypeptide herein conjugated to one or more cytotoxic agents, such as chemotherapeutic agents or drugs, growth inhibitory agents, toxins (*e.g*., protein toxins, enzymatically active toxins of bacterial, fungal, plant, or animal origin, or fragments thereof), or radioactive isotopes.

In one aspect of the disclosure, an immunoconjugate is an antibody-drug conjugate (ADC) in which an antibody is conjugated to one or more drugs, including but not limited to a maytansinoid (*see* U.S. Patent Nos. 5,208,020, 5,416,064 and European Patent EP 0 425 235 B1); an auristatin such as monomethylauristatin drug moieties DE and DF (MMAE and MMAF) (*see* U.S. Patent Nos. 5,635,483 and 5,780,588, and 7,498,298); a dolastatin; a calicheamicin or derivative thereof (*see* U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (*see* Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another aspect of the disclosure, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another aspect of the disclosure, an immunoconjugate comprises an antibody as described herein conjugated to a radioactive atom to form a radioconjugate. A variety of radioactive isotopes are available for the production of radioconjugates. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the radioconjugate is used for detection, it may comprise a radioactive atom for scintigraphic studies, for example Tc⁹⁹ or I¹²³, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. *See* WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (*e.g*., from Pierce Biotechnology, Inc., Rockford, IL., U.S.A).

### C. Binding Polypeptides

Provided herein are MiT binding polypeptide antagonists for use as a MiT antagonist in any of the methods described herein. MiT binding polypeptide antagonists are polypeptides that bind, preferably specifically, to a MiT polypeptide. In some aspects of the disclosure of any of the MiT binding polypeptide antagonists, the MiT binding polypeptide antagonist is a chimeric polypeptide.

In some aspects of the disclosure of any of the binding polypeptides, the MiT binding polypeptide antagonist is a MITF binding polypeptide antagonist. In some aspect of the disclosure, the MiT binding polypeptide antagonist is a MITF-translocation binding polypeptide antagonist. In some aspects of the disclosure of any of the binding polypeptides, the MiT binding polypeptide antagonist is a TFEB binding polypeptide antagonist. In some aspects of the disclosure, the MiT binding polypeptide antagonist is a TFEB -translocation binding polypeptide antagonist. In some aspects of the disclosure of any of the binding polypeptides, the MiT binding polypeptide antagonist is a TFEC binding polypeptide antagonist. In some aspects of the disclosure, the MiT small molecule antagonist is a TFEC -translocation binding polypeptide antagonist. In some aspects of the disclosure of any of the small molecules, the MiT binding polypeptide antagonist is a TFE3 binding polypeptide antagonist. In some aspect of the disclosure, the MiT binding polypeptide antagonist is a TFE3 -translocation binding polypeptide antagonist.

In some aspects of the disclosure of any of the binding polypeptides, the binding polypeptide binds to a MITF-translocation fusion polypeptide. In some aspects of the disclosure, binding polypeptide specifically binds a MITF-translocation fusion polypeptide, but do not substantially bind wild-type MITF and/or a second gene of the translocation. In some aspects of the disclosure, the MITF-translocation fusion polypeptide comprises *ACTG1* and *MITF.* In some aspects of the disclosure, the MITF translocation (*e.g*., rearrangement and/or fusion) comprises *ACTG1* exon 3. In some aspects of the disclosure, the MITF translocation (*e.g.*, rearrangement and/or fusion) comprises *ACTG1* exon 3 and *MITF* exon 3. In some aspects of the disclosure, the MITF-translocation fusion polypeptide comprises SEQ ID NO:30 and/or 14. In some aspects of the disclosure, the MITF translocation (*e.g.*, rearrangement and/or fusion) comprises *AP3S1* and *MITF.* In some aspects of the disclosure, the MITF translocation (*e.g*., rearrangement and/or fusion) comprises *AP3S1* exon 3. In some aspects of the disclosure, the MITF translocation (*e.g.*, rearrangement and/or fusion) comprises *ACTG1* exon 3 and *MITF* exon 3. In some aspects of the disclosure, the MITF translocation (*e.g*., rearrangement and/or fusion) is driven by the *AP3S1* promoter.

In some aspects of the disclosure of any of the binding polypeptides, the binding polypeptide binds to a TFEB-translocation fusion polypeptide. In some aspects of the disclosure, binding polypeptide specifically binds a TFEB-translocation fusion polypeptide, but do not substantially bind wild-type TFEC and/or a second gene of the translocation. In some aspects of the disclosure, the TFEB -translocation fusion polypeptide comprises *CLTC* and *TFEB.* In some aspects of the disclosure, the TFEB translocation (*e.g.*, rearrangement and/or fusion) comprises *CLTC* exon 17. In some aspects of the disclosure, the TFEB translocation (*e.g*., rearrangement and/or fusion) comprises *CLTC* exon 17and *TFEB* exon 6. In some aspects of the disclosure, the SBNO2 translocation (*e.g.*, rearrangement and/or fusion) comprises MIDN promoter and *SBNO2* exon 1. In some aspects of the disclosure, the MITF-translocation fusion polypeptide comprises SEQ ID NO:20 and/or 31.

In some aspects of the disclosure of any of the binding polypeptides, the binding polypeptide binds to a TFEC-translocation fusion polypeptide. In some aspects of the disclosure, binding polypeptide specifically binds a TFEC-translocation fusion polypeptide, but do not substantially bind wild-type TFEC and/or a second gene of the translocation.

In some aspects of the disclosure of any of the binding polypeptides, the binding polypeptide binds to a TFE3-translocation fusion polypeptide. In some aspects of the disclosure, binding polypeptide specifically binds a TFE3-translocation fusion polypeptide, but do not substantially bind wild-type TFE3 and/or a second gene of the translocation.

In some aspects of the disclosure, the MiT binding polypeptide antagonist is a MET pathway binding polypeptide antagonist. For example, C-met receptor molecules or fragments thereof that specifically bind to HGF can be used in the methods of the invention, e.g., to bind to and sequester the HGF protein, thereby preventing it from signaling. Preferably, the c-met receptor molecule, or HGF binding fragment thereof, is a soluble form. In some aspects of the disclosure, a soluble form of the receptor exerts an inhibitory effect on the biological activity of the c-met protein by binding to HGF, thereby preventing it from binding to its natural receptors present on the surface of target cells. Also included are c-met receptor fusion proteins, examples of which are described below. In some aspects of the disclosure, the MET pathway binding polypeptide antagonist comprises a soluble form of the c-met receptor, including a chimeric receptor protein. HGF molecules or fragments thereof that specifically bind to c-met and block or reduce activation of c-met, thereby preventing it from signaling, can be used in the methods of the invention.

In some aspects of the disclosure, the MiT binding polypeptide antagonist is a BIRC7 binding polypeptide antagonist. In one aspect of the disclosure the BIRC7 binding polypeptide antagonist comprises EERTCKVCLDRAVSIVFVPCGHLVCAECAPGLQLCPICRAPVRSRVRTFL (SEQ ID NO: 32). In one aspect of the disclosure the BIRC7 binding polypeptide antagonist comprises CRAPVRSRVRTFLS (SEQ ID NO:33).

Binding polypeptides may be chemically synthesized using known polypeptide synthesis methodology or may be prepared and purified using recombinant technology. Binding polypeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such binding polypeptides that are capable of binding, preferably specifically, to a target, MiT polypeptide, as described herein. Binding polypeptides may be identified without undue experimentation using well known techniques. In this regard, it is noted that techniques for screening polypeptide libraries for binding polypeptides that are capable of specifically binding to a polypeptide target are well known in the art (*see*, *e.g*., U.S. Patent Nos. 5,556,762, 5,750,373, 4,708,871, 4,833,092, 5,223,409, 5,403,484, 5,571,689, 5,663,143; PCT Publication Nos. WO 84/03506 and WO84/03564; Geysen et al., Proc. Natl. Acad. Sci. U.S.A., 81:3998-4002 (1984); Geysen et al., Proc. Natl. Acad. Sci. U.S.A., 82:178-182 (1985); Geysen et al., in Synthetic Peptides as Antigens, 130-149 (1986); Geysen et al., J. Immunol. Meth 102:259-274 (1987); Schoofs et al., J. Immunol., 140:611-616 (1988), Cwirla, S. E. et al., (1990) Proc. Natl. Acad. Sci. USA, 87:6378; Lowman, H.B. et al., (1991) Biochemistry, 30:10832; Clackson, T. et al., (1991) Nature, 352: 624; Marks, J. D. et al., (1991), J. Mol. Biol., 222:581; Kang, A.S. et al., (1991) Proc. Natl. Acad. Sci. USA, 88:8363, and Smith, G. P. (1991) Current Opin. Biotechnol., 2:668).

In this regard, bacteriophage (phage) display is one well known technique which allows one to screen large polypeptide libraries to identify member(s) of those libraries which are capable of specifically binding to a target polypeptide, MiT polypeptide. Phage display is a technique by which variant polypeptides are displayed as fusion proteins to the coat protein on the surface of bacteriophage particles (Scott, J.K. and Smith, G. P. (1990) Science, 249: 386). The utility of phage display lies in the fact that large libraries of selectively randomized protein variants (or randomly cloned cDNAs) can be rapidly and efficiently sorted for those sequences that bind to a target molecule with high affinity. Display of peptide (Cwirla, S. E. et al., (1990) Proc. Natl. Acad. Sci. USA, 87:6378) or protein (Lowman, H.B. et al., (1991) Biochemistry, 30:10832; Clackson, T. et al., (1991) Nature, 352: 624; Marks, J. D. et al., (1991), J. Mol. Biol., 222:581; Kang, A.S. et al., (1991) Proc. Natl. Acad. Sci. USA, 88:8363) libraries on phage have been used for screening millions of polypeptides or oligopeptides for ones with specific binding properties (Smith, G. P. (1991) Current Opin. Biotechnol., 2:668). Sorting phage libraries of random mutants requires a strategy for constructing and propagating a large number of variants, a procedure for affinity purification using the target receptor, and a means of evaluating the results of binding enrichments. U.S. Patent Nos. 5,223,409, 5,403,484, 5,571,689, and 5,663,143.

Although most phage display methods have used filamentous phage, lambdoid phage display systems (WO 95/34683; U.S. 5,627,024), T4 phage display systems (Ren et al., Gene, 215: 439 (1998); Zhu et al., Cancer Research, 58(15): 3209-3214 (1998); Jiang et al., Infection & Immunity, 65(11): 4770-4777 (1997); Ren et al., Gene, 195(2):303-311 (1997); Ren, Protein Sci., 5: 1833 (1996); Efimov et al., Virus Genes, 10: 173 (1995)) and T7 phage display systems (Smith and Scott, Methods in Enzymology, 217: 228-257 (1993); U.S. 5,766,905) are also known.

Additional improvements enhance the ability of display systems to screen peptide libraries for binding to selected target molecules and to display functional proteins with the potential of screening these proteins for desired properties. Combinatorial reaction devices for phage display reactions have been developed (WO 98/14277) and phage display libraries have been used to analyze and control bimolecular interactions (WO 98/20169; WO 98/20159) and properties of constrained helical peptides (WO 98/20036). WO 97/35196 describes a method of isolating an affinity ligand in which a phage display library is contacted with one solution in which the ligand will bind to a target molecule and a second solution in which the affinity ligand will not bind to the target molecule, to selectively isolate binding ligands. WO 97/46251 describes a method of biopanning a random phage display library with an affinity purified antibody and then isolating binding phage, followed by a micropanning process using microplate wells to isolate high affinity binding phage. The use of *Staphylococcus aureus* protein A as an affinity tag has also been reported (Li et al., (1998) Mol Biotech., 9:187). WO 97/47314 describes the use of substrate subtraction libraries to distinguish enzyme specificities using a combinatorial library which may be a phage display library. A method for selecting enzymes suitable for use in detergents using phage display is described in WO 97/09446. Additional methods of selecting specific binding proteins are described in U.S. Patent Nos. 5,498,538, 5,432,018, and WO 98/15833.

Methods of generating peptide libraries and screening these libraries are also disclosed in U.S. Patent Nos. 5,723,286, 5,432,018, 5,580,717, 5,427,908, 5,498,530, 5,770,434, 5,734,018, 5,698,426, 5,763,192, and 5,723,323.

### D. Binding Small Molecules

Provided herein are MiT small molecule antagonists for use as a MiT antagonist in any of the methods described herein.

In some aspects of the disclosure of any of the small molecules, the MiT small molecule antagonist is a MITF small molecule antagonist. In some aspect of the disclosure, the MiT small molecule antagonist is a MITF-translocation small molecule antagonist. In some aspects of the disclosure of any of the small molecules, the MiT small molecule antagonist is a TFEB small molecule antagonist. In some aspect of the disclosure, the MiT small molecule antagonist is a TFEB -translocation small molecule antagonist. In some aspects of the disclosure of any of the small molecules, the MiT small molecule antagonist is a TFEC small molecule antagonist. In some aspect of the disclosure, the MiT small molecule antagonist is a TFEC -translocation small molecule antagonist. In some aspects of the disclosure of any of the small molecules, the MiT small molecule antagonist is a TFE3 small molecule antagonist. In some aspect of the disclosure, the MiT small molecule antagonist is a TFE3 -translocation small molecule antagonist.

In some aspects of the disclosure of any of the small molecules, the small molecule binds to a MITF-translocation fusion polypeptide. In some aspects of the disclosure, small molecule specifically binds a MITF-translocation fusion polypeptide, but do not substantially bind wild-type MITF and/or a second gene of the translocation. In some aspects of the disclosure, the MITF-translocation fusion polypeptide comprises *ACTG1* and *MITF.* In some aspects of the disclosure, the MITF translocation (*e.g.*, rearrangement and/or fusion) comprises *ACTG1* exon 3. In some aspects of the disclosure, the MITF translocation (*e.g.*, rearrangement and/or fusion) comprises *ACTG1* exon 3 and *MITF* exon 3. In some aspects of the disclosure, the MITF-translocation fusion polypeptide comprises SEQ ID NO:30 and/or 14. In some aspects of the disclosure, the MITF translocation (*e.g*., rearrangement and/or fusion) comprises *AP3S1* and *MITF.* In some aspects of the disclosure, the MITF translocation (*e.g.*, rearrangement and/or fusion) comprises *AP3S1* exon 3. In some aspects of the disclosure, the MITF translocation (*e.g.*, rearrangement and/or fusion) comprises *ACTG1* exon 3 and *MITF* exon 3. In some aspects of the disclosure, the MITF translocation (*e.g.*, rearrangement and/or fusion) is driven by the *AP3S1* promoter.

In some aspects of the disclosure of any of the small molecules, the small molecule binds to a TFEB-translocation fusion polypeptide. In some aspects of the disclosure, small molecule specifically binds a TFEB-translocation fusion polypeptide, but do not substantially bind wild-type TFEC and/or a second gene of the translocation. In some aspects of the disclosure, the TFEB - translocation fusion polypeptide comprises *CLTC* and *TFEB..* In some aspects of the disclosure, the TFEB translocation (*e.g.*, rearrangement and/or fusion) comprises *CLTC* exon 17. In some aspects of the disclosure, the TFEB translocation (*e.g.*, rearrangement and/or fusion) comprises *CLTC* exon 17and *TFEB* exon 6. In some aspects of the disclosure, the SBNO2 translocation (*e.g.*, rearrangement and/or fusion) comprises *MIDN* promoter and *SBNO2* exon 1. In some aspects of the disclosure, the MITF-translocation fusion polypeptide comprises SEQ ID NO:20 and/or 31.

In some aspects of the disclosure of any of the small molecules, the small molecule binds to a TFEC-translocation fusion polypeptide. In some aspects of the disclosure, small molecule specifically binds a TFEC-translocation fusion polypeptide, but do not substantially bind wild-type TFEC and/or a second gene of the translocation.

In some aspects of the disclosure of any of the small molecules, the small molecule binds to a TFE3-translocation fusion polypeptide. In some aspects of the disclosure, small molecule specifically binds a TFE3-translocation fusion polypeptide, but do not substantially bind wild-type TFE3 and/or a second gene of the translocation.

Small molecules are preferably organic molecules other than binding polypeptides or antibodies as defined herein that bind, preferably specifically, to MiT polypeptide as described herein. Organic small molecules may be identified and chemically synthesized using known methodology (*see*, *e.g.*, PCT Publication Nos. WO00/00823 and WO00/39585). Organic small molecules are usually less than about 2000 Daltons in size, alternatively less than about 1500, 750, 500, 250 or 200 Daltons in size, wherein such organic small molecules that are capable of binding, preferably specifically, to a polypeptide as described herein may be identified without undue experimentation using well known techniques. In this regard, it is noted that techniques for screening organic small molecule libraries for molecules that are capable of binding to a polypeptide target are well known in the art (*see*, *e.g.*, PCT Publication Nos. WO00/00823 and WO00/39585). Organic small molecules may be, for example, aldehydes, ketones, oximes, hydrazones, semicarbazones, carbazides, primary amines, secondary amines, tertiary amines, N-substituted hydrazines, hydrazides, alcohols, ethers, thiols, thioethers, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds, acid chlorides, or the like.

In some aspects of the disclosure of any of the small molecules, the MiT small molecule antagonist is a c-met small molecule antagonist. In one aspect of the disclosure, the c-met antagonist binds c-met extracellular domain. In some aspects of the disclosure, the c-met small molecule antagonist binds c-met kinase domain. In some aspects of the disclosure, the c-met small molecule antagonist competes for c-met binding with HGF. In some aspects of the disclosure, the c-met small molecule antagonist competes for HGF binding to c-met. In some aspects of the disclosure, the c-met small molecule antagonist binds HGF.

In certain aspects of the disclosure, the c-met small molecule antagonist is any one of: SGX-523, Crizotinib; JNJ-38877605 (CAS no. 943540-75-8), BMS-698769, PHA-665752 (Pfizer), SU5416, INC-280 (Incyte; SU11274 (Sugen; [(3Z)-N-(3-chlorophenyl)-3-({3,5-dimethyl-4-[(4-methylpiperazin-1-yl)carbonyl]-1H-pyrrol-2-yl}methylene)-N-methyl-2-oxoindoline-5-sulfonamide; CAS no. 658084-23-2]), Foretinib, MGCD-265 (MethylGene; MGCD-265 targets the c-MET, VEGFR1, VEGFR2, VEGFR3, Ron and Tie-2 receptors; CAS no. 875337-44-3), Tivantinib (ARQ 197), LY-2801653 (Lilly), LY2875358 (Lilly), MP-470, EMD 1214063 (Merck Sorono), EMD 1204831 (Merck Serono), NK4, Cabozantinib (carbozantinib is a dual inhibitor of met and VEGFR2), MP-470 (SuperGen; is an inhibitor of c-KIT, MET, PDGFR, Flt3, and AXL), Comp-1, E7050 (Cas no. 1196681-49-8; E7050 is a dual c-met and VEGFR2 inhibitor (Esai); MK-2461 (Merck; N-((2R)-1,4-Dioxan-2-ylmethyl)-N-methyl-N'-[3-(1-methyl-1H-pyrazol-4-yl)-5-oxo-5H-benzo[4,5]cyclohepta[1,2-b]pyridin-7-yl]sulfamide; CAS no. 917879-39-1); MK8066 (Merck), PF4217903 (Pfizer), AMG208 (Amgen), SGX-126, RP1040, LY2801653, AMG458, EMD637830, BAY-853474, DP-3590. In certain aspects of the disclosure, the c-met small molecule antagonist is any one or more of crizotinib, tivantinib, carbozantinib, MGCD-265, , h224G11, DN-30, MK-2461, E7050, MK-8033, PF-4217903, AMG208, JNJ-38877605, EMD1204831, INC-280, LY-2801653, SGX-126, RP1040, LY2801653, BAY-853474, and/or LA480. In certain aspects of the disclosure, c-met small molecule antagonist is any one or more of crizotinib, tivantinib, carbozantinib, MGCD-265, and/or foretinib. In certain aspects of the disclosure, the c-met small molecule antagonist is any one or more of crizotinib, foretinib or carbozantinib.

In some aspects of the disclosure of any of the small molecules, the MiT small molecule antagonist is a BIRC7 pathway small molecule antagonist. In some aspects of the disclosure, the BIRC7 pathway small molecule antagonist is any one or more of: MV1, BV6, GDC-0152, LBW242, SM-164, HGS1029, TL32711.

In some aspects of the disclosure, the BIRC7 pathway antagonist is a monovalent antagonist. In some aspects of the disclosure, the BIRC7 pathway antagonist is a bivalent antagonist.

### E. Antagonist Polynucleotides

Provided herein are MiT polynucleotide antagonists for use as a MiT antagonist in any of the methods described herein. The polynucleotide may be an antisense nucleic acid and/or a ribozyme. The antisense nucleic acids comprise a sequence complementary to at least a portion of an RNA transcript of a MiT gene. However, absolute complementarity, although preferred, is not required. In some aspects of the disclosure, MiT polynucleotide is a MITF polynucleotide. In some aspects of the disclosure, MiT polynucleotide is a TFEB polynucleotide. In some aspects of the disclosure, MiT polynucleotide is a TFE3 polynucleotide. In some aspects of the disclosure, MiT polynucleotide is a TFEC polynucleotide. In some aspects of the disclosure, MiT polynucleotide is a SBNO2 polynucleotide.

In some aspects of the disclosure of any of the polynucleotide antagonists, the polynucleotide binds to a MITF polynucleotide. In some aspect of the disclosure, the polynucleotide binds to a TFEB-translocation polynucleotide. In some aspects of the disclosure of any of the polynucleotides, the polynucleotide binds to a TFEB polynucleotide. In some aspect of the disclosure, the polynucleotide binds to a TFEB -translocation polynucleotide. In some aspect of the disclosures of any of the polynucleotide antagonists, the polynucleotide binds to a TFEC polynucleotide. In some aspect of the disclosure, the polynucleotide binds to a TFEC -translocation polynucleotide. In some aspects of the disclosure of any of the polynucleotide antagonists, the polynucleotide binds to a TFE3 polynucleotide. In some aspect of the disclosure, the polynucleotide binds to a TFE3 -translocation polynucleotide.

In some aspect of the disclosures of any of the polynucleotide antagonists, the polynucleotide binds to a MITF-translocation fusion polypeptide. In some aspects of the disclosure, the polynucleotide specifically binds a MITF-translocation fusion polynucleotide, but does not substantially bind wild-type MITF polynucleotide and/or a second gene of the translocation. In some aspects of the disclosure, the MITF-translocation fusion polynucleotide comprises *ACTG1* and *MITF.* In some aspects of the disclosure, the MITF translocation (*e.g.*, rearrangement and/or fusion) comprises *ACTG1* exon 3. In some aspects of the disclosure, the MITF translocation (*e.g.*, rearrangement and/or fusion) comprises *ACTG1* exon 3 and *MITF* exon 3. In some aspects of the disclosure, the MITF-translocation fusion polynucleotide comprises SEQ ID NO: 13. In some aspects of the disclosure, the MITF translocation (*e.g*., rearrangement and/or fusion) comprises *AP3S1* and *MITF.* In some aspects of the disclosure, the MITF translocation (*e.g.*, rearrangement and/or fusion) comprises *AP3S1* exon 3. In some aspects of the disclosure, the MITF translocation (*e.g.*, rearrangement and/or fusion) comprises *ACTG1* exon 3 and *MITF* exon 3. In some aspects of the disclosure, the MITF translocation (*e.g.*, rearrangement and/or fusion) is driven by the *AP3S1* promoter.

In some aspects of the disclosure of any of the polynucleotide antagonists, the polynucleotide binds to a TFEB-translocation fusion polynucleotide. In some aspects of the disclosure, the polynucleotide specifically binds a TFEB-translocation fusion polynucleotide, but does not substantially bind wild-type TFEC polynucleotide and/or a second gene of the translocation. In some aspects of the disclosure, the TFEB -translocation fusion polynucleotide comprises *CLTC* and *TFEB.* In some aspects of the disclosure, the TFEB translocation (*e.g.*, rearrangement and/or fusion) comprises *CLTC* exon 17. In some aspects of the disclosure, the TFEB translocation (*e.g.*, rearrangement and/or fusion) comprises *CLTC* exon 17and *TFEB* exon 6. In some aspects of the disclosure, the TFEB-translocation fusion polynucleotide comprises SEQ ID NO: 19.

In some aspects of the disclosure of any of the polynucleotide antagonists, the polynucleotide binds to a TFEC-translocation fusion polynucleotide. In some aspects of the disclosure, the polynucleotide specifically binds a TFEC-translocation fusion polynucleotide, but does not substantially bind wild-type TFEC and/or a second gene of the translocation.

In some aspects of the disclosure of any of the polynucleotide antagonists, the polynucleotide binds to a TFE3-translocation fusion polynucleotide. In some aspects of the disclosure, polynucleotide specifically binds a TFE3-translocation fusion polynucleotide, but do not substantially bind wild-type TFE3 polynucleotide and/or a second gene of the translocation.

A sequence "complementary to at least a portion of an RNA," referred to herein, means a sequence having sufficient complementarity to be able to hybridize with the RNA, forming a stable duplex; in the case of double stranded MiT antisense nucleic acids, a single strand of the duplex DNA may thus be tested, or triplex formation may be assayed. The ability to hybridize will depend on both the degree of complementarity and the length of the antisense nucleic acid. Generally, the larger the hybridizing nucleic acid, the more base mismatches with an MiT RNA it may contain and still form a stable duplex (or triplex as the case may be). One skilled in the art can ascertain a tolerable degree of mismatch by use of standard procedures to determine the melting point of the hybridized complex.

Polynucleotides that are complementary to the 5' end of the message, *e.g*., the 5' untranslated sequence up to and including the AUG initiation codon, should work most efficiently at inhibiting translation. However, sequences complementary to the 3' untranslated sequences of mRNAs have been shown to be effective at inhibiting translation of mRNAs as well. *See generally*, Wagner, R., 1994, Nature 372:333-335. Thus, oligonucleotides complementary to either the 5'- or 3'-non-translated, non-coding regions of the MiT gene, could be used in an antisense approach to inhibit translation of endogenous MiT mRNA. Polynucleotides complementary to the 5' untranslated region of the mRNA should include the complement of the AUG start codon.
Antisense polynucleotides complementary to mRNA coding regions are less efficient inhibitors of translation but could be used in accordance with the invention. Whether designed to hybridize to the 5'-, 3'- or coding region of MiT mRNA, antisense nucleic acids should be at least six nucleotides in length, and are preferably oligonucleotides ranging from 6 to about 50 nucleotides in length. In specific aspects the oligonucleotide is at least 10 nucleotides, at least 17 nucleotides, at least 25 nucleotides or at least 50 nucleotides.

In one aspect of the disclosure, the MiT antisense nucleic acid of the invention is produced intracellularly by transcription from an exogenous sequence. For example, a vector or a portion thereof, is transcribed, producing an antisense nucleic acid (RNA) of the MiT gene. Such a vector would contain a sequence encoding the MiT antisense nucleic acid. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others know in the art, used for replication and expression in vertebrate cells. Expression of the sequence encoding MiT, or fragments thereof, can be by any promoter known in the art to act in vertebrate, preferably human cells. Such promoters can be inducible or constitutive. Such promoters include, but are not limited to, the SV40 early promoter region (Bernoist and Chambon, Nature 29:304-310 (1981), the promoter contained in the 3' long terminal repeat of Rous sarcoma virus (Yamamoto et al., Cell 22:787-797 (1980), the herpes thymidine promoter (Wagner et al., Proc. Natl. Acad. Sci. U.S.A. 78:1441-1445 (1981), the regulatory sequences of the metallothionein gene (Brinster et al., Nature 296:39-42 (1982)), etc.

### F. Antibody and Binding Polypeptide Variants

In certain aspects of the disclosure, amino acid sequence variants of the antibodies and/or the binding polypeptides provided herein are contemplated. For example, it may be desirable to improve the binding affinity and/or other biological properties of the antibody and/or binding polypeptide. Amino acid sequence variants of an antibody and/or binding polypeptides may be prepared by introducing appropriate modifications into the nucleotide sequence encoding the antibody and/or binding polypeptide, or by peptide synthesis. Such modifications include, for example, deletions from, and/or insertions into and/or substitutions of residues within the amino acid sequences of the antibody and/or binding polypeptide. Any combination of deletion, insertion, and substitution can be made to arrive at the final construct, provided that the final construct possesses the desired characteristics, *e.g*., target-binding.

In certain aspects of the disclosure, antibody variants and/or binding polypeptide variants having one or more amino acid substitutions are provided. Sites of interest for substitutional mutagenesis include the HVRs and FRs. Conservative substitutions are shown in Table 1 under the heading of "conservative substitutions." More substantial changes are provided in Table 1 under the heading of "exemplary substitutions," and as further described below in reference to amino acid side chain classes. Amino acid substitutions may be introduced into an antibody and/or binding polypeptide of interest and the products screened for a desired activity, *e.g*., retained/improved antigen binding, decreased immunogenicity, or improved ADCC or CDC.

**TABLE 1**

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr(T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g*., a humanized or human antibody). Generally, the resulting variant(s) selected for further study will have modifications (*e.g*., improvements) in certain biological properties (*e.g*., increased affinity, reduced immunogenicity) relative to the parent antibody and/or will have substantially retained certain biological properties of the parent antibody. An exemplary substitutional variant is an affinity matured antibody, which may be conveniently generated, *e.g*., using phage display-based affinity maturation techniques such as those described herein. Briefly, one or more HVR residues are mutated and the variant antibodies displayed on phage and screened for a particular biological activity (*e.g*., binding affinity).

Alterations (*e.g.*, substitutions) may be made in HVRs, *e.g.*, to improve antibody affinity. Such alterations may be made in HVR "hotspots," *i.e.*, residues encoded by codons that undergo mutation at high frequency during the somatic maturation process (*see*, *e.g.*, Chowdhury, Methods Mol. Biol. 207:179-196 (2008)), and/or SDRs (a-CDRs), with the resulting variant VH or VL being tested for binding affinity. Affinity maturation by constructing and reselecting from secondary libraries has been described, *e.g.*, in Hoogenboom et al., in METHODS IN MOL. BIOL. 178:1-37 (O'Brien et al., ed., Human Press, Totowa, NJ, (2001).) In some aspects of the disclosure of affinity maturation, diversity is introduced into the variable genes chosen for maturation by any of a variety of methods (*e.g*., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (*e.g*., 4-6 residues at a time) are randomized. HVR residues involved in antigen binding may be specifically identified, *e.g*., using alanine scanning mutagenesis or modeling. CDR-H3 and CDR-L3 in particular are often targeted.

In certain aspects of the disclosure, substitutions, insertions, or deletions may occur within one or more HVRs so long as such alterations do not substantially reduce the ability of the antibody to bind antigen. For example, conservative alterations (*e.g*., conservative substitutions as provided herein) that do not substantially reduce binding affinity may be made in HVRs. Such alterations may be outside of HVR "hotspots" or SDRs. In certain aspects of the disclosure of the variant VH and VL sequences provided above, each HVR either is unaltered, or contains no more than one, two or three amino acid substitutions.

A useful method for identification of residues or regions of the antibody and/or the binding polypeptide that may be targeted for mutagenesis is called "alanine scanning mutagenesis" as described by Cunningham and Wells (1989) Science, 244:1081-1085. In this method, a residue or group of target residues (*e.g*., charged residues such as arg, asp, his, lys, and glu) are identified and replaced by a neutral or negatively charged amino acid (*e.g*., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (*e.g.*, for ADEPT) or a polypeptide which increases the serum half-life of the antibody.

### G. Antibody and Binding Polypeptide Derivatives

In certain aspects of the disclosure, an antibody and/or binding polypeptide provided herein may be further modified to contain additional nonproteinaceous moieties that are known in the art and readily available. The moieties suitable for derivatization of the antibody and/or binding polypeptide include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (*e.g*., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody and/or binding polypeptide may vary, and if more than one polymer is attached, they can be the same or different molecules. In general, the number and/or type of polymers used for derivatization can be determined based on considerations including, but not limited to, the particular properties or functions of the antibody and/or binding polypeptide to be improved, whether the antibody derivative and/or binding polypeptide derivative will be used in a therapy under defined conditions, etc.

In another aspect of the disclosure, conjugates of an antibody and/or binding polypeptide to nonproteinaceous moiety that may be selectively heated by exposure to radiation are provided. In one aspect of the disclosure, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody and/or binding polypeptide-nonproteinaceous moiety are killed.

### H. Recombinant Methods and Compositions

Antibodies and/or binding polypeptides may be produced using recombinant methods and compositions, *e.g*., as described in U.S. Patent No. 4,816,567. In one aspect of the disclosure, isolated nucleic acid encoding an anti-MiT antibody. Such nucleic acid may encode an amino acid sequence comprising the VL and/or an amino acid sequence comprising the VH of the antibody (*e.g*., the light and/or heavy chains of the antibody). In a further aspect of the disclosure, one or more vectors (*e.g*., expression vectors) comprising such nucleic acid encoding the antibody and/or binding polypeptide are provided. In a further aspect of the disclosure, a host cell comprising such nucleic acid is provided. In one such aspect of the disclosure, a host cell comprises (*e.g*., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one aspect of the disclosure, the host cell is eukaryotic, *e.g.*, a Chinese Hamster Ovary (CHO) cell or lymphoid cell (*e.g.*, Y0, NS0, Sp20 cell). In one aspect of the disclosure, a method of making an antibody such as an anti-MiT antibody and/or binding polypeptide is provided, wherein the method comprises culturing a host cell comprising a nucleic acid encoding the antibody and/or binding polypeptide, as provided above, under conditions suitable for expression of the antibody and/or binding polypeptide, and optionally recovering the antibody and/or polypeptide from the host cell (or host cell culture medium).

For recombinant production of an antibody such as an anti-MiT antibody and/or a binding polypeptide, nucleic acid encoding the antibody and/or the binding polypeptide, *e.g*., as described above, is isolated and inserted into one or more vectors for further cloning and/or expression in a host cell. Such nucleic acid may be readily isolated and sequenced using conventional procedures (*e.g*., by using oligonucleotide probes that are capable of binding specifically to genes encoding the heavy and light chains of the antibody).

Suitable host cells for cloning or expression of vectors include prokaryotic or eukaryotic cells described herein. For example, antibodies may be produced in bacteria, in particular when glycosylation and Fc effector function are not needed. For expression of antibody fragments and polypeptides in bacteria, *see*, *e.g.*, U.S. Patent Nos. 5,648,237, 5,789,199, and 5,840,523. (*See* also Charlton, METHODS IN MOL. BIOL., Vol. 248 (B.K.C. Lo, ed., Humana Press, Totowa, NJ, 2003), pp. 245-254, describing expression of antibody fragments in *E. coli.*) After expression, the antibody may be isolated from the bacterial cell paste in a soluble fraction and can be further purified.

In addition to prokaryotes, eukaryotic microbes such as filamentous fungi or yeast are suitable cloning or expression hosts for vectors, including fungi and yeast strains whose glycosylation pathways have been "humanized," resulting in the production of an antibody with a partially or fully human glycosylation pattern. *See* Gerngross, Nat. Biotech. 22:1409-1414 (2004), and Li et al., Nat. Biotech. 24:210-215 (2006).

Suitable host cells for the expression of glycosylated antibody and/or glycosylated binding polypeptides are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See*, *e.g.*, US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, *e.g.*, in Graham et al., J. Gen Virol. 36:59 (1977)); baby hamster kidney cells (BHK); mouse sertoli cells (TM4 cells as described, *e.g*., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, *e.g.*, in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production and/or binding polypeptide production, *see*, *e.g.*, Yazaki and Wu, METHODS IN MOL. BIOL., Vol. 248 (B.K.C. Lo, ed., Humana Press, Totowa, NJ), pp. 255-268 (2003).

While the description relates primarily to production of antibodies and/or binding polypeptides by culturing cells transformed or transfected with a vector containing antibody- and binding polypeptide-encoding nucleic acid. It is, of course, contemplated that alternative methods, which are well known in the art, may be employed to prepare antibodies and/or binding polypeptides. For instance, the appropriate amino acid sequence, or portions thereof, may be produced by direct peptide synthesis using solid-phase techniques [*see*, *e.g.*, Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the antibody and/or binding polypeptide may be chemically synthesized separately and combined using chemical or enzymatic methods to produce the desired antibody and/or binding polypeptide.

### IV. Methods of Screening and/or Identifying MiT Antagonists With Desired Function

Techniques for generating MiT antagonists such as antibodies, binding polypeptides, and/or small molecules have been described above. Additional MiT antagonists such as anti-MiT antibodies, binding polypeptides, small molecules, and/or polynucleotides provided herein may be identified, screened for, or characterized for their physical/chemical properties and/or biological activities by various assays known in the art.

Provided herein are methods of screening for and/or identifying a MiT antagonist which inhibits MiT signaling, induces cancer cell cycle arrest, inhibits cancer cell proliferation, and/or promotes cancer cell death said method comprising: (a) contacting (i) a cancer cell, cancer tissue, and/or cancer sample, wherein the cancer cell, cancer tissue, and/or cancer comprises one or more biomarkers, and (ii) a reference cancer cell, reference cancer tissue, and/or reference cancer sample with a MiT candidate antagonist, (b) determining presence or absence of MiT binding, the level of MiT signaling, distribution of cell cycle stage, level of cell proliferation, and/or level of cancer cell death, whereby decreased level of MiT signaling, a difference in distribution of cell cycle stage, decreased level of cell proliferation, and/or increased level of cancer cell death between the cancer cell, cancer tissue, and/or cancer sample, wherein the cancer cell, cancer tissue, and/or cancer comprises one or more biomarkers, and reference cancer cell, reference cancer tissue, and/or reference cancer sample identifies the MiT candidate antagonist as an MiT antagonist which binds MiT, inhibits MiT signaling, induces cancer cell cycle arrest, inhibits cancer cell proliferation, and/or promotes cancer cell cancer death. In some aspects of the disclosure, the MiT antagonist is a MET pathway antagonist. In some aspects of the disclosure, the MiT antagonist is a BIRC7 pathway antagonist.

Further provided herein are methods of screening for and/or identifying a MiT antagonist which binds MiT, inhibits MiT signaling, induces cancer cell cycle arrest, inhibits cancer cell proliferation, and/or promotes cancer cell death said method comprising: (a) contacting a cancer cell, cancer tissue, and/or cancer sample, wherein the cancer cell, cancer tissue, and/or cancer comprises one or more biomarkers with a MiT candidate antagonist, (b) determining presence or absence or MiT binding, the level of MiT signaling, distribution of cell cycle stage, level of cell proliferation, and/or level of cancer cell death to the cancer cell, cancer tissue, and/or cancer sample in the absence of the MiT candidate antagonist, whereby decreased level of MiT binding, MiT signaling, a difference in distribution of cell cycle stage, decreased level of cell proliferation, and/or increased level of cancer cell death between the cancer cell, cancer tissue, and/or cancer sample in the presence of the MiT candidate antagonist and the cancer cell, cancer tissue, and/or cancer sample in the absence of the MiT candidate antagonist identifies the MiT candidate antagonist as an MiT antagonist which does not bind MiT, inhibits MiT signaling, induces cancer cell cycle arrest, inhibits cancer cell proliferation, and/or promotes cancer cell cancer death. In some aspects of the disclosure, the MiT antagonist is a MET pathway antagonist. In some aspects of the disclosure, the MiT antagonist is a BIRC7 pathway antagonist.

In some aspects of the disclosure of any of the methods, the one or more biomarkers comprise one or more MiT (e.g., MITF, TFEB, TFE3, TFEC, and/or SBNO2). In some aspects of the disclosure, presence of the one or more biomarkers is indicated by the presence of elevated expression levels (*e.g*., compared to reference) of one or more MiT (e.g., MITF, TFEB, TFE3, TFEC, and/or SBNO2). In some aspects of the disclosure, expression is polypeptide expression. In some aspects of the disclosure, expression is nucleic acid expression. In some aspects of the disclosure, the one or more biomarkers comprises MITF. In some aspects of the disclosure, the one or more biomarkers comprises TFEB. In some aspects of the disclosure, the one or more biomarkers comprises TFE3. In some aspects of the disclosure, the one or more biomarkers comprises TFEC. In some aspects of the disclosure, the one or more biomarkers comprises SBNO2.

In some aspects of the disclosure of any of the methods, the one or more biomarkers comprise MET and/or BIRC7. In some aspects of the disclosure, presence of the one or more biomarkers is indicated by the presence of elevated expression levels (*e.g*., compared to reference) of MET and/or BIRC7. In some aspects of the disclosure, expression is polypeptide expression. In some aspects of the disclosure, expression is nucleic acid expression. In some aspects of the disclosure, the one or more biomarkers comprises MET. In some aspects of the disclosure, the one or more biomarkers comprises BIRC7.

In some aspects of the disclosure of any of the methods, the one or more biomarkers comprise one or more MiT mutation (e.g., mutation(s) in one or more of MITF, TFEB, TFE3, TFEC, and/or SBNO2).

In some aspects of the disclosure of any of the articles of manufacture, the one or more biomarkers comprise an amplification of one or more genes selected from MITF, TFEB, TFE3, TFEC, and/or SBNO2.

In some aspects of the disclosure of any of the methods, the one or more biomarkers comprise a translocation or inversion (*e.g.*, rearrangement and/or fusion) of one or more genes selected from MITF, TFEB, TFE3, TFEC, and/or SBNO2. In some aspects of the disclosure, the presence of one or more biomarkers comprises the presence of a translocation (*e.g*., rearrangement and/or fusion) of one or more genes selected from MITF, TFEB, TFE3, TFEC, and/or SBNO2.

In some aspects of the disclosure of any of the methods, the one or more biomarkers comprise a translocation or inversion (*e.g*., rearrangement and/or fusion) of one or more genes selected from MITF, TFEB, TFE3, TFEC, and/or SBNO2, and one or more of MET and/or BIRC7. In some aspects of the disclosure, the presence of one or more biomarkers comprises the presence of a translocation (*e.g*., rearrangement and/or fusion) of one or more genes selected from MITF, TFEB, TFE3, TFEC, and/or SBNO2, and overexpression of one or more of MET and/or BIRC7.

In some aspects of the disclosure of any of the methods, the translocation (*e.g*., rearrangement and/or fusion) is a MITF translocation (*e.g*., rearrangement and/or fusion). In some aspects of the disclosure, the MITF translocation (*e.g*., rearrangement and/or fusion) comprises *ACTG1* and *MITF.* In some aspects of the disclosure, the MITF translocation (*e.g.*, rearrangement and/or fusion) comprises *ACTG1* exon 3. In some aspects of the disclosure, the MITF translocation (*e.g.*, rearrangement and/or fusion) comprises *ACTG1* exon 3 and *MITF* exon 3. In some aspects of the disclosure, the MITF translocation (*e.g*., rearrangement and/or fusion) comprises SEQ ID NO:13 and/or 30. In some aspects of the disclosure, the MITF translocation (*e.g*., rearrangement and/or fusion) comprises SEQ ID NO: 30. In some aspects of the disclosure, the MITF translocation (*e.g*., rearrangement and/or fusion) is detectable by primers which include SEQ ID NO:11 and/or 12. In some aspects of the disclosure, the MITF translocation (*e.g*., rearrangement and/or fusion) is detectable by primers which include SEQ ID NO:9, 10, 11 and/or 12. In some aspects of the disclosure, the MITF translocation (*e.g.*, rearrangement and/or fusion) is driven by the *ACTG1* promoter. In some aspects of the disclosure, the MITF translocation (*e.g*., rearrangement and/or fusion) comprises *AP3S1* and *MITF.* In some aspects of the disclosure, the MITF translocation (*e.g.*, rearrangement and/or fusion) comprises *AP3S1* exon 3. In some aspects of the disclosure, the MITF translocation (*e.g.*, rearrangement and/or fusion) comprises *ACTG1* exon 3 and *MITF* exon 3. In some aspects of the disclosure, the MITF translocation (*e.g*., rearrangement and/or fusion) is driven by the *AP3S1* promoter. In some aspects of the disclosure, the ASP3S1-MITF translocation is present in a clear cell RCC.

In some aspects of the disclosure of any of the methods, the translocation (*e.g*., rearrangement and/or fusion) is a TFEB translocation (*e.g*., rearrangement and/or fusion). In some aspects of the disclosure, the TFEB translocation (*e.g*., rearrangement and/or fusion) comprises *CLTC* and TFEB. In some aspects of the disclosure, the TFEB translocation (*e.g.*, rearrangement and/or fusion) comprises *CLTC* exon 17. In some aspects of the disclosure, the TFEB translocation (*e.g.*, rearrangement and/or fusion) comprises *CLTC* exon 17and *TFEB* exon 6. In some aspects of the disclosure, the TFEB translocation (*e.g.*, rearrangement and/or fusion) comprises SEQ ID NO:19. In some aspects of the disclosure, the TFEB translocation (*e.g*., rearrangement and/or fusion) is detectable by primers which include SEQ ID NO:17 and/or 18. In some aspects of the disclosure, the TFEB translocation (*e.g*., rearrangement and/or fusion) is detectable by primers which include SEQ ID NO:15, 16, 17 and/or 18. In some aspects of the disclosure, the TFEB translocation (*e.g.*, rearrangement and/or fusion) is driven by the *CLTC* promoter.

In some aspects of the disclosure of any of the methods, the translocation (*e.g*., rearrangement and/or fusion) is a SBNO2 translocation (*e.g*., rearrangement and/or fusion). In some aspects of the disclosure, the SBNO2 translocation (*e.g*., rearrangement and/or fusion) comprises *MIDN* and *SBNO2.* In some aspects of the disclosure, the SBNO2 translocation (*e.g.*, rearrangement and/or fusion) comprises MIDN promoter. In some aspects of the disclosure, the SBNO2 translocation (*e.g.*, rearrangement and/or fusion) comprises MIDN promoter and *SBNO2* exon 1. In some aspects of the disclosure, the SBNO2 translocation (*e.g*., rearrangement and/or fusion) comprises SEQ ID NO:25. In some aspects of the disclosure, the SBNO2 translocation (*e.g*., rearrangement and/or fusion) is detectable by primers which include SEQ ID NO:23 and/or 24. In some aspects of the disclosure, the SBNO2 translocation (*e.g*., rearrangement and/or fusion) is detectable by primers which include SEQ ID NO:21, 22, 23, and/or 25. In some aspects of the disclosure, the SBNO2 translocation (*e.g.*, rearrangement and/or fusion) is driven by the *CLTC* promoter.

Methods of determining the level of MiT signaling are known in the art and are described in the Examples herein. In some aspects of the disclosure, the levels of MiT signaling are determined using a luciferase reporter assay as described in the Examples. In some aspects of the disclosure, the MiT antagonist inhibits MiT signaling by reducing the level of MiT signaling by about any of 10, 20, 30, 40, 50, 60, 70, 80, 90, or 100%.

The growth inhibitory effects of a MiT antagonist described herein may be assessed by methods known in the art, *e.g*., using cells which express MiT either endogenously or following transfection with the respective gene(s). For example, appropriate tumor cell lines, and MiT polypeptide-transfected cells may be treated with a MiT antagonist described herein at various concentrations for a few days (*e.g.*, 2-7) days and stained with crystal violet or MTT or analyzed by some other colorimetric assay. Another method of measuring proliferation would be by comparing ³H-thymidine uptake by the cells treated in the presence or absence an antibody, binding polypeptide, small molecule, and/or polynucleotides of the invention. After treatment, the cells are harvested and the amount of radioactivity incorporated into the DNA quantitated in a scintillation counter. Appropriate positive controls include treatment of a selected cell line with a growth inhibitory antibody known to inhibit growth of that cell line. Growth inhibition of tumor cells in vivo can be determined in various ways known in the art.

Methods of determining the distribution of cell cycle stage, level of cell proliferation, and/or level of cell death are known in the art. In some aspects of the disclosure, cancer cell cycle arrest is arrest in G1.

In some aspects of the disclosure, the MiT antagonist will inhibit cancer cell proliferation of the cancer cell, cancer tissue, or cancer sample in vitro or in vivo by about 25-100% compared to the untreated cancer cell, cancer tissue, or cancer sample, more preferably, by about 30-100%, and even more preferably by about 50-100% or about 70-100%. For example, growth inhibition can be measured at a MiT antagonist concentration of about 0.5 to about 30 µg/ml or about 0.5 nM to about 200 nM in cell culture, where the growth inhibition is determined 1-10 days after exposure of the tumor cells to the MiT candidate antagonist. The MiT antagonist is growth inhibitory in vivo if administration of the MiT candidate antagonist at about 1 µg/kg to about 100 mg/kg body weight results in reduction in tumor size or reduction of tumor cell proliferation within about 5 days to 3 months from the first administration of the MiT candidate antagonist, preferably within about 5 to 30 days.

To select for a MiT antagonists which induces cancer cell death, loss of membrane integrity as indicated by, *e.g*., propidium iodide (PI), trypan blue or 7AAD uptake may be assessed relative to a reference. A PI uptake assay can be performed in the absence of complement and immune effector cells. MiT-expressing tumor cells are incubated with medium alone or medium containing the appropriate a MiT antagonist. The cells are incubated for a 3-day time period. Following each treatment, cells are washed and aliquoted into 35 mm strainer-capped 12 x 75 tubes (1 ml per tube, 3 tubes per treatment group) for removal of cell clumps. Tubes then receive PI (10 µg/ml). Samples may be analyzed using a FACSCAN® flow cytometer and FACSCONVERT® CellQuest software (Becton Dickinson). Those MiT antagonists that induce statistically significant levels of cell death as determined by PI uptake may be selected as cell death-inducing antibodies, binding polypeptides, small molecules, and/or polynucleotides.

To screen for MiT antagonists which bind to an epitope on or interact with a polypeptide bound by an antibody of interest, a routine cross-blocking assay such as that described in Antibodies, A Laboratory Manual, Cold Spring Harbor Laboratory, Ed Harlow and David Lane (1988), can be performed. This assay can be used to determine if a candidate MiT antagonist binds the same site or epitope as a known antibody. Alternatively, or additionally, epitope mapping can be performed by methods known in the art. For example, the antibody and/or binding polypeptide sequence can be mutagenized such as by alanine scanning, to identify contact residues. The mutant antibody is initially tested for binding with polyclonal antibody and/or binding polypeptide to ensure proper folding. In a different method, peptides corresponding to different regions of a polypeptide can be used in competition assays with the candidate antibodies and/or polypeptides or with a candidate antibody and/or binding polypeptide and an antibody with a characterized or known epitope.

In some aspects of the disclosure of any of the methods of screening and/or identifying, the MiT candidate antagonist is an antibody, binding polypeptide, small molecule, or polynucleotide. In some aspects of the disclosure, the MiT candidate antagonist is an antibody. In some aspects of the disclosure, the MiT antagonist antagonist is a small molecule.

In one aspect, a MiT antagonist is tested for its antigen binding activity, *e.g*., by known methods such as ELISA, Western blot, etc.

### V. Pharmaceutical Formulations

Pharmaceutical formulations of a MiT antagonist as described herein are prepared by mixing such antibody having the desired degree of purity with one or more optional pharmaceutically acceptable carriers (REMINGTON'S PHARMA. SCI. 16th edition, Osol, A. Ed. (1980)), in the form of lyophilized formulations or aqueous solutions. In some aspects of the disclosure, the MiT antagonist is a small molecule, an antibody, binding polypeptide, and/or polynucleotide. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*., Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized formulations are described in US Patent No. 6,267,958. Aqueous antibody formulations include those described in US Patent No. 6,171,586 and WO 2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in REMINGTON'S PHARMA. SCI. 16th edition, Osol, A. Ed. (1980).

Sustained-release preparations may be prepared. Suitable examples of sustained-release preparations include semipermeable matrices of solid hydrophobic polymers containing the MiT antagonist, which matrices are in the form of shaped articles, *e.g*., films, or microcapsules.

The formulations to be used for *in vivo* administration are generally sterile. Sterility may be readily accomplished, *e.g*., by filtration through sterile filtration membranes.

### VI. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a MiT antagonist (*e.g*., R-spondin antagonist, *e.g*., R-spondin-translocation antagonist) described herein. The label or package insert indicates that the composition is used for treating the condition of choice. Moreover, the article of manufacture may comprise (a) a first container with a composition contained therein, wherein the composition comprises a MiT antagonist (*e.g.*, R-spondin antagonist, *e.g.*, R-spondin-translocation antagonist); and (b) a second container with a composition contained therein, wherein the composition comprises a further cytotoxic or otherwise therapeutic agent.

In some aspects of the disclosure, the article of manufacture comprises a container, a label on said container, and a composition contained within said container; wherein the composition includes one or more reagents (*e.g*., primary antibodies that bind to one or more biomarkers or probes and/or primers to one or more of the biomarkers described herein), the label on the container indicating that the composition can be used to evaluate the presence of one or more biomarkers in a sample, and instructions for using the reagents for evaluating the presence of one or more biomarkers in a sample. The article of manufacture can further comprise a set of instructions and materials for preparing the sample and utilizing the reagents. In some aspects of the disclosure, the article of manufacture may include reagents such as both a primary and secondary antibody, wherein the secondary antibody is conjugated to a label, *e.g.*, an enzymatic label. In some aspects of the disclosure, the article of manufacture one or more probes and/or primers to one or more of the biomarkers described herein.

In some aspects of the disclosure of any of the articles of manufacture, the one or more biomarkers comprise one or more MiT (*e.g*., MITF, TFEB, TFE3, TFEC, and/or SBNO2). In some aspects of the disclosure, presence of the one or more biomarkers is indicated by the presence of elevated expression levels (*e.g*., compared to reference) of one or more MiT (e.g., MITF, TFEB, TFE3, TFEC, and/or SBNO2). In some aspects of the disclosure, expression is polypeptide expression. In some aspects of the disclosure, expression is nucleic acid expression. In some aspects of the disclosure, the one or more biomarkers comprises MITF. In some aspects of the disclosure, the one or more biomarkers comprises TFEB. In some aspects of the disclosure, the one or more biomarkers comprises TFE3. In some aspects of the disclosure, the one or more biomarkers comprises TFEC. In some aspects of the disclosure, the one or more biomarkers comprises SBNO2.

In some aspects of the disclosure of any of the articles of manufacture, the one or more biomarkers comprise MET and/or BIRC7 In some aspects of the disclosure, presence of the one or more biomarkers is indicated by the presence of elevated expression levels (*e.g*., compared to reference) of MET and/or BIRC7. In some aspects of the disclosure, expression is polypeptide expression. In some aspects of the disclosure, expression is nucleic acid expression. In some aspects of the disclosure, the one or more biomarkers comprises MET. In some aspects of the disclosure, the one or more biomarkers comprises BIRC7.

In some aspects of the disclosure of any of the articles of manufacture, the one or more biomarkers comprise one or more of MITF, TFEB, TFE3, TFEC, SBNO2, MET and/or BIRC7). In some aspects of the disclosure, presence of the one or more biomarkers is indicated by the presence of elevated expression levels (*e.g*., compared to reference) of one or more of MITF, TFEB, TFE3, TFEC, SBNO2, MET and/or BIRC7.

In some aspects of the disclosure of any of the articles of manufacture, the one or more biomarkers comprise a translocation (*e.g*., rearrangement and/or fusion) of one or more genes selected from MITF, TFEB, TFE3, TFEC, and/or SBNO2. In some aspects of the disclosure, the presence of one or more biomarkers comprises the presence of a translocation (*e.g*., rearrangement and/or fusion) of one or more genes selected from MITF, TFEB, TFE3, TFEC, and/or SBNO2.

In some aspects of the disclosure of any of the articles of manufacture, the one or more biomarkers comprise a mutation of one or more genes selected from MITF, TFEB, TFE3, TFEC, and/or SBNO2.

In some aspects of the disclosure of any of the articles of manufacture, the one or more biomarkers comprise a amplification of one or more genes selected from MITF, TFEB, TFE3, TFEC, and/or SBNO2.

In some aspects of the disclosure of any of the articles of manufacture, the one or more biomarkers comprise a translocation (*e.g*., rearrangement and/or fusion) of one or more genes selected from MITF, TFEB, TFE3, TFEC, and/or SBNO2, and one or more of MET and/or BIRC7. In some aspects of the disclosure, the presence of one or more biomarkers comprises the presence of a translocation (*e.g*., rearrangement and/or fusion) of one or more genes selected from MITF, TFEB, TFE3, TFEC, and/or SBNO2, and overexpression of one or more of MET and/or BIRC7.

In some aspects of the disclosure of any of the articles of manufacture, the translocation (*e.g.*, rearrangement and/or fusion) is a MITF translocation (*e.g.*, rearrangement and/or fusion). In some aspects of the disclosure, the MITF translocation (*e.g*., rearrangement and/or fusion) comprises *ACTG1* and *MITF.* In some aspects of the disclosure, the MITF translocation (*e.g.*, rearrangement and/or fusion) comprises *ACTG1* exon 3. In some aspects of the disclosure, the MITF translocation (*e.g.*, rearrangement and/or fusion) comprises *ACTG1* exon 3 and *MITF* exon 3. In some aspects of the disclosure, the MITF translocation (*e.g*., rearrangement and/or fusion) comprises SEQ ID NO:13 and/or 30. In some aspects of the disclosure, the MITF translocation (*e.g*., rearrangement and/or fusion) is detectable by primers which include SEQ ID NO:11 and/or 12. In some aspects of the disclosure, the MITF translocation (*e.g*., rearrangement and/or fusion) is detectable by primers which include SEQ ID NO:9, 10, 11 and/or 12. In some aspects of the disclosure, the MITF translocation (*e.g.,* rearrangement and/or fusion) is driven by the *ACTG1* promoter. In some aspects of the disclosure, the MITF translocation (*e.g*., rearrangement and/or fusion) comprises *AP3S1* and *MITF.* In some aspects of the disclosure, the MITF translocation (*e.g.*, rearrangement and/or fusion) comprises *AP3S1* exon 3. In some aspects of the disclosure, the MITF translocation (*e.g.*, rearrangement and/or fusion) comprises *ACTG1* exon 3 and *MITF* exon 3. In some aspects of the disclosure, the MITF translocation (*e.g*., rearrangement and/or fusion) is driven by the *AP3S1* promoter.

In some aspects of the disclosure of any of the articles of manufacture, the translocation (*e.g.*, rearrangement and/or fusion) is a TFEB translocation (*e.g.*, rearrangement and/or fusion). In some aspects of the disclosure, the TFEB translocation (*e.g*., rearrangement and/or fusion) comprises *CLTC* and TFEB. In some aspects of the disclosure, the TFEB translocation (*e.g.*, rearrangement and/or fusion) comprises *CLTC* and TFEB. In some aspects of the disclosure, the TFEB translocation (*e.g.*, rearrangement and/or fusion) comprises *CLTC* exon 17. In some aspects of the disclosure, the TFEB translocation *(e.g.,* rearrangement and/or fusion) comprises *CLTC* exon 17and *TFEB* exon 6. In some aspects of the disclosure, the TFEB translocation (*e.g.*, rearrangement and/or fusion) comprises SEQ ID NO:19. In some aspects of the disclosure, the TFEB translocation (e.g., rearrangement and/or fusion) is detectable by primers which include SEQ ID NO:17 and/or 18. In some aspects of the disclosure, the TFEB translocation (e.g., rearrangement and/or fusion) is detectable by primers which include SEQ ID NO:15, 16, 17 and/or 18. In some aspects of the disclosure, the TFEB translocation (*e.g.,* rearrangement and/or fusion) is driven by the *CLTC* promoter.

In some aspects of the disclosure of any of the articles of manufacture, the translocation (*e.g.,* rearrangement and/or fusion) is a SBNO2 inversion (e.g*.,* rearrangement and/or fusion). In some aspects of the disclosure, the SBNO2 inversion (*e.g*., rearrangement and/or fusion) comprises *MIDN* and *SBNO2.* In some aspects of the disclosure, the SBNO2 inversion (*e.g.*, rearrangement and/or fusion) comprises *MIDN* promoter and *SBNO2* exon 1. In some aspects of the disclosure, the SBNO2 inversion (*e.g*., rearrangement and/or fusion) comprises SEQ ID NO:25. In some aspects of the disclosure, the SBNO2 inversion (*e.g*., rearrangement and/or fusion) is detectable by primers which include SEQ ID NO:23 and/or 24. In some aspects of the disclosure, the SBNO2 inversion (*e.g*., rearrangement and/or fusion) is detectable by primers which include SEQ ID NO:21, 22, 23, and/or 25. In some aspects of the disclosure, the SBNO2 inversion (*e.g*., rearrangement and/or fusion) is driven by the *CLTC* promoter.

In some aspects of the disclosure of any of the articles of manufacture, the articles of manufacture comprise primers. In some aspects of the disclosure, the primers are any of SEQ ID NO: 9, 10, 11, 12, 15, 16, 17, 18, 21, 22, 23, and/or 24. In some aspects of the disclosure, the primers are any one or more of SEQ ID NOs: 9, 10, 11, and/or 12. In some aspects of the disclosure, the primers are any one or more of SEQ ID NOs: 15, 16, 17, and/or 18. In some aspects of the disclosure, the primers are any one or more of SEQ ID NOs: 21, 22, 23, and/or 24.

In some aspects of the disclosure of any of the article of manufacture, the MiT antagonist an antibody, binding polypeptide, small molecule, or polynucleotide. In some aspects of the disclosure, the MiT antagonist is a small molecule. In some aspects of the disclosure, the MiT antagonist is an antibody. In some aspects of the disclosure, the antibody is a monoclonal antibody. In some aspects of the disclosure, the antibody is a human, humanized, or chimeric antibody. In some aspects of the disclosure, the antibody is an antibody fragment and the antibody fragment binds MiT polypeptide .

The article of manufacture in this aspect of the disclosure of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Other optional components in the article of manufacture include one or more buffers (*e.g.*, block buffer, wash buffer, substrate buffer, etc), other reagents such as substrate (*e.g.*, chromogen) which is chemically altered by an enzymatic label, epitope retrieval solution, control samples (positive and/or negative controls), control slide(s) etc.

It is understood that any of the above articles of manufacture may include an immunoconjugate described herein in place of or in addition to a MiT antagonist.

**Sequences**

| Name | SEQUENCE | SEQ ID NO: |
|---|---|---|
| Human MITF amino acid sequence | | 1 |
| Human MITF nucleic acid sequence | | 2 |
| | | |
| Human TFEB amino acid sequence | | 3 |
| | | |
| Human TFEB nucleic acid sequence | | 4 |
| | | |
| Human TFE3 amino acid sequence | | 5 |
| Human TFE3 nucleic acid sequence | | 6 |
| | | |
| Human TFEC amino acid sequence | | 7 |
| Human TFEC nucleic acid sequence | | 8 |
| | | |
| | | |
| ACTG1-MITF translocation 5' fusion sequence | | 9 |
| ACTG1-MITF translocation 3' fusion sequence | | 10 |
| ACTG1-MITF translocation 5' primer sequence | CAT TGA GCA TGG CAT CGT CAC | 11 |
| ACTG1-MITF translocation 3' primer sequence | GGT TTG GAC ATG GCA AGC TC | 12 |
| A ACTG1-MITF translocation nucleic acid sequence | | 13 |
| | | |
| A ACTG1-MITF translocation peptide sequence | | 14 |
| CLTC-TFEB translocation 5' fusion sequence | | 15 |
| CLTC-TFEB translocation 3' fusion sequence | | 16 |
| CLTC-TFEB translocation 5' primer sequence | GCT TCT GCC TTG GCT AGA GG | 17 |
| CLTC-TFEB translocation 3' primer sequence | GTC GCT GCT GTA CAC ATT CAG | 18 |
| A CLTC-TFEB translocation nucleic acid sequence | | 19 |
| A CLTC-TFEB translocation peptide sequence | | 20 |
| MIDN-SBNO2 inversion 5' fusion sequence | | 21 |
| MIDN-SBNO2 inversion 3' fusion sequence | | 22 |
| MIDN-SBNO2 inversion 5' primer sequence | GTA CTC GCA GCA CTT GGA GC | 23 |
| MIDN-SBNO2 inversion 3' primer sequence | ATC TGG GCT TCT CGC TCC GTG | 24 |
| MIDN-SBNO2 inversion nucleic acid sequence | | 25 |
| ACTG1-MITF nucleic acid | | 26 |
| | | |
| | | |
| ACTG1-MITF protein | | 27 |
| | | |
| CLTC-TFEB nucleic acid | | 28 |
| | | |
| CLTC-TFEB protein | | 29 |
| | | |
| A ACTG1-MITF translocation peptide sequence | KMTQVQTH | 30 |
| A CLTC-TFEB translocation peptide sequence | DLELINLPLSS | 31 |

### EXAMPLES

The following are examples of methods and compositions of the invention. It is understood that various other embodiments may be practiced, given the general description provided above.

### Materials and Methods for Examples

**Samples, DNA and RNA preps:** 167 human primary nccRCC samples and their adjacent normal tissue were analyzed in this study. The nccRCC samples included 67 pRCC, 49 chRCC, 35 RO, 8 unclassified type carcinomas, 6 tRCC and 2 samples with sarcomatoid dedifferentiation (**Table 2** and **3**).

**Table 2: Sample summary table**

| **Subtype** | **RNA-Seq** | | |
|---|---|---|---|
| | **Tumor** | **Normal** | **Paired (Tumor + matched Normal)** |
| **Papillary** | 64 | 50 | 48 |
| **Chromophobe** | 46 | 33 | 32 |
| **Oncocytoma** | 35 | 29 | 29 |
| **Unclassified** | 7 | 6 | 5 |
| **Translocation** | 5 | 3 | 3 |
| **Sarcomatoid** | 2 | 2 | 2 |
| **Total:** | **159** | **123** | **119** |

**Table 3- Sample Information**

| **Patient ID** | **Inventory Sample Name** | **Primary Tissue** | **Histological Diagnosis** | **Subtype** | **% tumor content** | **RNA-Seq discovery/validation cohort** |
|---|---|---|---|---|---|---|
| 23 | 159T | Kidney | papillary | papillary | 95 | discovery |
| 25 | 1216T | Kidney | papillary | papillary | 65 | discovery |
| 47 | 1626T | Kidney | papillary | papillary | 90 | discovery |
| 57 | 9305T | Kidney | papillary | papillary | 85 | discovery |
| 58 | XP140T | Kidney | papillary | papillary | 85 | discovery |
| 59 | 9606T | Kidney | papillary | papillary | 90 | discovery |
| 60 | 9638T | Kidney | papillary | papillary | 90 | discovery |
| 61 | XP178T | Kidney | papillary | papillary | 90 | discovery |
| 63 | 14518T | Kidney | papillary | papillary | 75 | discovery |
| 64 | 14987T | Kidney | papillary | papillary | 80 | discovery |
| 65 | 16880T | Kidney | papillary | papillary | 90 | discovery |
| 66 | 17301T | Kidney | papillary | papillary | 90 | discovery |
| 67 | 18355T | Kidney | papillary | papillary | 85 | discovery |
| 68 | 18682T | Kidney | papillary | papillary | 70 | discovery |
| 69 | 18734T | Kidney | papillary | papillary | 90 | discovery |
| 70 | 18981T | Kidney | papillary | papillary | 85 | discovery |
| 71 | 19236T | Kidney | papillary | papillary | 75 | discovery |
| 72 | 15486T | Kidney | papillary | papillary | 85 | discovery |
| 73 | 13367T | Kidney | papillary | papillary | 85 | discovery |
| 74 | 13763T | Kidney | papillary | papillary | 70 | discovery |
| 75 | 13906T | Kidney | papillary | papillary | 80 | discovery |
| 76 | 16784T | Kidney | papillary | papillary | 75 | discovery |
| 77 | 16864T | Kidney | papillary | papillary | 75 | discovery |
| 78 | 16847T | Kidney | papillary | papillary | 85 | discovery |
| 79 | 18246T | Kidney | papillary | papillary | 90 | discovery |
| 80 | 19676T | Kidney | papillary | papillary | 90 | discovery |
| 81 | 19677T | Kidney | papillary | papillary | 80 | discovery |
| 82 | 20035T | Kidney | papillary | papillary | 95 | discovery |
| 83 | 17213T | Kidney | papillary | papillary | 80 | discovery |
| 84 | 4445T | Kidney | papillary | papillary | 85 | discovery |
| 85 | 12620T | Kidney | papillary | papillary | 85 | discovery |
| 86 | 3891T | Kidney | papillary | papillary | 65 | discovery |
| 87 | 2774T | Kidney | papillary | papillary | 85 | discovery |
| 88 | 55T | Kidney | papillary | papillary | 80 | discovery |
| 89 | 1241T | Kidney | papillary | papillary | 80 | discovery |
| 90 | 1337T | Kidney | papillary | papillary | 80 | discovery |
| 91 | 8264T | Kidney | papillary | papillary | 70 | discovery |
| 92 | 005T | Kidney | papillary | papillary | 85 | discovery |
| 93 | 71T | Kidney | papillary | papillary | 90 | discovery |
| 94 | 110T | Kidney | papillary | papillary | 95 | discovery |
| 95 | 25T | Kidney | papillary | papillary | 90 | discovery |
| 96 | 310T | Kidney | papillary | papillary | 80 | discovery |
| 98 | 4471T | Kidney | papillary | papillary | 95 | discovery |
| 101 | 8102T | Kidney | papillary | papillary | 75 | discovery |
| 102 | 17958T | Kidney | papillary | papillary | 70 | discovery |
| 103 | 6854T | Kidney | papillary | papillary | 80 | discovery |
| 114 | XP458aT1 | Kidney | papillary | papillary | 75 | discovery |
| 116 | XP348aT2 | Kidney | papillary | papillary | 90 | discovery |
| 2151 | 21587T1 | Kidney | papillary | papillary | 85 | validation |
| 2154 | 22300-2 | Kidney | papillary | papillary | 90 | validation |
| 2155 | 22301T1 | Kidney | papillary | papillary | 80 | validation |
| 2156 | 22440T3 | Kidney | papillary | papillary | 85 | validation |
| 2158 | 22589T2 | Kidney | papillary | papillary | 90 | validation |
| 2162 | 22810T1 | Kidney | papillary | papillary | 70 | validation |
| 2163 | 22811T2 | Kidney | papillary | papillary | 60 | validation |
| 2167 | 21825T1 | Kidney | papillary | papillary | 90 | validation |
| 2174 | 23128T2 | Kidney | papillary | papillary | 85 | validation |
| 2183 | 21299T1 | Kidney | papillary | papillary | 70 | validation |
| 2184 | XP408T2 | Kidney | papillary | papillary | 95 | validation |
| 2185 | XP467T1 | Kidney | papillary | papillary | 95 | validation |
| 2190 | 23049T1 | Kidney | papillary | papillary | 80 | validation |
| 2196 | 23357T2 | Kidney | papillary | papillary | 80 | validation |
| 2197 | 23597T2 | Kidney | papillary | papillary | 90 | validation |
| 2199 | 23699T1 | Kidney | papillary | papillary | 95 | validation |
| 2202 | XP429T2 | Kidney | papillary | papillary | 95 | validation |
| 2204 | 024T2 | Kidney | papillary | papillary | 80 | validation |
| 2207 | 015bT1 | Kidney | papillary | papillary | 80 | validation |
| 1 | 9335T | Kidney | chromophobe, classic | chromophobe | 95 | discovery |
| 2 | 12335T | Kidney | chromophobe, classic | chromophobe | 95 | discovery |
| 3 | XP238T | Kidney | chromophobe, eosinophilic | chromophobe | 85 | discovery |
| 4 | 15324T | Kidney | chromophobe, classic | chromophobe | 95 | discovery |
| 5 | 15473T | Kidney | chromophobe, classic | chromophobe | 95 | discovery |
| 6 | 16429T | Kidney | chromophobe, classic | chromophobe | 95 | discovery |
| 7 | 16373T | Kidney | chromophobe, eosinophilic | chromophobe | 75 | discovery |
| 8 | 17542T | Kidney | chromophobe, classic | chromophobe | 85 | discovery |
| 9 | 17827T | Kidney | chromophobe, eosinophilic | chromophobe | 95 | discovery |
| 10 | 18647T | Kidney | chromophobe, classic | chromophobe | 95 | discovery |
| 11 | 19752T | Kidney | chromophobe, classic | chromophobe | 95 | discovery |
| 12 | 9716T | Kidney | chromophobe, classic | chromophobe | 90 | discovery |
| 13 | 11343T | Kidney | chromophobe, classic | chromophobe | 95 | discovery |
| 14 | 1212T | Kidney | chromophobe, classic | chromophobe | 90 | discovery |
| 15 | 1655T | Kidney | chromophobe, classic | chromophobe | 85 | discovery |
| 16 | 1673T | Kidney | chromophobe, classic | chromophobe | 90 | discovery |
| 17 | 7640T | Kidney | chromophobe, classic | chromophobe | 80 | discovery |
| 19 | 81T | Kidney | chromophobe, classic | chromophobe | 85 | discovery |
| 21 | 17460T | Kidney | chromophobe, eosinophilic | chromophobe | 80 | discovery |
| 22 | 114T | Kidney | chromophobe, classic | chromophobe | 85 | discovery |
| 24 | 152T | Kidney | chromophobe, classic | chromophobe | 90 | discovery |
| 26 | 17479T | Kidney | chromophobe, classic | chromophobe | 95 | discovery |
| 27 | 18673T | Kidney | chromophobe, eosinophilic | chromophobe | 85 | discovery |
| 28 | 19181T | Kidney | chromophobe, eosinophilic | chromophobe | 95 | discovery |
| 29 | 18918T | Kidney | chromophobe, eosinophilic | chromophobe | 90 | discovery |
| 51 | 219T | Kidney | Renal oncocytic neoplasm, favor chromophobe, eosinophilic | chromophobe | 90 | discovery |
| 99 | 297T | Kidney | chromophobe, classic | chromophobe | 95 | discovery |
| 2150 | 21523T2 | Kidney | chromophobe, classic | chromophobe | 80 | validation |
| 2153 | 22214T1 | Kidney | chromophobe, classic | chromophobe | 95 | validation |
| 2159 | 22684T3 | Kidney | chromophobe, classic | chromophobe | 95 | validation |
| 2160 | 22592T2 | Kidney | chromophobe, classic | chromophobe | 95 | validation |
| 2165 | 21656T1 | Kidney | chromophobe, classic | chromophobe | 95 | validation |
| 2166 | 21639T2 | Kidney | chromophobe, eosinophilic | chromophobe | 90 | validation |
| 2170 | 20287T1 | Kidney | chromophobe, classic | chromophobe | 90 | validation |
| 2171 | 20433T2 | Kidney | chromophobe, classic | chromophobe | 95 | validation |
| 2175 | 23129T1 | Kidney | chromophobe, classic | chromophobe | 90 | validation |
| 2176 | 20286T2 | Kidney | chromophobe, classic | chromophobe | 95 | validation |
| 2177 | 20452T1 | Kidney | chromophobe, eosinophilic | chromophobe | 95 | validation |
| 2178 | 20706T1 | Kidney | chromophobe, eosinophilic | chromophobe | 80 | validation |
| 2181 | 21202T1 | Kidney | chromophobe, classic | chromophobe | 95 | validation |
| 2186 | 22913T1 | Kidney | chromophobe, classic | chromophobe | 95 | validation |
| 2187 | 22880-2 | Kidney | chromophobe, classic | chromophobe | 90 | validation |
| 2188 | 22868-2 | Kidney | chromophobe, classic | chromophobe | 75 | validation |
| 2191 | 23291T1 | Kidney | chromophobe, eosinophilic | chromophobe | 75 | validation |
| 2193 | 23314T2 | Kidney | chromophobe, classic | chromophobe | 90 | validation |
| 2198 | 23494T2 | Kidney | chromophobe, classic | chromophobe | 90 | validation |
| 2203 | XP505T2 | Kidney | chromophobe, eosinophilic | chromophobe | 90 | validation |
| 2205 | 011cT2 | Kidney | chromophobe, classic | chromophobe | 95 | validation |
| 2208 | 016aT1 | Kidney | chromophobe, classic | chromophobe | 90 | validation |
| 20 | 9787T | Kidney | oncocytoma | oncocytoma | 80 | discovery |
| 31 | 18593T | Kidney | oncocytoma | oncocytoma | 90 | discovery |
| 32 | 18990T | Kidney | Renal oncocytic neoplasm, favor oncocytoma | oncocytoma | 95 | discovery |
| 33 | XP174T | Kidney | Renal oncocytic neoplasm, favor oncocytoma | oncocytoma | 70 | discovery |
| 34 | 9720T | Kidney | oncocytoma | oncocytoma | 90 | discovery |
| 35 | 11795T | Kidney | oncocytoma | oncocytoma | 75 | discovery |
| 36 | 13730T | Kidney | oncocytoma | oncocytoma | 90 | discovery |
| 38 | 17624T | Kidney | oncocytoma | oncocytoma | 95 | discovery |
| 39 | 14465T | Kidney | oncocytoma | oncocytoma | 90 | discovery |
| 40 | 19091T | Kidney | oncocytoma | oncocytoma | 90 | discovery |
| 41 | XP370T | Kidney | oncocytoma | oncocytoma | 90 | discovery |
| 42 | 006T | Kidney | oncocytoma | oncocytoma | 95 | discovery |
| 43 | 7139T | Kidney | oncocytoma | oncocytoma | 90 | discovery |
| 44 | 7243T | Kidney | Renal oncocytic neoplasm, favor oncocytoma | oncocytoma | 95 | discovery |
| 45 | 13067T | Kidney | Renal oncocytic neoplasm, favor oncocytoma | oncocytoma | 90 | discovery |
| 46 | 3138T | Kidney | oncocytoma | oncocytoma | 90 | discovery |
| 48 | 1907T | Kidney | oncocytoma | oncocytoma | 90 | discovery |
| 49 | 7630T | Kidney | oncocytoma | oncocytoma | 85 | discovery |
| 50 | 15579T | Kidney | oncocytoma | oncocytoma | 85 | discovery |
| 52 | 327T | Kidney | oncocytoma | oncocytoma | 95 | discovery |
| 53 | 189T | Kidney | oncocytoma | oncocytoma | 85 | discovery |
| 54 | 1274T | Kidney | oncocytoma | oncocytoma | 80 | discovery |
| 55 | 59T | Kidney | oncocytoma | oncocytoma | 85 | discovery |
| 56 | 4489T | Kidney | oncocytoma | oncocytoma | 85 | discovery |
| 100 | 882T | Kidney | oncocytoma | oncocytoma | 80 | discovery |
| 2152 | 21978T2 | Kidney | oncocytoma | oncocytoma | 90 | validation |
| 2157 | 22453T1 | Kidney | oncocytoma | oncocytoma | 85 | validation |
| 2161 | 22808T1 | Kidney | oncocytoma | oncocytoma | 80 | validation |
| 2168 | 21841T2 | Kidney | oncocytoma | oncocytoma | 90 | validation |
| 2182 | 21254T2 | Kidney | oncocytoma | oncocytoma | 85 | validation |
| 2189 | 23027T1 | Kidney | oncocytoma | oncocytoma | 95 | validation |
| 2192 | 23251-2 | Kidney | oncocytoma | oncocytoma | 95 | validation |
| 2195 | XP513T1 | Kidney | oncocytoma | oncocytoma | 95 | validation |
| 2201 | 23130T1 | Kidney | oncocytoma | oncocytoma | 75 | validation |
| 2206 | 013cT1 | Kidney | oncocytoma | oncocytoma | 95 | validation |
| 104 | 8207T | Kidney | sarcomatoid | sarcomatoid | 70 | discovery |
| 107 | 123T | Kidney | sarcomatoid | sarcomatoid | 80 | discovery |
| 108 | 14336T | Kidney | translocation | translocation | 85 | discovery |
| 109 | PtS1T | Kidney (Lung met) | translocation | translocation | 90 | discovery |
| 110 | TB1489T | Kidney | translocation | translocation | 80 | discovery |
| 111 | PtS10T | Kidney | translocation | translocation | 70 | discovery |
| 2164 | 22702T1 | Kidney | translocation | translocation | 80 | validation |
| 2200 | XP558T1 | Kidney | translocation | translocation | 95 | validation |
| 18 | 8432T | Kidney | unclassified | unclassified | 70 | discovery |
| 30 | 18277T | Kidney | unclassified | unclassified | 85 | discovery |
| 97 | 4274T | Kidney | unclassified | unclassified | 95 | discovery |
| 112 | XP213T | Kidney | unclassified | unclassified | 90 | discovery |
| 113 | 18051T | Kidney | unclassified | unclassified | 75 | discovery |
| 115 | XP462aT2 | Kidney | unclassified | unclassified | 70 | discovery |
| 2173 | 23042T1 | Kidney | unclassified | unclassified | 85 | validation |
| 2179 | 20825T1 | Kidney | unclassified | unclassified | 85 | validation |

Samples used in the study were obtained from patients undergoing surgery for a renal mass or metastasis for RCC at Saint Paul University Hospital, Parkland Memorial Hospital and Zale Lipshy University Hospital. These hospitals represent a tertiary care referral centers (Saint Paul and Zale Lipshy) as well as a county hospital (Parkland Memorial) and serve a wide variety of patients of multiple ethnicities including Caucasian, Hispanic, African-American, Asian and South Asian. Patients were excluded if they were known to have HIV, HBV, HCV or TB infections.

This study was conducted with appropriate IRB approval and written patient informed consent. Human tissue samples were de-identified prior to their use and are not considered human subject research under the US Department of Human and Health Services regulations and related guidance (45 CFR Part 46). Tumor and adjacent normal kidney samples were frozen fresh in liquid nitrogen and stored at -80 °C. Perpendicular sections immediately flanking 1-3 mm thick fragments of all frozen tumor and normal tissue were reviewed by a pathologist to confirm the diagnosis and tumor content¹. Basic demographic information for the patient samples in the study, where available, is included in **Table 3.** Tissue processing as well as simultaneous extraction of high-quality genomic DNA and total RNA from the same samples were performed as previously described¹.

**RNA-seq:** We obtained RNA-seq data for 159 tumor samples (119 with data for tumor/normal pairs). RNA-seq libraries were prepared using TruSeq RNA Sample Preparation kit (Illumina, CA). The libraries were multiplexed three per lane and sequenced on HiSeq 2000 to obtain on average ∼68 million paired-end (2 x 75bp) reads per sample.

**Sequence data processing:** All sequencing reads were evaluated for quality using the Bioconductor ShortRead package². To confirm that all samples were identified correctly, all exome and RNA-seq data variants that overlapped with the Illumina 2.5 M array data were compared and checked for consistency. An all-against-all sample comparison was done on germline variants to confirm the patient matched tumor-normal pairing prior to additional data analysis.

**Variant calling:** Sequencing reads were mapped to UCSC human genome (GRCh37/hg19) using BWA software³ set to default parameters. Local realignment, duplicate marking and raw variant calling were performed as described previously⁴. Somatic variant calling on tumor and its matched normal BAM file was performed using Strelka⁵. We used a minimum Strelka variant quality score of 1 to filter the variants. Known germline variants represented in dbSNP Build 131⁶ or 6515 previously published normal exomes⁷, but not represented in COSMIC v62⁸, were filtered out for all samples. In addition germline variants that were present in both the tumor and normal samples were removed. To evaluate the performance of this algorithm we randomly selected 178 protein-altering variants and validated them using Sequenom nucleic acid technology as described previously⁹. Of these, 92% (164/178) validated as somatic. All variants that were invalidated by Sequenom were removed from the final set. Variants labeled VALIDATED:RNA-Seq show confirmed expression of the variant in the RNA-seq data. In addition to dbSNP variant filtering described above, unpaired samples had their initial called variants filtered against normal variants from this data set as well as normals from a previously published colon data set¹⁰. The effect of all non-synonymous somatic mutations on gene function was predicted using PolyPhen¹¹, SIFT¹², and Condel¹³. All variants were annotated using Ensembl (release 63, www.ensembl.org).

**RNA-seq data analysis:** RNA-seq reads were aligned to the human genome version NCBI GRCh37 using GSNAP²⁰. Expression counts per gene were obtained by counting the number of reads aligned concordantly within a pair and uniquely to each gene locus as defined by NCBI and Ensembl gene annotations, and RefSeq mRNA sequences. Differential gene expression analysis performed using edgeR²¹ and DESeq2²² was used to compute the variance stabilized expression values for plotting the expression heatmaps. Variants in RNA-seq data were determined using the GATK⁴.

**Gene fusion detection and validation:** Putative fusions were identified using a computational pipeline we have developed called GSTRUCT-fusions¹⁸. Only fusion events that had at least 3 reads mapping to the fusion junction and were not found in any of the normal samples were included for further consideration. Furthermore we removed events that included unannotated exons or fusion partners that had closely related sequence as these are likely false positives. Validation of gene fusions was done using a RT-PCR with nccRCC tumor and matched normal samples as previously described. ¹⁸

**Cells and plasmids:** NIH3T3 and HEK293T cells obtained from Genentech cell bank were maintained in DMEM supplemented with 10%FBS. Clones expressing c-terminally Myc/DDK tagged *MITF*, *ACTG1* and *MET* from pCMV6 expression vector were purchased from Origene, MD. *ACTG1-MITF* fusion with a 3' c-terminally Myc/DDK tag sequence was generated using splicing by overlap PCR and cloned in pCMV6. CA).

**FISH analysis:** Three microns sections of FFPE (Formalin-fixed, paraffin-embedded) tissue were mounted on positively-charged glass slides. Selection of tissue and the target areas on the hematoxylin and eosin (H & E)-stained slide was performed by a board certified pathologist (PK). Using the H&E slide as a reference, target areas were etched with a diamond-tipped etcher on the back of the unstained slide to be assayed. Pretreatment, hybridization and post-washes are performed according to the microwave method in the DAKO Histology FISH Accessory kit guide (SSK5799CE_001/EFG/LMA/2012). DNA probe sets for TFE3 (Xp11.2) and TFEB (6p21.1) were obtained from Agilent Technologies, CA.

**MITF stability analysis:** HEK293T (1X10⁵ cells/well) were transfected with pCMV6-MITF (0.5µg) and pCMV6-ACTG1-MITF (0.3µg) using Fugene 6 according to manufacturer's instructions (Roche, CA). We used a lower amount of pCMV6-ACTG1-MITF in transfections as we found its expression to be higher compared to the wildtype MITF. At 24 h post transfection cells were treated with 50µg/ml cycloheximide (Sigma, MO) to block translation. Sample were processed and subject to western blot as described previously²⁸. MITF proteins were assessed by western blot using mouse-anti c-myc antibody (9E10, Genentech Inc., CA). hsp90 expression was assessed using rabbit anti-hsp90 antibody (Santa Cruz Biotechnology, CA) and was used as loading control. Expression was analyzed using appropriate secondary antibodies on a LI-COR Odyssey imager (LI-COR Biotechnology, NE).

**Western blot analysis:** Cell lysates from NIH3T3 (5 x 10⁶ cell) stably expressing either wildtype or mutant was prepared and used for western blotting as described previously²⁸. Phosphorylation status of MET was assessed using anti-phosho-Met (Y1234/35) antibody (Cell signaling, CA). Expression of MET, ACTG1, MITF, ACTG1-MITF, hsp90 in NIH3T3 stable lines was assessed by western blot using ant-Flag antibody (Sigma, MO) or anti-hsp90 antibody (Santa Cruz, CA) as indicated in the figure. Immunoblot was performed using appropriate secondary antibodies as described previously²¹.

**Anchorage independent growth:** The assay was performed as previously described²⁸. Briefly, NIH3T3 20,000 cells stably expressing Flag-tagged MET-WT, MET mutants, ACTG1, MITF or ACTG1-MITF were mixed with 0.35% agar in DMEM (high glucose) and plated in triplicate on 0.5% base agar in a 6-well plate. Plated cells were then overlaid with complete growth media (1ml) and incubated at 37 °C. The number of colonies formed in each plate was assessed using GelCount (Oxford Optronix Ltd, UK) after 3 weeks. Student's t-test (two tailed) was used for statistical analyses to compare treatment groups using GraphPad Prism 5.00 (GraphPad Software, San Diego, CA). A p-value <0.05 was considered statistically significant (*p<0.05 and **p<0.01).

**Cell growth assay:** NIH3T3 stable cells expressing wild type or D153Y-mutant MET were plated in complete medium. After 24 hrs complete medium was replaced with serum free medium and treated with indicated concentration of recombinant HGF (R&D system, MN). Cell growth were measured after 3 days with Cell Titer-Glo luminescence cell viability kit (Promega Corp., WI) as described previously²⁸. Student's t-test (two tailed) was used for statistical analyses to compare treatment groups using GraphPad Prism 5.00 (GraphPad Software, San Diego, CA). A p-value <0.05 was considered statistically significant (*p<0.05 and **p<0.01).

**Quantitative PCR analysis:** RNA (1ug) isolated at 24 h post transfection from cells transfected with MITF or MITF fusion construct using RNeasy mini kit (Qiagen), was reverse-transcribed to produce cDNA using SuperScript® VILO™ Master Mix (Life Technologies, CA). cDNA was then diluted and used for quantitative PCR using Taqman Gene Expression Master Mix on ViiA 7 Real Time PCR System (Life Technologies, CA). Primer and probe sets (20x) used for Taqman Gene Expression Assays were obtained from Life Technologies. The primer and probe sets used include *GAPDH* (Hs02758991_g1), *MITF* (Hs01117294_m1), *HIFIA* (Hs00153153_m1), *MET* (Hs01565584_m1), and *APEX1* (Hs00959050_g1). The relative gene expression values were normalized against *GAPDH* expression and then further normalized to *MITF* mRNA levels in the transfected HEK293T cells.

### Results

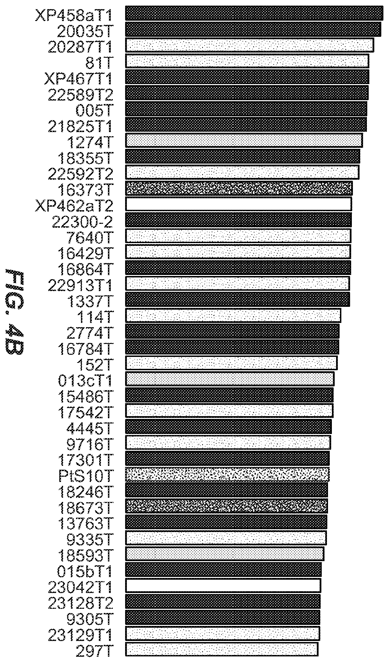

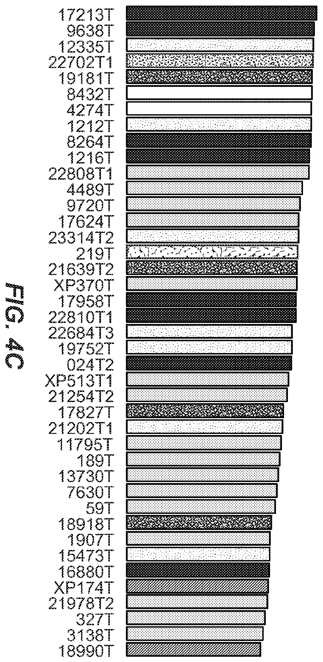

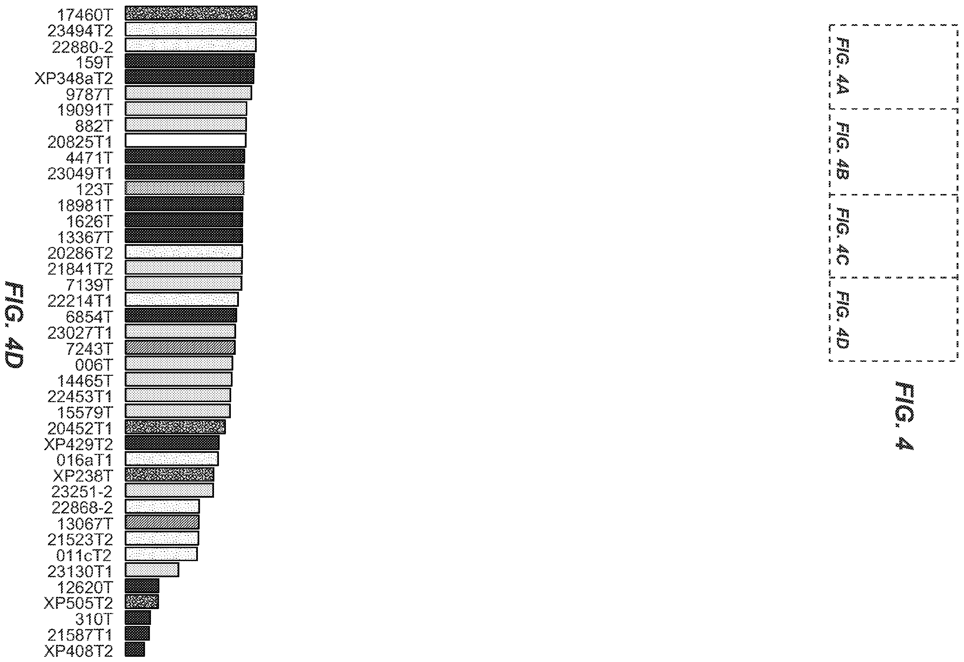

Gene fusions, besides mutations, copy number changes and expression alterations, are increasingly recognized to play a significant driver role in solid tumors⁶⁴. Some of the MiTF basic helix-loop-helix (bHLH) transcription factors, *TFE3,* TFEB, *TFEC,* and *MITF*⁶⁵ are deregulated in cancers. Translocations involving *TFE3* and *TFEB* are known in tRCCs. Detection of TFE3 expression by IHC and/or its translocation with FISH has been used in classifying *TFE3* fusion positive tRCCs. *TFEB* translocations are low frequency events and are often missed in the clinic. We analyzed our RNA-seq data to evaluate its utility for discovery of novel fusions and detection of known fusions in tRCCs. Of the samples classified as tRCC by TFE3 IHC, we found evidence for the previously reported *ASPSCR1-TFE3* fusion^{18,66 67} and *PRCC-TFE3* fusion⁶⁷. This was further confirmed by FISH (**Fig. 1b****,c**). We did not detect fusion events involving *TFE3* (or other MiTF members) in the two tRCC samples (14336T and PtS1T) even though they showed elevated *TFE3* expression (**Fig. 2**). Further, we did not find evidence for *TFE3* amplification in these samples, suggesting the involvement of an alternate mechanism leading to its upregulation. However, in one of the tRCC sample (14336T) lacking *TFE3* fusion, we identified a fusion involving midnolin (*MIDN*), a nucleolar protein, and strawberry notch homolog 2 (*SBNO2*), DExD/H helicase family corepressor. *MIDN* and *SBNO2* are located on chromosome 19p13.3 and are encoded by opposite strands (**Fig. 3**). The observed fusion is likely due to a genomic inversion in 19p13.3, that when transcribed and spliced places the non-coding exon of *MIDN* at the 5' end of the second exon of *SBNO2.* This results in a transcript coding for full length *SBNO2* that is under the control of *MIDN* promoter (**Fig. 3**). Interestingly, the sample with *MIDN-SBNO2* had the second highest level of *SBNO2* expression (**Fig. 4**). Further, the second tRCC sample (PtS1T) lacking *TFE3* fusion, had the highest level of *SBNO2* expression, although the exact mechanism leading to upregulation of *SBNO2* remains to be determined (**Fig. 4**). Recently, SBNO2 has been shown to play a critical role in bone homeostasis through activation of MITF⁶⁸. However, whether SBNO2 can modulate the levels and transcriptional activity of other MiTF members including TFE3 needs further investigation.

In addition to *TFE3* fusions, we found an unreported gene fusion involving *CLTC* and T*FEB* (*CLTC-TFEB*) in an nccRCC sample (8432T; **Fig. 5**) that was designated as unclassified. We confirmed this fusion using FISH (**Fig. 1d**). The *CLTC-TFEB* codes for an in-frame fusion protein that contains the activation and HLH domains of TFEB, as observed in other known TFEB fusions⁶⁶ indicating that it is likely functional.

Similarly, we found *PRCC-TFE3* fusion in another unclassified sample (20825T1). Based on the presence of fusions both 8432T and 20825T1 were re-classified as tRCC following additional pathology review.

A pRCC sample (1216T), showed amplification of a 490Kb region in chromosome 6 that included *TFEB* (**Fig. 6a**). We confirmed the amplification event using FISH (**Fig. 1a**). Consistent with the amplification, this sample had the highest level of *TFEB* expression compared to all other samples (**Fig. 6e****)** indicating that, besides previously known *TFEB* translocations in tRCC, amplification might be an additional cancer-relevant *TFEB* alteration in pRCC subtype. Given the TFEB amplification we re-evaluated the histology of this sample and found that it also had features consistent with tRCC.

Although ccRCC predisposing germline mutations in *MITF* have been discovered, no gene fusions involving *MITF* in nccRCC, to our knowledge, have been reported⁶⁶. We have in this study identified *ACTG1-MITF* gene fusion involving *ACTG1* and *MITF* (**Fig. 7**). We validated and confirmed the *ACTG1-MITF* to be somatic (**Fig. 7**). The sample (159T) expressing the *ACTG1- MITF* fusion was histologically classified as pRCC. The fusion protein encoded by *ACTG1-MITF* is about the same size as the wildtype MITF, except that the first 118 amino acids of MITF were replaced by the N-terminal 121 amino acids of ACTG1 (**Fig. 7b**). We found that the tumor expressing the fusion had a higher level of *MITF* compared to the matched normal (**Fig. 7c**). To further understand the ACTG1-MITF fusion protein, we transfected a cDNA expressing the fusion protein in HEK293T cells and tested the expression of known MITF target genes^{69,70}. We found that the ACTG1-MITF fusion showed a significant increase in the induction of *HIF1A, MET* and *APEX1* gene compared to the wildtype MITF (**Fig. 8** **and** **Fig. 9**). Given that ccRCC predisposing mutation in *MITF* is thought to function by increasing its stability⁷¹, we assessed the ACTG1-MITF fusion for its stability by following its turnover in cells. We found the ACTG1-MITF protein to be more stable compared to the wildtype MITF (**Fig. 8b**). Further, we tested the transforming ability of *ACTG1-MITF* by stably expressing the fusion and wildtype MITF proteins in NIH3T3 cells and assessed them for anchorage independent growth (**Fig. 8c-e**). We found that the ACTG1-MITF expressing cells, compared to wildtype MITF had a significant number of anchorage independent colonies (**Fig. 8d****-e).** Taken together, these data suggest that the MITF fusion, like the TFE3/TFEB fusions, can contribute to tumorigenesis in nccRCC.

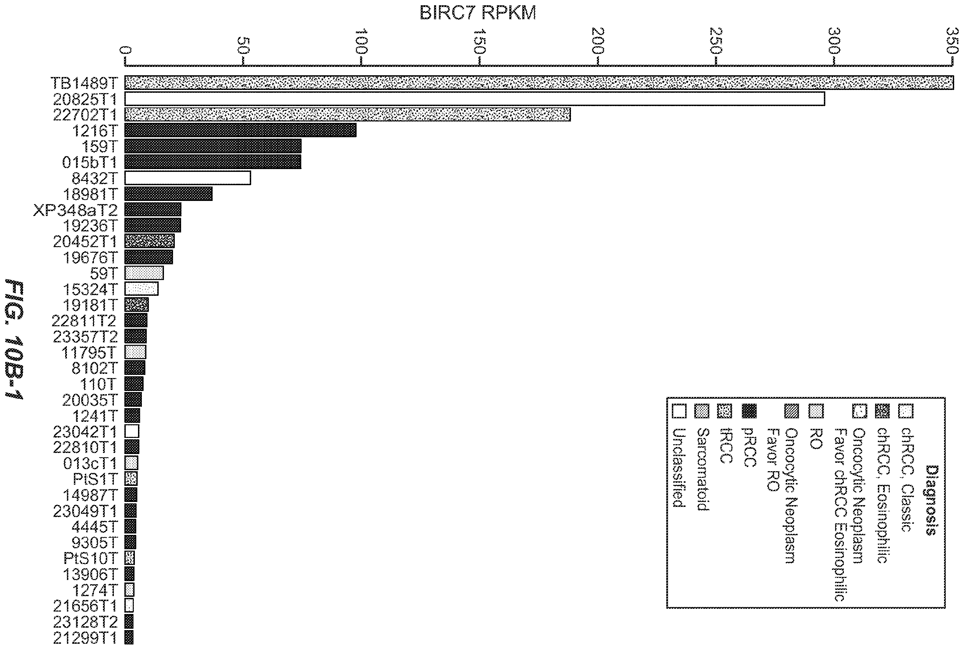

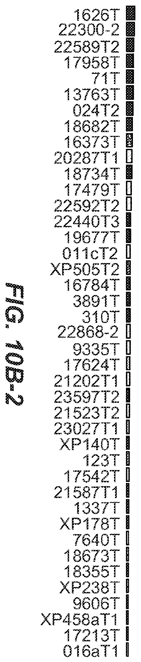

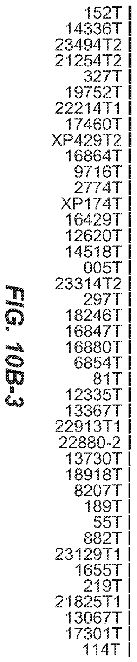

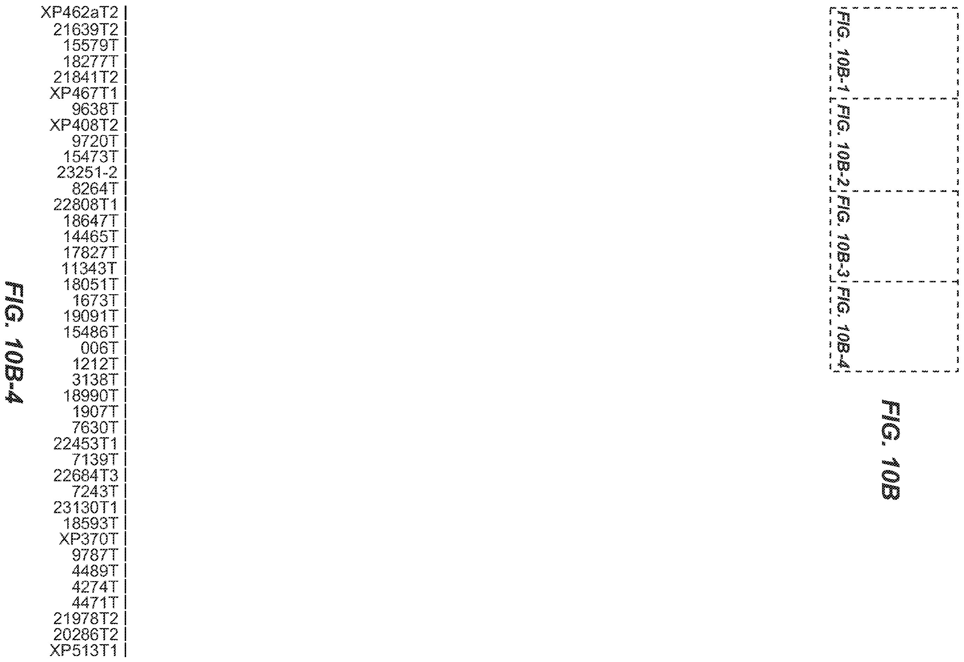

The MiTF proteins homo- or heterodimerize with other family members in various combinations and bind similar DNA elements to modulate gene expression⁷². Given this, we assessed the samples with MiTF fusions/amplification for genes upregulated in common between them that could serve as drug targets. We found a majority (4/5) of the MiTF fusion/amplification samples showed elevated *BIRC7* expression (**Fig. 10**). BIRC7, an anti-apoptotic protein, is a MITF target gene that is known to be upregulated in several cancers^{69,70}. Our data suggests that *BIRC7* expression may aid in the diagnosis of translocation carcinomas and those that overexpress MiTF members. Small molecule BIRC7 inhibitors that sensitize cancer cells to apoptosis are in clinical development⁶⁹ and may prove effective in treating the MiTF fusion positive or overexpressing tumors that currently remain intractable.

Translocations involving MiTF members *TFEB* and *TFE3* are known in tRCCs. However, the current histological diagnosis does not rely on a comprehensive assessment of the MiTF fusions. We show RNA-seq to be effective and comprehensive in detecting known *TFEB* and *TFE3* fusions. It is also useful in discovering unreported fusions like *ACTG1-MITF* and *CLTC-TFEB.* Also, RNA-seq showed the absence of fusions in samples that were classified as tRCC solely based on TFE3 expression. However, the fusion negative samples overexpressed *TFE3,* though the precise mechanism of this upregulation warrants further investigation. Our analysis illustrates the complexity involved in classifying tRCC samples based on histology alone and calls for the use of integrative histogenomics approach for diagnosis.

Our comprehensive analysis of translocations using RNA-seq identified *ACTG1-MITF* fusion involving *MITF* and previously unreported *TFEB* fusion involving a new fusion partner *CLTC,* expanding the fusions observed in nccRCC. We show that the MITF fusion is capable of inducing the expression of downstream target genes and is more stable compared to wildtype MITF. In a subset of nccRCCs the upregulation of MiTF members appear to be a common underlying mechanism that is achieved either through gene fusion, amplification or other, yet to be discovered mechanism. This suggests that the tumors with MiTF fusions, encompassing the tRCCs, and/or MiTF (*MITF*/*TFE3*/*TFEB*) overexpression likely form a distinct nccRCC MiTF-high subtype.

Our efforts to identify drug targets in the MiTF-high subtype showed that a majority of these tumors express the anti-apoptotic protein, BIRC7. This suggests that the MitF-high tumors may be candidates for therapy involving a BIRC7 inhibitor that can sensitize these tumors to induction of apoptosis.

### Partial reference list

1. Argani, P., Olgac, S., Tickoo, S.K., Goldfischer, M., Moch, H., Chan, D.Y., Eble, J.N., Bonsib, S.M., Jimeno, M., Lloreta, J., et al. (2007). Xp11 translocation renal cell carcinoma in adults: expanded clinical, pathologic, and genetic spectrum. The American journal of surgical pathology 31, 1149-1160.
2. Beleut, M., Zimmermann, P., Baudis, M., Bruni, N., Buhlmann, P., Laule, O., Luu, V.D., Gruissem, W., Schraml, P., and Moch, H. (2012). Integrative genome-wide expression profiling identifies three distinct molecular subgroups of renal cell carcinoma with different patient outcome. BMC Cancer 12, 310.
3. Bellmunt, J., and Dutcher, J. (2013). Targeted therapies and the treatment of non-clear cell renal cell carcinoma. Annals of oncology : official journal of the European Society for Medical Oncology / ESMO 24, 1730-1740.
4. Burwinkel, B., Scott, J.W., Buhrer, C., van Landeghem, F.K., Cox, G.F., Wilson, C.J., Grahame Hardie, D., and Kilimann, M.W. (2005). Fatal congenital heart glycogenosis caused by a recurrent activating R531Q mutation in the gamma 2-subunit of AMP-activated protein kinase (PRKAG2), not by phosphorylase kinase deficiency. Am J Hum Genet 76, 1034-1049.
5. Calderaro, J., Labrune, P., Morcrette, G., Rebouissou, S., Franco, D., Prevot, S., Quaglia, A., Bedossa, P., Libbrecht, L., Terracciano, L., et al. (2013). Molecular characterization of hepatocellular adenomas developed in patients with glycogen storage disease type I. Journal of hepatology 58, 350-357.
6. Carling, D., Mayer, F.V., Sanders, M.J., and Gamblin, S.J. (2011). AMP-activated protein kinase: nature's energy sensor. Nat Chem Biol 7, 512-518.
7. Cheng, H., Fukushima, T., Takahashi, N., Tanaka, H., and Kataoka, H. (2009). Hepatocyte growth factor activator inhibitor type 1 regulates epithelial to mesenchymal transition through membrane-bound serine proteinases. Cancer Res 69, 1828-1835.
8. Choueiri, T.K., Vaishampayan, U., Rosenberg, J.E., Logan, T.F., Harzstark, A.L., Bukowski, R.M., Rini, B.I., Srinivas, S., Stein, M.N., Adams, L.M., et al. (2013). Phase II and biomarker study of the dual MET/VEGFR2 inhibitor foretinib in patients with papillary renal cell carcinoma. Journal of clinical oncology : official journal of the American Society of Clinical Oncology 31, 181-186.
9. Contractor, H., Zariwala, M., Bugert, P., Zeisler, J., and Kovacs, G. (1997). Mutation of the p53 tumour suppressor gene occurs preferentially in the chromophobe type of renal cell tumour. The Journal of pathology 181, 136-139.
10. Davies, J.K., Wells, D.J., Liu, K., Whitrow, H.R., Daniel, T.D., Grignani, R., Lygate, C.A., Schneider, J.E., Noel, G., Watkins, H., et al. (2006). Characterization of the role of gamma2 R531G mutation in AMP-activated protein kinase in cardiac hypertrophy and Wolff-Parkinson-White syndrome. American journal of physiology Heart and circulatory physiology 290, H1942-1951.
11. de Moor, R.A., Schweizer, J.J., van Hoek, B., Wasser, M., Vink, R., and Maaswinkel-Mooy, P.D. (2000). Hepatocellular carcinoma in glycogen storage disease type IV. Arch Dis Child 82, 479-480.
12. Dynek, J.N., Chan, S.M., Liu, J., Zha, J., Fairbrother, W.J., and Vucic, D. (2008). Microphthalmia-associated transcription factor is a critical transcriptional regulator of melanoma inhibitor of apoptosis in melanomas. Cancer Res 68, 3124-3132.
13. Farley, M.N., Schmidt, L.S., Mester, J.L., Pena-Llopis, S., Pavia-Jimenez, A., Christie, A., Vocke, C.D., Ricketts, C.J., Peterson, J., Middelton, L., et al. (2013). A novel germline mutation in BAP1 predisposes to familial clear-cell renal cell carcinoma. Molecular cancer research : MCR 11, 1061-1071.
14. Forbes, S.A., Tang, G., Bindal, N., Bamford, S., Dawson, E., Cole, C., Kok, C.Y., Jia, M., Ewing, R., Menzies, A., et al. (2010). COSMIC (the Catalogue of Somatic Mutations in Cancer): a resource to investigate acquired mutations in human cancer. Nucleic Acids Res 38, D652-657.
15. Fu, L., Wang, G., Shevchuk, M.M., Nanus, D.M., and Gudas, L.J. (2011). Generation of a Mouse Model of Von Hippel-Lindau Kidney Disease Leading to Renal Cancers by Expression of a Constitutively Active Mutant of HIF1 Cancer Res 71, 6848-6856.
16. Gad, S., Lefevre, S.H., Khoo, S.K., Giraud, S., Vieillefond, A., Vasiliu, V., Ferlicot, S., Molinie, V., Denoux, Y., Thiounn, N., et al. (2007). Mutations in BHD and TP53 genes, but not in HNF1beta gene, in a large series of sporadic chromophobe renal cell carcinoma. Br J Cancer 96, 336-340.
17. Gandino, L., Longati, P., Medico, E., Prat, M., and Comoglio, P.M. (1994). Phosphorylation of serine 985 negatively regulates the hepatocyte growth factor receptor kinase. The Journal of biological chemistry 269, 1815-1820.
18. Gonzalez-Perez, A., and Lopez-Bigas, N. (2011). Improving the assessment of the outcome of nonsynonymous SNVs with a consensus deleteriousness score, Condel. Am J Hum Genet 88, 440-449.
19. Hagenkord, J.M., Gatalica, Z., Jonasch, E., and Monzon, F.A. (2011). Clinical genomics of renal epithelial tumors. Cancer genetics 204, 285-297.
20. Hakimi, A.A., Pham, C.G., and Hsieh, J.J. (2013). A clear picture of renal cell carcinoma. Nat Genet 45, 849-850.
21. Haq, R., and Fisher, D.E. (2011). Biology and clinical relevance of the micropthalmia family of transcription factors in human cancer. Journal of clinical oncology : official journal of the American Society of Clinical Oncology 29, 3474-3482.
22. Hoek, K.S., Schlegel, N.C., Eichhoff, O.M., Widmer, D.S., Praetorius, C., Einarsson, S.O., Valgeirsdottir, S., Bergsteinsdottir, K., Schepsky, A., Dummer, R., et al. (2008). Novel MITF targets identified using a two-step DNA microarray strategy. Pigment cell & melanoma research 21, 665-676.
23. Jeon, S.M., Chandel, N.S., and Hay, N. (2012). AMPK regulates NADPH homeostasis to promote tumour cell survival during energy stress. Nature 485, 661-665.
24. Kan, Z., Jaiswal, B.S., Stinson, J., Janakiraman, V., Bhatt, D., Stern, H.M., Yue, P., Haverty, P.M., Bourgon, R., Zheng, J., et al. (2010). Diverse somatic mutation patterns and pathway alterations in human cancers. Nature 466, 869-873.
25. Lager, D.J., Huston, B.J., Timmerman, T.G., and Bonsib, S.M. (1995). Papillary renal tumors. Morphologic, cytochemical, and genotypic features. Cancer 76, 669-673.
26. Lawrence, M.S., Stojanov, P., Polak, P., Kryukov, G.V., Cibulskis, K., Sivachenko, A., Carter, S.L., Stewart, C., Mermel, C.H., Roberts, S.A., et al. (2013). Mutational heterogeneity in cancer and the search for new cancer-associated genes. Nature 499, 214-218.
27. Linehan, W.M., and Ricketts, C.J. (2013). The metabolic basis of kidney cancer. Semin Cancer Biol 23, 46-55.
28. Manzia, T.M., Angelico, R., Toti, L., Cillis, A., Ciano, P., Orlando, G., Anselmo, A., Angelico, M., and Tisone, G. (2011). Glycogen Storage Disease Type Ia and VI Associated With Hepatocellular Carcinoma: Two Case Reports. Transplantation Proceedings 43, 1181-1183.
29. Maruyama, K., Uematsu, S., Kondo, T., Takeuchi, O., Martino, M.M., Kawasaki, T., and Akira, S. (2013). Strawberry notch homologue 2 regulates osteoclast fusion by enhancing the expression of DC-STAMP. The Journal of experimental medicine 210, 1947-1960.
30. Miller, M., Ginalski, K., Lesyng, B., Nakaigawa, N., Schmidt, L., and Zbar, B. (2001). Structural basis of oncogenic activation caused by point mutations in the kinase domain of the MET proto-oncogene: modeling studies. Proteins 44, 32-43.
31. Monzon, F.A., Hagenkord, J.M., Lyons-Weiler, M.A., Balani, J.P., Parwani, A.V., Sciulli, C.M., Li, J., Chandran, U.R., Bastacky, S.I., and Dhir, R. (2008). Whole genome SNP arrays as a potential diagnostic tool for the detection of characteristic chromosomal aberrations in renal epithelial tumors. Modern pathology : an official journal of the United States and Canadian Academy of Pathology, Inc 21, 599-608.
32. Nagashima, Y., Kuroda, N., and Yao, M. (2013). Transition of organizational category on renal cancer. Jap J Clin Oncol 43, 233-242.
33. Ng, P.C., and Henikoff, S. (2002). Accounting for human polymorphisms predicted to affect protein function. Genome Res 12, 436-446.
34. Osunkoya, A.O. (2010). Editorial comment to molecular pathology of chromophobe renal cell carcinoma: a review. International journal of urology : official journal of the Japanese Urological Association 17, 600-601.
35. Palmer, R.E., Lee, S.B., Wong, J.C., Reynolds, P.A., Zhang, H., Truong, V., Oliner, J.D., Gerald, W.L., and Haber, D.A. (2002). Induction of BAIAP3 by the EWS-WT1 chimeric fusion implicates regulated exocytosis in tumorigenesis. Cancer Cell 2, 497-505.
36. Palmieri, F. (2013). The mitochondrial transporter family SLC25: identification, properties and physiopathology. Molecular aspects of medicine 34, 465-484.
37. Pena-Llopis, S., and Brugarolas, J. (2013). Simultaneous isolation of high-quality DNA, RNA, miRNA and proteins from tissues for genomic applications. Nature protocols 8, 2240-2255.
38. Pena-Llopis, S., Vega-Rubin-de-Celis, S., Liao, A., Leng, N., Pavia-Jimenez, A., Wang, S., Yamasaki, T., Zhrebker, L., Sivanand, S., Spence, P., et al. (2012). BAP1 loss defines a new class of renal cell carcinoma. Nat Genet 44, 751-759.
39. Picken, M.M. (2010). Editorial comment from Dr Picken to Molecular pathology of renal oncocytoma: a review. International journal of urology : official journal of the Japanese Urological Association 17, 613-614.
40. Pleasance, E.D., Stephens, P.J., O'Meara, S., McBride, D.J., Meynert, A., Jones, D., Lin, M.-L., Beare, D., Lau, K.W., Greenman, C., et al. (2009). A small-cell lung cancer genome with complex signatures of tobacco exposure. Nature 463, 184-190.
41. Popova, T., Hebert, L., Jacquemin, V., Gad, S., Caux-Moncoutier, V., Dubois-d'Enghien, C., Richaudeau, B., Renaudin, X., Sellers, J., Nicolas, A., et al. (2013). Germline BAP1 mutations predispose to renal cell carcinomas. Am J Hum Genet 92, 974-980.
42. Ramensky, V., Bork, P., and Sunyaev, S. (2002). Human non-synonymous SNPs: server and survey. Nucleic Acids Res 30, 3894-3900.
43. Righi, L., Rapa, I., Votta, A., Papotti, M., and Sapino, A. (2012). Human achaete-scute homolog-1 expression in neuroendocrine breast carcinoma. Virchows Archiv : an international journal of pathology 460, 415-421.
44. Rohan, S., Tu, J.J., Kao, J., Mukherjee, P., Campagne, F., Zhou, X.K., Hyjek, E., Alonso, M.A., and Chen, Y.T. (2006). Gene expression profiling separates chromophobe renal cell carcinoma from oncocytoma and identifies vesicular transport and cell junction proteins as differentially expressed genes. Clinical cancer research: an official journal of the American Association for Cancer Research 12, 6937-6945.
45. Sato, Y., Yoshizato, T., Shiraishi, Y., Maekawa, S., Okuno, Y., Kamura, T., Shimamura, T., Sato-Otsubo, A., Nagae, G., Suzuki, H., et al. (2013). Integrated molecular analysis of clear-cell renal cell carcinoma. Nat Genet 45, 860-867.
46. Schmidt, L., Duh, F.M., Chen, F., Kishida, T., Glenn, G., Choyke, P., Scherer, S.W., Zhuang, Z., Lubensky, I., Dean, M., et al. (1997). Germline and somatic mutations in the tyrosine kinase domain of the MET proto-oncogene in papillary renal carcinomas. Nat Genet 16, 68-73.
47. Schmidt, L., Junker, K., Nakaigawa, N., Kinjerski, T., Weirich, G., Miller, M., Lubensky, I., Neumann, H.P., Brauch, H., Decker, J., et al. (1999). Novel mutations of the MET proto-oncogene in papillary renal carcinomas. Oncogene 18, 2343-2350.
48. Scott, J.W., Ross, F.A., Liu, J.K., and Hardie, D.G. (2007). Regulation of AMP-activated protein kinase by a pseudosubstrate sequence on the gamma subunit. The EMBO journal 26, 806-815.
49. Siegel, R., Naishadham, D., and Jemal, A. (2013). Cancer statistics, 2013. CA: a cancer journal for clinicians 63, 11-30.
50. Srigley, J.R., Delahunt, B., Eble, J.N., Egevad, L., Epstein, J.I., Grignon, D., Hes, O., Moch, H., Montironi, R., Tickoo, S.K., et al. (2013). The International Society of Urological Pathology (ISUP) Vancouver Classification of Renal Neoplasia. The American journal of surgical pathology 37, 1469-1489.
51. Steinberg, G.R., and Kemp, B.E. (2009). AMPK in Health and Disease. Physiol Rev 89, 1025-1078.
52. Steingrimsson, E., Copeland, N.G., and Jenkins, N.A. (2004). Melanocytes and the microphthalmia transcription factor network. Annu Rev Genet 38, 365-411.
53. TCGA (2013). Comprehensive molecular characterization of clear cell renal cell carcinoma. Nature 499, 43-49.
54. Toker, L., Bersudsky, Y., Plaschkes, I., Chalifa-Caspi, V., Berry, G.T., Buccafusca, R., Moechars, D., Belmaker, R.H., and Agam, G. (2014). Inositol-related gene knockouts mimic lithium's effect on mitochondrial function. Neuropsychopharmacology : official publication of the American College of Neuropsychopharmacology 39, 319-328.
55. Venkateswarlu, K., and Cullen, P.J. (1999). Molecular cloning and functional characterization of a human homologue of centaurin-alpha. Biochem Biophys Res Commun 262, 237-244.
56. Wang, W., Marimuthu, A., Tsai, J., Kumar, A., Krupka, H.I., Zhang, C., Powell, B., Suzuki, Y., Nguyen, H., Tabrizizad, M., et al. (2006). Structural characterization of autoinhibited c-Met kinase produced by coexpression in bacteria with phosphatase. Proceedings of the National Academy of Sciences of the United States of America 103, 3563-3568.
57. Weimer, J.M., Chattopadhyay, S., Custer, A.W., and Pearce, D.A. (2005). Elevation of Hook1 in a disease model of Batten disease does not affect a novel interaction between Ankyrin G and Hook1. Biochem Biophys Res Commun 330, 1176-1181.
58. Yokoyama, S., Woods, S.L., Boyle, G.M., Aoude, L.G., MacGregor, S., Zismann, V., Gartside, M., Cust, A.E., Haq, R., Harland, M., et al. (2011). A novel recurrent mutation in MITF predisposes to familial and sporadic melanoma. Nature 480, 99-103.
59. Young, A.N. (2010). Editorial comment from Dr Young to Molecular pathology of renal oncocytoma: A review. International journal of urology : official journal of the Japanese Urological Association 17, 612-613.
60. Yusenko, M.V. (2010a). Molecular pathology of chromophobe renal cell carcinoma: a review. International journal of urology : official journal of the Japanese Urological Association 17, 592-600.
61. Yusenko, M.V. (2010b). Molecular pathology of renal oncocytoma: a review. International journal of urology : official journal of the Japanese Urological Association 17, 602-612.
62. Yusenko, M.V., Kuiper, R.P., Boethe, T., Ljungberg, B., van Kessel, A.G., and Kovacs, G. (2009). High-resolution DNA copy number and gene expression analyses distinguish chromophobe renal cell carcinomas and renal oncocytomas. BMC Cancer 9, 152.
63. Zimmermann, P. (2006). The prevalence and significance of PDZ domain-phosphoinositide interactions. Biochimica et biophysica acta 1761, 947-956.

Although the foregoing invention has been described in some detail by way of illustration n and example for purposes of clarity of understanding, the descriptions and examples should not be construed as limiting the scope of the invention.

### ABSTRACT OF THE DISCLOSURE

Provided are therapies related to the treatment of pathological conditions, such as cancer.

## Claims

1. A method for determining expression of a translocation or inversion of a MITF gene, comprising the step of determining whether a sample from an individual expresses a translocation or inversion of a MITF gene.

2. The method of claim 1, wherein the MITF translocation comprises ACTG1 and MITF.

3. The method of claim 2, wherein the MITF translocation comprises ACTG1 exon 3, ACTG1 exon 3 and MITF exon 3, SEQ ID NO:13 and/or 30, or wherein the MITF translocation is detectable by primers which consist of or comprise SEQ ID NO:9, 10, 11 and/or 12.

4. The method of claim 1, wherein the MITF translocation is driven by the ACTG1 promoter.

5. The method of claim 1, wherein the MITF translocation comprises AP3S1 and MITF, AP3S1 exon 3, or ACTG1 exon 3 and MITF exon 3.

6. The method of any one of the claims 1-3 and 5, wherein the MITF translocation is driven by the AP3S1 promoter.

7. The methods of any one of the claims 1-6 wherein the MiT translocation results in elevated expression levels of MET, elevated activity and/or activation of MET, elevated expression levels of BIRC7, or elevated activity and/or activation of BIRC7.

8. The method of any one of the claims 1-7, wherein the translocation is a somatic translocation, an intra-chromosomal translocation, an interchromosomal translocation, an inversion, or a deletion.

9. The method of any one of claims 1-8, wherein the translocation is a translocation fusion polynucleotide and/or functional translocation fusion polypeptide.

10. The method of any one of the claims 1-9, wherein the sample is a cancer sample.

11. The method of claim 10, wherein the cancer is squamous cell cancer, lung cancer, small-cell lung cancer (SCLC), non-small cell lung cancer (NSCLC), adenocarcinoma of the lung and squamous carcinoma of the lung, cancer of the peritoneum, hepatocellular cancer, gastric or stomach cancer, gastrointestinal cancer, pancreatic cancer, glioblastoma, cervical cancer, ovarian cancer, liver cancer, bladder cancer, hepatoma, breast cancer, metastatic breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, tumors of the biliary tract, or head and neck cancer.

12. The method of claim 11, wherein the cancer is renal cell carcinoma (RCC).

13. The method of claim 12, wherein the RCC is non-clear cell renal cell carcinoma (nccRCC) or translocation RCC (tRCC).

14. The method of claim 13, wherein the RCC is nccRCC.

15. The method of any one of claims 1-14, wherein the MITF translocation or inversion is detected using a method comprising performing gene expression profiling, PCR, rtPCR RNA-seq, microarray analysis, SAGE, MassARRAY technique or FISH on a sample.

16. The method of any one of claims 1-14, wherein the MITF translocation or inversion is detected by detecting DNA, mRNA, cDNA, proteins, protein fragments and/or gene copy number.

## Patentansprüche

1. Verfahren zum Bestimmen der Expression einer Translokation oder Inversion eines MITF-Gens, umfassend den Schritt des Bestimmens, ob eine Probe von einem Individuum eine Translokation oder Inversion eines MITF-Gens exprimiert.

2. Verfahren nach Anspruch 1, wobei die MITF-Translokation ACTG1 und MITF umfasst.

3. Verfahren nach Anspruch 2, wobei die MITF-Translokation ACTG1-Exon 3, ACTG1-Exon 3 und MITF-Exon 3, SEQ ID NO:13 und/oder 30 umfasst oder wobei die MITF-Translokation mit Primem, die aus SEQ ID NO:9, 10, 11 und/oder12 bestehen oder diese umfassen, nachgewiesen werden kann.

4. Verfahren nach Anspruch 1, wobei die MITF-Translokation von dem ACTG1-Promotor kontrolliert wird.

5. Verfahren nach Anspruch 1, wobei die MITF-Translokation AP3S1 und MITF, AP3S1-Exon 3 oder ACTG1-Exon 3 und MITF-Exon 3 umfasst.

6. Verfahren nach einem der Ansprüche 1-3 und 5, wobei die MITF-Translokation von dem AP3S1-Promotor kontrolliert wird.

7. Verfahren nach einem der Ansprüche 1-6, wobei die MIT-Translokation zu erhöhten Expressionsniveaus von MET, erhöhter Aktivität und/oder Aktivierung von MET, erhöhten Expressionsniveaus von BIRC7 oder erhöhter Aktivität und/oder Aktivierung von BIRC7 führt.

8. Verfahren nach einem der Ansprüche 1-7, wobei die Translokation eine somatische Translokation, eine intrachromosomale Translokation, eine interchromosomale Translokation, eine Inversion oder eine Deletion ist.

9. Verfahren nach einem der Ansprüche 1-8, wobei die Translokation ein Translokations-Fusionspolynukleotid und/oder funktionelles Translokations-Fusionspolypeptid ist.

10. Verfahren nach einem der Ansprüche 1-9, wobei die Probe eine Krebsprobe ist.

11. Verfahren nach Anspruch 10, wobei der Krebs Plattenepithelkarzinom, Lungenkrebs, kleinzelliger Lungenkrebs (SCLC), nicht-kleinzelliger Lungenkrebs (NSCLC), Adenokarzinom der Lunge und Plattenepithelkarzinom der Lunge, Krebs des Peritoneums, Leberzellkrebs, Bauch- oder Magenkrebs, Magen-Darm-Krebs, Bauchspeicheldrüsenkrebs, Glioblastom, Gebärmutterhalskrebs, Eierstockkrebs, Leberkrebs, Blasenkrebs, Hepatom, Brustkrebs, metastatischer Brustkrebs, Kolonkrebs, Mastdarmkrebs, Kolorektalkrebs, Endometrium- oder Gebärmutterkarzinom, Speicheldrüsenkarzinom, Nieren- oder renaler Krebs, Prostatakrebs, Vulvakrebs, Schilddrüsenkrebs, Leberkarzinom, Analkarzinom, Peniskarzinom, Hodenkrebs, Speiseröhrenkrebs, Tumore des Gallengangsystems oder Kopf- und Halskrebs ist.

12. Verfahren nach Anspruch 11, wobei der Krebs Nierenzellkarzinom (RCC) ist.

13. Verfahren nach Anspruch 12, wobei das RCC nicht-klarzelliges Nierenzellkarzinom (nccRCC) oder Translokations-RCC (tRCC) ist.

14. Verfahren nach Anspruch 13, wobei das RCC nccRCC ist.

15. Verfahren nach einem der Ansprüche 1-14, wobei die MITF-Translokation oder -Inversion unter Anwendung eines Verfahrens nachgewiesen wird, das das Durchführen von Genexpressionsprofiling, PCR, rtPCR RNA-Seq, Mikroarrayanalyse, SAGE, MassARRAY-Technik oder FISH an einer Probe umfasst.

16. Verfahren nach einem der Ansprüche 1-14, wobei die MITF-Translokation oder -Inversion durch Nachweisen von DNA, mRNA, cDNA, Proteinen, Proteinfragmenten und/oder Genkopiezahl nachgewiesen wird.

## Revendications

1. Méthode de détermination de l'expression d'une translocation ou d'une inversion d'un gène MITF, comprenant l'étape de détermination si un échantillon provenant d'un individu exprime une translocation ou une inversion d'un gène MITF.

2. Méthode selon la revendication 1, dans laquelle la translocation de MITF comprend ACTG1 et MITF.

3. Méthode selon la revendication 2, dans laquelle la translocation de MITF comprend l'exon 3 d'ACTG1, l'exon 3 d'ACTG1 et l'exon 3 de MITF, la SEQ ID NO : 13 et/ou 30, ou dans laquelle la translocation de MITF est détectable par des amorces qui sont constituées des ou comprennent les SEQ ID NO : 9, 10, 11 et/ou 12.

4. Méthode selon la revendication 1, dans laquelle la translocation de MITF est entraînée par le promoteur d'ACTG1.

5. Méthode selon la revendication 1, dans laquelle la translocation de MITF comprend AP3S1 et MITF, l'exon 3 d'AP3S1 ou l'exon 3 d'ACTG1 et l'exon 3 de MITF.

6. Méthode selon l'une quelconque des revendications 1 à 3 et 5, dans laquelle la translocation de MITF est entraînée par le promoteur d'AP3S1.

7. Méthodes selon l'une quelconque des revendications 1 à 6 dans lesquelles la translocation de MiT entraîne une augmentation des niveaux d'expression de MET, une augmentation de l'activité et/ou de l'activation de MET, une augmentation des niveaux d'expression de BIRC7 ou une augmentation de l'activité et/ou de l'activation de BIRC7.

8. Méthode selon l'une quelconque des revendications 1 à 7, dans laquelle la translocation est une translocation somatique, une translocation intrachromosomique, une translocation interchromosomique, une inversion ou une délétion.

9. Méthode selon l'une quelconque des revendications 1 à 8, dans laquelle la translocation est un polynucléotide de fusion de translocation et/ou un polypeptide de fusion de translocation fonctionnelle.

10. Méthode selon l'une quelconque des revendications 1 à 9, dans laquelle l'échantillon est un échantillon de cancer.

11. Méthode selon la revendication 10, dans laquelle le cancer est un cancer à cellules squameuses, un cancer du poumon, un cancer du poumon à petites cellules (CPPC), un cancer du poumon non à petites cellules (CPNPC), un adénocarcinome du poumon et un carcinome squameux du poumon, un cancer du péritoine, un cancer hépatocellulaire, un cancer gastrique ou de l'estomac, un cancer gastro-intestinal, un cancer du pancréas, un glioblastome, un cancer du col de l'utérus, un cancer de l'ovaire, un cancer du foie, un cancer de la vessie, un hépatome, un cancer du sein, un cancer du sein métastatique, un cancer du côlon, un cancer rectal, un cancer colorectal, un carcinome de l'endomètre ou de l'utérus, un carcinome de la glande salivaire, un cancer du rein ou rénal, un cancer de la prostate, un cancer de la vulve, un cancer de la thyroïde, un carcinome hépatique, un carcinome anal, un carcinome du pénis, un cancer du testicule, un cancer de l'œsophage, des tumeurs des voies biliaires ou un cancer de la tête et du cou.

12. Méthode selon la revendication 11, dans laquelle le cancer est un carcinome à cellules rénales (CCR).

13. Méthode selon la revendication 12, dans laquelle le CCR est un carcinome à cellules rénales non à cellules claires (CCRncc) ou un CCR à translocation (CCRt).

14. Méthode selon la revendication 13, dans laquelle le CCR est un CCRncc.

15. Méthode selon l'une quelconque des revendications 1 à 14, dans laquelle la translocation ou l'inversion de MITF est détectée en utilisant une méthode comprenant la mise en œuvre d'un profilage de l'expression génique, d'une PCR, d'une rtPCR à séquençage d'ARN, d'une analyse par puce à ADN, d'une SAGE, d'une technique MassARRAY ou d'une FISH sur un échantillon.

16. Méthode selon l'une quelconque des revendications 1 à 14, dans laquelle la translocation ou l'inversion de MITF est détectée par détection d'ADN, d'ARNm, d'ADNc, de protéines, de fragments de protéines et/ou du nombre de copies du gène.


### SEQUENCE LISTING

<110> GENENTECH, INC. ET AL.
<120> MIT BIOMARKERS AND METHODS USING THE SAME
<130> P5829R1-WO
<140>
   <141>
<150> 62/109,775
   <151> 2015-01-30
<150> 62/059,362
   <151> 2014-10-03
<150> 62/002,612
   <151> 2014-05-23
<160> 57
<170> PatentIn version 3.5
<210> 1
   <211> 419
   <212> PRT
   <213> Homo sapiens
<400> 1
<210> 2
   <211> 4472
   <212> DNA
   <213> Homo sapiens
<400> 2
   ctcgggatac cttgtttata gtaccttctc tttgccagtc catcttcaaa ttggaattat      60
   agaaagtaga gggagggata gtctaccgtc tctcactgga ttggtgccac ctaaaacatt     120
   gttatgctgg aaatgctaga atataatcac tatcaggtgc agacccacct cgaaaacccc     180
   accaagtacc acatacagca agcccaacgg cagcaggtaa agcagtacct ttctaccact     240
   ttagcaaata aacatgccaa ccaagtcctg agcttgccat gtccaaacca gcctggcgat     300
   catgtcatgc caccggtgcc ggggagcagc gcacccaaca gccccatggc tatgcttacg     360
   cttaactcca actgtgaaaa agagggattt tataagtttg aagagcaaaa cagggcagag     420
   agcgagtgcc caggcatgaa cacacattca cgagcgtcct gtatgcagat ggatgatgta     480
   atcgatgaca tcattagcct agaatcaagt tataatgagg aaatcttggg cttgatggat     540
   cctgctttgc aaatggcaaa tacgttgcct gtctcgggaa acttgattga tctttatgga     600
   aaccaaggtc tgcccccacc aggcctcacc atcagcaact cctgtccagc caaccttccc     660
   aacataaaaa gggagctcac agcgtgtatt tttcccacag agtctgaagc aagagcactg     720
   gccaaagaga ggcagaaaaa ggacaatcac aacctgattg aacgaagaag aagatttaac     780
   ataaatgacc gcattaaaga actaggtact ttgattccca agtcaaatga tccagacatg     840
   cgctggaaca agggaaccat cttaaaagca tccgtggact atatccgaaa gttgcaacga     900
   gaacagcaac gcgcaaaaga acttgaaaac cgacagaaga aactggagca cgccaaccgg     960
   catttgttgc tcagaataca ggaacttgaa atgcaggctc gagctcatgg actttccctt    1020
   attccatcca cgggtctctg ctctccagat ttggtgaatc ggatcatcaa gcaagaaccc    1080
   gttcttgaga actgcagcca agacctcctt cagcatcatg cagacctaac ctgtacaaca    1140
   actctcgatc tcacggatgg caccatcacc ttcaacaaca acctcggaac tgggactgag    1200
   gccaaccaag cctatagtgt ccccacaaaa atgggatcca aactggaaga catcctgatg    1260
   gacgacaccc tttctcccgt cggtgtcact gatccactcc tttcctcagt gtcccccgga    1320
   gcttccaaaa caagcagccg gaggagcagt atgagcatgg aagagacgga gcacacttgt    1380
   tagcgaatcc tccctgcact gcattcgcac aaactgcttc ctttcttgat tcgtagattt 1440
   aataacttac ctgaaggggt tttcttgata attttccttt aatatgaaat tttttttcat 1500
   gctttatcaa tagcccagga tatattttat ttttagaatt ttgtgaaaca gacttgtata 1560
   ttctatttta caactacaaa tgcctccaaa gtattgtaca aataagtgtg cagtatctgt 1620
   gaactgaatt caccacagac tttagctttc tgagcaagag gattttgcgt cagagaaatg 1680
   tctgtccatt tttattcagg ggaaacttga tttgagattt ttatgcctgt gacttccttg 1740
   gaaatcaaat gtaaagttta attgaaagaa tgtaaagcaa ccaaaaagaa aaaaaaaaag 1800
   aaagaaagag gaaaagaaat ccatactaac ccttttccat tttataaatg tattgattca 1860
   ttggtactgc cttaaagata cagtacccct ctagctttgt ttagtcttta tactgcaaac 1920
   tatttaaaga aatatgtatt ctgtaaaaga aaaaaaaaat gcggcctttt catgaggatc 1980
   gtctggttag aaaacataac tgataccaac cgaaactgaa gggagttaga ccaaggctct 2040
   gaaatataaa gtctaatctt gctctctttt attctgtgct gttacagttt tcttcatcaa 2100
   tgagtgtgat ccagtttttc ataagatatt ttattttgaa atggaaatta atgtcctctc 2160
   aaagtaaaat attgaggagc actgaaagta tgttttactt tttttttatt ttatttttgc 2220
   ttttgataag aaaaccgaac tgggcatatt tctaattggc tttactattt ttatttttaa 2280
   attatgtttt actgttcatt tgatttgtac agattcttta ttatcattgt tcttttcaat 2340
   atatttgtat taatttgtaa gaatatgcat cttaaaatgg caagttttcc atatttttac 2400
   aactcactgg tggttttccg cattctttgt acacccatga aagaaaactt ttatgcaagg 2460
   tcttgcattt aaaagacagc tttgcgaata ttttgtaaat tacagtctca ctcagaactg 2520
   tttttggaca catttaaggt gtagtattaa taggttaaaa ccaggctttc tagaaagaat 2580
   aaacttacat atttattttt aggacatgaa aatagcaata ttcttggaga ttgataacca 2640
   tagcattaat acgcccatta tggtcattta aattggggtt tatttcagca aacttgttga 2700
   atttattttt aagaaagaaa tactgtattg ggaagttact gttacttgat aacaatgttt 2760
   taacaagaag caatgttata aagttagttt cagtgcatta tctacttgtg tagtcctatg 2820
   caataacagt agtgttacat gtatcaagcc tagatgtttt atacagatgc catatagtgt 2880
   tatgagccag gctgttgaat ggaatttctc agtagcagcc tacaactgaa tagcaagtgg 2940
   cataaagcat atccattcag aatgaagtgc cttaaatata gcagtagtct tttttggact 3000
   agcactgact gaactgtaat gtaggggaaa gtttcatgat ggtatctata gtcaagacga 3060
   acatgtagca tggtgcctat gtagacaata taagagcttc caattttcct tcagatattt 3120
   ttaatattaa atatatttta gtgacagagt gccaacttct ttcatcagga aaccttattc 3180
   aggagggttt ttaaaaagtg tttaaatgtc aaatgtgaat tggtgatggg tgatggaggg 3240
   ttcagagagg agtgatcgtc agatgtgtga atggacggtt taggtgaaaa taatcaactg 3300
   catagttccc atgcacgctg ggcaatgaga atccttggaa acattggtga tgctatcagt 3360
   tttatagctt tatttcttaa gggggtaggg aaaattagtt cccattcttt caaccccctt 3420
   aactgtatag ctcttttcct agaatagtga cgcaaatctg catgaacagc taattgtacc 3480
   atagtgttca ttgatacaat catagcattg tctatttttc tcttcatatt tatatggggg 3540
   ggagggcgct ggatgcaaaa gttgaagatc gtgatgctat gatgttagtt ttccttagct 3600
   gattttgagg gtttttaaaa ataaagcaag gttgactaac ctacggccac gggaacagga 3660
   ccatggttaa gcaaccatat agaaagcttt gttgaaagaa agtatggcat cttgtaccac 3720
   tgccctgact gtcacaactc ctaaccttgc cattgcctgc ctccccctcc ccttctcctt 3780
   aagagacaat ttctgcaggt ggcaggtgag caagcccagg agaatgctgc aatcttgggg 3840
   gtggttttat ttatttcttt tttgccaaat agagtgtgga ttcatttcag gggctagcta 3900
   agccaagagg cagtggtttg ggcttgttgt ttgtaacaag aaaatgatcc acaccactcc 3960
   cccgattccc gggtgcagaa ttgtaactcg gggttgggcc tctatatgga gtgaccaaaa 4020
   tgccaaaatt gtccatctgc ctctgagtag ggcaatggaa ataccaaacc ttctgacttt 4080
   gccaaaaagc atacaagcaa cctggtcata cataggatga caaaattctt tctggttgtt 4140
   tttaaacaat aaagcaataa gaacaaatac aatacatagg aagttaaaag cacaaaggaa 4200
   tgaacttatt aatatttttg aaaaatgcac tgggaaaaag ttgatgtcaa taacagtata 4260
   aaacagccct atttcttgat aaaaaatgac aaatgactgt ctcttgcgga tgcttggtac 4320
   tgtaatgtta ataatagtca cctgctgttg gatgcagcaa taatttctgt atggtccata 4380
   gcactgtata ttatggatcg atattaatgt atccaatgaa ataatcgact tgttcttgat 4440
   agcctcatta aagcatttgg tttttcacat ag 4472
<210> 3
   <211> 476
   <212> PRT
   <213> Homo sapiens
<400> 3
   Met Ala Ser Arg Ile Gly Leu Arg Met Gln Leu Met Arg Glu Gln Ala
   1               5                   10                  15
   Gln Gln Glu Glu Gln Arg Glu Arg Met Gln Gln Gln Ala Val Met His
               20                  25                  30
   Tyr Met Gln Gln Gln Gln Gln Gln Gln Gln Gln Gln Leu Gly Gly Pro
           35                  40                  45
   Pro Thr Pro Ala Ile Asn Thr Pro Val His Phe Gln Ser Pro Pro Pro
       50                  55                  60
   Val Pro Gly Glu Val Leu Lys Val Gln Ser Tyr Leu Glu Asn Pro Thr
   65                  70                  75                  80
   Ser Tyr His Leu Gln Gln Ser Gln His Gln Lys Val Arg Glu Tyr Leu
                   85                  90                  95
   Ser Glu Thr Tyr Gly Asn Lys Phe Ala Ala His Ile Ser Pro Ala Gln
               100                 105                 110
   Gly Ser Pro Lys Pro Pro Pro Ala Ala Ser Pro Gly Val Arg Ala Gly
           115                 120                 125
   His Val Leu Ser Ser Ser Ala Gly Asn Ser Ala Pro Asn Ser Pro Met
       130                 135                 140
   Ala Met Leu His Ile Gly Ser Asn Pro Glu Arg Glu Leu Asp Asp Val
   145                 150                 155                 160
   Ile Asp Asn Ile Met Arg Leu Asp Asp Val Leu Gly Tyr Ile Asn Pro
                   165                 170                 175
   Glu Met Gln Met Pro Asn Thr Leu Pro Leu Ser Ser Ser His Leu Asn
               180                 185                 190
   Val Tyr Ser Ser Asp Pro Gln Val Thr Ala Ser Leu Val Gly Val Thr
           195                 200                 205
   Ser Ser Ser Cys Pro Ala Asp Leu Thr Gln Lys Arg Glu Leu Thr Asp
       210                 215                 220
   Ala Glu Ser Arg Ala Leu Ala Lys Glu Arg Gln Lys Lys Asp Asn His
   225                 230                 235                 240
   Asn Leu Ile Glu Arg Arg Arg Arg Phe Asn Ile Asn Asp Arg Ile Lys
                   245                 250                 255
   Glu Leu Gly Met Leu Ile Pro Lys Ala Asn Asp Leu Asp Val Arg Trp
               260                 265                 270
   Asn Lys Gly Thr Ile Leu Lys Ala Ser Val Asp Tyr Ile Arg Arg Met
           275                 280                 285
   Gln Lys Asp Leu Gln Lys Ser Arg Glu Leu Glu Asn His Ser Arg Arg
       290                 295                 300
   Leu Glu Met Thr Asn Lys Gln Leu Trp Leu Arg Ile Gln Glu Leu Glu
   305                 310                 315                 320
   Met Gln Ala Arg Val His Gly Leu Pro Thr Thr Ser Pro Ser Gly Met
                   325                 330                 335
   Asn Met Ala Glu Leu Ala Gln Gln Val Val Lys Gln Glu Leu Pro Ser
               340                 345                 350
   Glu Glu Gly Pro Gly Glu Ala Leu Met Leu Gly Ala Glu Val Pro Asp
           355                 360                 365
   Pro Glu Pro Leu Pro Ala Leu Pro Pro Gln Ala Pro Leu Pro Leu Pro
       370                 375                 380
   Thr Gln Pro Pro Ser Pro Phe His His Leu Asp Phe Ser His Ser Leu
   385                 390                 395                 400
   Ser Phe Gly Gly Arg Glu Asp Glu Gly Pro Pro Gly Tyr Pro Glu Pro
                   405                 410                 415
   Leu Ala Pro Gly His Gly Ser Pro Phe Pro Ser Leu Ser Lys Lys Asp
               420                 425                 430
   Leu Asp Leu Met Leu Leu Asp Asp Ser Leu Leu Pro Leu Ala Ser Asp
           435                 440                 445
   Pro Leu Leu Ser Thr Met Ser Pro Glu Ala Ser Lys Ala Ser Ser Arg
       450                 455                 460
   Arg Ser Ser Phe Ser Met Glu Glu Gly Asp Val Leu
   465                 470                 475
<210> 4
   <211> 4728
   <212> DNA
   <213> Homo sapiens
<400> 4
   ggccgggcgg gcatgggcct tcccggcccg gagctgggag tcgaaggggc gggaggcgtg        60
   atggtgaact cgcaagaagt ttgagggacg cgcgggcccc gcgcccactc cccctccacc       120
   ggacacggct ggggccggcg atgcctgaga gggggtcgga ggacgcagtg aacatatatg       180
   catgtacagt gtggatcctc atctgagagg agggagatga aaacacaccc acctcacagg       240
   ctgttgtgag gactaagggt gcggcagtgc ctggtacatg ggagccagcg ccggcagcca       300
   ccatggcgtc acgcataggg ttgcgcatgc agctcatgcg ggagcaggcg cagcaggagg       360
   agcagcggga gcgcatgcag caacaggctg tcatgcatta catgcagcag cagcagcagc       420
   agcaacagca gcagctcgga gggccgccca ccccggccat caataccccc gtccacttcc 480
   agtcgccacc acctgtgcct ggggaggtgt tgaaggtgca gtcctacctg gagaatccca 540
   catcctacca tctgcagcag tcgcagcatc agaaggtgcg ggagtacctg tccgagacct 600
   atgggaacaa gtttgctgcc cacatcagcc cagcccaggg ctctccgaaa cccccaccag 660
   ccgcctcccc aggggtgcga gctggacacg tgctgtcctc ctccgctggc aacagtgctc 720
   ccaatagccc catggccatg ctgcacattg gctccaaccc tgagagggag ttggatgatg 780
   tcattgacaa cattatgcgt ctggacgatg tccttggcta catcaatcct gaaatgcaga 840
   tgcccaacac gctacccctg tccagcagcc acctgaatgt gtacagcagc gacccccagg 900
   tcacagcctc cctggtgggc gtcaccagca gctcctgccc tgcggacctg acccagaagc 960
   gagagctcac agatgctgag agcagggccc tggccaagga gcggcagaag aaagacaatc 1020
   acaacttaat tgaaaggaga cgaaggttca acatcaatga ccgcatcaag gagttgggaa 1080
   tgctgatccc caaggccaat gacctggacg tgcgctggaa caagggcacc atcctcaagg 1140
   cctctgtgga ttacatccgg aggatgcaga aggacctgca aaagtccagg gagctggaga 1200
   accactctcg ccgcctggag atgaccaaca agcagctctg gctccgtatc caggagctgg 1260
   agatgcaggc tcgagtgcac ggcctcccta ccacctcccc gtccggcatg aacatggctg 1320
   agctggccca gcaggtggtg aagcaggagc tgcctagcga agagggccca ggggaggccc 1380
   tgatgctggg ggctgaggtc cctgaccctg agccactgcc agctctgccc ccgcaagccc 1440
   cgctgcccct gcccacccag ccaccatccc cattccatca cctggacttc agccacagcc 1500
   tgagctttgg gggcagggag gacgagggtc ccccgggcta ccccgaaccc ctggcgccgg 1560
   ggcatggctc cccattcccc agcctgtcca agaaggatct ggacctcatg ctcctggacg 1620
   actcactgct accgctggcc tctgatccac ttctgtccac catgtccccc gaggcctcca 1680
   aggccagcag ccgccggagc agcttcagca tggaggaggg cgatgtgctg tgaccctggc 1740
   tgcccctgtg ccagggaaca ggggccggcc tgggggctgg gagggccagg ggcacctccc 1800
   tcccaccctt caggctgcac tgtgtgtgaa gtagccacct gccctgcctc cctcctcccc 1860
   gttggcccct gtttggactt agtgcctgtc tggcagcctg tggggtcagg agaagcaccc 1920
   ccagggcagc cctcttgact ggcgcagtgg gaagaggcct tcagcccctc tcccggagat 1980
   ggaatcgcgg ggcagggagg ggcagggtgt tctagaggtg agaagagggc ctggtggaga 2040
   ttccctgtct tctgagcccg agcccctcat taccagtgaa ggacatgctt gaggggttcg 2100
   ggaagctcct catctgaggc aactggtcct gggggtgctc aggcctgcct ttttgggact 2160
   cagatggcag gaggtccacc ccgcagcctg gtcctcggct ctcccacagg tgggcacccc 2220
   ccactttggt gctaatagct ctccaccagg tggtgtgagc gcgggggctg ccagaagcgg 2280
   gaggggtcac tgccggaaga gcagctgccc tccgacccct cactttgtgc ctttagtaaa 2340
   cactgtgctt tgtaaaaaaa aaaaggccgg gcgggcatgg gccttcccgg cccggagctg 2400
   ggagtcgaag gggcgggagg cgtgatggtg aactcgcaag aagtttgagg gacgcgcggg 2460
   ccccgcgccc actccccctc caccggacac ggctggggcc ggcgatgcct gagagggggt 2520
   cggaggacgc agtgaacata tatgcatgta cagtgtggat cctcatctga gaggagggag 2580
   atgaaaacac acccacctca caggctgttg tgaggactaa gggtgcggca gtgcctggta 2640
   catgggagcc agcgccggca gccaccatgg cgtcacgcat agggttgcgc atgcagctca 2700
   tgcgggagca ggcgcagcag gaggagcagc gggagcgcat gcagcaacag gctgtcatgc 2760
   attacatgca gcagcagcag cagcagcaac agcagcagct cggagggccg cccaccccgg 2820
   ccatcaatac ccccgtccac ttccagtcgc caccacctgt gcctggggag gtgttgaagg 2880
   tgcagtccta cctggagaat cccacatcct accatctgca gcagtcgcag catcagaagg 2940
   tgcgggagta cctgtccgag acctatggga acaagtttgc tgcccacatc agcccagccc 3000
   agggctctcc gaaaccccca ccagccgcct ccccaggggt gcgagctgga cacgtgctgt 3060
   cctcctccgc tggcaacagt gctcccaata gccccatggc catgctgcac attggctcca 3120
   accctgagag ggagttggat gatgtcattg acaacattat gcgtctggac gatgtccttg 3180
   gctacatcaa tcctgaaatg cagatgccca acacgctacc cctgtccagc agccacctga 3240
   atgtgtacag cagcgacccc caggtcacag cctccctggt gggcgtcacc agcagctcct 3300
   gccctgcgga cctgacccag aagcgagagc tcacagatgc tgagagcagg gccctggcca 3360
   aggagcggca gaagaaagac aatcacaact taattgaaag gagacgaagg ttcaacatca 3420
   atgaccgcat caaggagttg ggaatgctga tccccaaggc caatgacctg gacgtgcgct 3480
   ggaacaaggg caccatcctc aaggcctctg tggattacat ccggaggatg cagaaggacc 3540
   tgcaaaagtc cagggagctg gagaaccact ctcgccgcct ggagatgacc aacaagcagc 3600
   tctggctccg tatccaggag ctggagatgc aggctcgagt gcacggcctc cctaccacct 3660
   ccccgtccgg catgaacatg gctgagctgg cccagcaggt ggtgaagcag gagctgccta 3720
   gcgaagaggg cccaggggag gccctgatgc tgggggctga ggtccctgac cctgagccac 3780
   tgccagctct gcccccgcaa gccccgctgc ccctgcccac ccagccacca tccccattcc 3840
   atcacctgga cttcagccac agcctgagct ttgggggcag ggaggacgag ggtcccccgg 3900
   gctaccccga acccctggcg ccggggcatg gctccccatt ccccagcctg tccaagaagg 3960
   atctggacct catgctcctg gacgactcac tgctaccgct ggcctctgat ccacttctgt 4020
   ccaccatgtc ccccgaggcc tccaaggcca gcagccgccg gagcagcttc agcatggagg 4080
   agggcgatgt gctgtgaccc tggctgcccc tgtgccaggg aacaggggcc ggcctggggg 4140
   ctgggagggc caggggcacc tccctcccac ccttcaggct gcactgtgtg tgaagtagcc 4200
   acctgccctg cctccctcct ccccgttggc ccctgtttgg acttagtgcc tgtctggcag 4260
   cctgtggggt caggagaagc acccccaggg cagccctctt gactggcgca gtgggaagag 4320
   gccttcagcc cctctcccgg agatggaatc gcggggcagg gaggggcagg gtgttctaga 4380
   ggtgagaaga gggcctggtg gagattccct gtcttctgag cccgagcccc tcattaccag 4440
   tgaaggacat gcttgagggg ttcgggaagc tcctcatctg aggcaactgg tcctgggggt 4500
   gctcaggcct gcctttttgg gactcagatg gcaggaggtc caccccgcag cctggtcctc 4560
   ggctctccca caggtgggca ccccccactt tggtgctaat agctctccac caggtggtgt 4620
   gagcgcgggg gctgccagaa gcgggagggg tcactgccgg aagagcagct gccctccgac 4680
   ccctcacttt gtgcctttag taaacactgt gctttgtaaa aaaaaaaa 4728
<210> 5
   <211> 575
   <212> PRT
   <213> Homo sapiens
<400> 5
<210> 6
   <211> 3467
   <212> DNA
   <213> Homo sapiens
<400> 6
   ttcctgtgga gtttccccat ccctgggagg agggaggagg gggaagaaga cgagggggag      60
   gagggcggtc gtccggggtt aggttgaggg ggggcgtcgg tccgttctgg gcgggggatg     120
   actcacagcc catcccatct ccccgacgcc gcccgcccgc gcagtgctag ctccatggct     180
   tagcggagga ggcggcggtg gcgagctggg gggagggggg actcttattt tgttaggggg     240
   accgggccga ggcccgaccg gcctggcagg gctcgcccgg ggccgggcgt catgtctcat     300
   gcggccgaac cagctcggga tggcgtagag gccagcgcgg agggccctcg agccgtgttc     360
   gtgctgttgg aggagcgcag gccggccgac tcggctcagc tgctcagcct gaactctttg     420
   cttccggaat ccgggattgt tgctgacata gaattagaaa acgtccttga tcctgacagc 480
   ttctacgagc tcaaaagcca acccttaccc cttcgctcaa gcctcccaat atcactgcag 540
   gccacaccag ccaccccagc tacactctct gcatcgtctt ctgcaggggg ctccaggacc 600
   cctgccatgt cgtcatcttc ttcatcgagg gtcttgctgc ggcagcagct aatgcgggcc 660
   caggcgcagg agcaggagag gcgtgagcgt cgggaacagg ccgccgcggc tcccttcccc 720
   agtcctgcac ctgcctctcc tgccatctct gtggttggcg tctctgctgg gggccacaca 780
   ttgagccgtc caccccctgc tcaggtgccc agggaggtgc tcaaggtgca gacccatctg 840
   gagaacccaa cgcgctacca cctgcagcag gcgcgccggc agcaggtgaa acagtacctg 900
   tccaccacac tcgggcccaa gctggcttcc caggccctca ccccaccgcc ggggcccgca 960
   agtgcccagc cactgcctgc ccctgaggct gcccacacta ccggccccac aggcagtgcg 1020
   cccaacagcc ccatggcgct gctcaccatc gggtccagct cagagaagga gattgatgat 1080
   gtcattgatg agatcatcag cctggagtcc agttacaatg atgaaatgct cagctatctg 1140
   cccggaggca ccacaggact gcagctcccc agcacgctgc ctgtgtcagg gaatctgctt 1200
   gatgtgtaca gtagtcaagg cgtggccaca ccagccatca ctgtcagcaa ctcctgccca 1260
   gctgagctgc ccaacatcaa acgggagatc tctgagaccg aggcaaaggc ccttttgaag 1320
   gaacggcaga agaaagacaa tcacaaccta attgagcgtc gcaggcgatt caacattaac 1380
   gacaggatca aggaactggg cactctcatc cctaagtcca gtgacccgga gatgcgctgg 1440
   aacaagggca ccatcctgaa ggcctctgtg gattatatcc gcaagctgca gaaggagcag 1500
   cagcgctcca aagacctgga gagccggcag cgatccctgg agcaggccaa ccgcagcctg 1560
   cagctccgaa ttcaggaact agaactgcag gcccagatcc atggcctgcc agtacctccc 1620
   actccagggc tgctttcctt ggccacgact tcggcttctg acagcctcaa gccagagcag 1680
   ctggacattg aggaggaggg caggccaggc gcagcaacgt tccatgtagg ggggggacct 1740
   gcccagaatg ctccccatca gcagccccct gcaccgccct cagatgccct tctggacctg 1800
   cactttccca gcgaccacct gggggacctg ggagacccct tccacctggg gctggaggac 1860
   attctgatgg aggaggagga gggggtggtg ggaggactgt cggggggtgc cctgtcccca 1920
   ctgcgggctg cctccgatcc cctgctctct tcagtgtccc ctgctgtctc caaggccagc 1980
   agccgccgca gcagcttcag catggaagag gagtcctgat caggcctcac ccctcccctg 2040
   ggactttccc acccaggaaa ggaggaccag tcaggatgag gccccgcctt ttcccccacc 2100
   ctcccatgag actgccctgc ccaggtatcc tgggggaaga ggagatgtga tcaggcccca 2160
   cccctgtaat caggcaagga ggaggagtca gatgaggccc tgcaccttcc ccaaaggaac 2220
   cgcccagtgc aggtatttca gaaggagaag gctggagaag gacatgagat cagggcctgc 2280
   cccctgggga tcacagcctc acccctgccc ctgtgggact catccttgcc caggtgaggg 2340
   aaggagacag gatgaggtct cgaccctgtc ccctagggac tgtcctagcc aggtctcctg 2400
   ggaaagggag atgtcaggat gttgctccat cctttgtctt ggaaccacca gtctagtccg 2460
   tcctggcaca gaagaggagt caagtaatgg aggtcccagc cctgggggtt taagctctgc 2520
   cccttcccca tgaaccctgc cctgctctgc ccaggcaagg aacagaagtg aggatgagac 2580
   ccagcccctt cccctgggaa ctctcctggc cttctaggaa tggaggagcc aggccccacc 2640
   ccttccctat aggaacagcc cagcacaggt atttcaggtg tgaaagaatc agtaggacca 2700
   ggccaccgct agtgcttgtg gagatcacag ccccacccct gtccctcagc aacatcccat 2760
   ctaagcattc cacactgcag ggaggagtgg tacttaagct cccctgcctt aacctgggac 2820
   caacctgacc taacctagga gggctctgag ccaaccttgc tcttggggaa ggggacagat 2880
   tatgaaattt catggatgaa ttttccagac ctatatctgg agtgagaggc ccccaccctt 2940
   gggcagagtc ctgccttctt ccttgagggg cagtttggga aggtgatggg tattagtggg 3000
   ggactgagtt caggttacca gaaccagtac ctcagtattc tttttcaaca tgtagggcaa 3060
   gaggatgaag gaaggggcta tcctgggacc tccccagccc aggaaaaact ggaagccttc 3120
   ccccagcaag gcagaagctt ggaggagggt tgtaaaagca tattgtaccc cctcatttgt 3180
   ttatctgatt tttttattgc tccgcatact gagaatctag gccaccccaa cctctgttcc 3240
   ccacccagtt cttcatttgg aggaatcacc ccatttcaga gttatcaaga gacactcccc 3300
   cctccattcc cacccctcat acctacaccc aaggttgtca gctttggatt gctggggcca 3360
   ggccccatgg agggtatact gaggggtcta taggtttgtg attaaaataa taaaagctag 3420
   gcgtgtttga tgcgctttta actttggcaa aaaaaaaaaa aaaaaaa 3467
<210> 7
   <211> 347
   <212> PRT
   <213> Homo sapiens
<400> 7
   Met Thr Leu Asp His Gln Ile Ile Asn Pro Thr Leu Lys Trp Ser Gln
   1               5                   10                  15
   Pro Ala Val Pro Ser Gly Gly Pro Leu Val Gln His Ala His Thr Thr
               20                  25                  30
   Leu Asp Ser Asp Ala Gly Leu Thr Glu Asn Pro Leu Thr Lys Leu Leu
           35                  40                  45
   Ala Ile Gly Lys Glu Asp Asp Asn Ala Gln Trp His Met Glu Asp Val
       50                  55                  60
   Ile Glu Asp Ile Ile Gly Met Glu Ser Ser Phe Lys Glu Glu Gly Ala
   65                  70                  75                  80
   Asp Ser Pro Leu Leu Met Gln Arg Thr Leu Ser Gly Ser Ile Leu Asp
                   85                  90                  95
   Val Tyr Ser Gly Glu Gln Gly Ile Ser Pro Ile Asn Met Gly Leu Thr
               100                 105                 110
   Ser Ala Ser Cys Pro Ser Ser Leu Pro Met Lys Arg Glu Ile Thr Glu
           115                 120                 125
   Thr Asp Thr Arg Ala Leu Ala Lys Glu Arg Gln Lys Lys Asp Asn His
       130                 135                 140
   Asn Leu Ile Glu Arg Arg Arg Arg Tyr Asn Ile Asn Tyr Arg Ile Lys
   145                 150                 155                 160
   Glu Leu Gly Thr Leu Ile Pro Lys Ser Asn Asp Pro Asp Met Arg Trp
                   165                 170                 175
   Asn Lys Gly Thr Ile Leu Lys Ala Ser Val Glu Tyr Ile Lys Trp Leu
               180                 185                 190
   Gln Lys Glu Gln Gln Arg Ala Arg Glu Leu Glu His Arg Gln Lys Lys
           195                 200                 205
   Leu Glu Gln Ala Asn Arg Arg Leu Leu Leu Arg Ile Gln Glu Leu Glu
       210                 215                 220
   Ile Gln Ala Arg Thr His Gly Leu Pro Thr Leu Ala Ser Leu Gly Thr
   225                 230                 235                 240
   Val Asp Leu Gly Ala His Val Thr Lys Gln Gln Ser His Pro Glu Gln
                   245                 250                 255
   Asn Ser Val Asp Tyr Cys Gln Gln Leu Thr Val Ser Gln Gly Pro Ser
               260                 265                 270
   Pro Glu Leu Cys Asp Gln Ala Ile Ala Phe Ser Asp Pro Leu Ser Tyr
           275                 280                 285
   Phe Thr Asp Leu Ser Phe Ser Ala Ala Leu Lys Glu Glu Gln Arg Leu
       290                 295                 300
   Asp Gly Met Leu Leu Asp Asp Thr Ile Ser Pro Phe Gly Thr Asp Pro
   305                 310                 315                 320
   Leu Leu Ser Ala Thr Ser Pro Ala Val Ser Lys Glu Ser Ser Arg Arg
                   325                 330                 335
   Ser Ser Phe Ser Ser Asp Asp Gly Asp Glu Leu
               340                 345
<210> 8
   <211> 6700
   <212> DNA
   <213> Homo sapiens
<400> 8
   acttctcttt tctctttcct caactaactg gattccaacc agcccaaagt attcattact      60
   cacggctaga tcgtttactt tggttgtccc ttctggcatg gtgcatatgt tatgggaaga     120
   gggattataa tttggtgctg tttgtagaga tgacaacact gataaaatcc actcattgct     180
   ggtcccagca cacctggaaa gttctgcaag gcctcagcta cagaaagccc agagacagaa     240
   agtaaactct ttcatgaccc ttgatcatca gatcatcaat ccaactctta aatggtcaca     300
   acctgcagtg ccaagtggtg ggcctcttgt gcagcatgca cacacaactc tggacagtga     360
   tgctggcctc acagaaaacc cactcaccaa gttactagct attgggaaag aagatgacaa     420
   tgcacaatgg catatggagg acgttattga ggatataatc ggtatggaat caagttttaa     480
   agaggaagga gcagactctc ctctgctaat gcaaagaaca ttatctggaa gtattttgga     540
   tgtgtatagc ggtgaacaag gaatttcacc aattaacatg gggcttacaa gtgcttcttg     600
   tccaagtagt ctaccaatga aaagagaaat tacagaaact gacactagag ctttagcaaa     660
   agagagacaa aaaaaggaca accacaacct cattgaaaga agaagaaggt ataatattaa     720
   ttaccgaatc aaggagcttg gcactcttat tccaaagtct aatgatcctg atatgcgctg     780
   gaacaaagga accattctaa aagcatcagt ggagtacatc aagtggctac aaaaagaaca     840
   acagagagcc cgagaattgg aacacagaca gaagaaatta gagcaggcta acaggcgact     900
   tctacttcgg attcaggaac tagaaattca ggctcgtact catggtctgc caaccctggc     960
   ttcacttggc acggttgatt taggtgctca tgtcaccaaa cagcagagcc atcctgagca    1020
   gaattcagta gactattgcc aacaactgac tgtgtctcag gggccaagcc ctgagctctg    1080
   tgatcaagct atagcctttt ctgatccttt gtcatacttc acagatttat catttagtgc    1140
   tgcattgaaa gaggaacaaa gattggatgg catgctattg gatgacacaa tctctccatt    1200
   tggaacagat cctctgctat ctgccacttc ccctgcagtt tccaaagaaa gcagtaggag    1260
   aagtagcttt agctcagatg atggtgatga attataagaa ataaacagac ccaattcatc    1320
   aactggaaag caattctatg ctggtgctat gcaattatgc tctgtgtttc atatgttgct    1380
   ttggcttatt ttttttctta aaggaatgtg ttgttcatga aaaactgata gaagcaacag    1440
   aagaattcgc aggaagaaaa atcatagtgt taatgaatta ttgagggcga aaaaaaggtg    1500
   ttttcttctt tgactacgga gtccaaatcc acttaaattc tgttttcctg aaaagaggta 1560
   cagcataaga aatagctctt tattgatgtt ttaaaagcag caacttggtg gtgtactact 1620
   ggaactaatg actgcaaagt gttaaacgac tgaaatatac aaacagtctc ttagttactc 1680
   atttccatct tctcttcaac tttcacatca gtcttccgga atcaagatca acatatcagg 1740
   tggtcattgc ctttctccat tgtctagtag acatgtctaa agttcaaact ttataggata 1800
   aataaatgta taatagatta tctgtcactt gtggttgaaa ggcaaatcta caataaatgt 1860
   gagaattttc cacaataaaa tatggataac ttataaaaac attggttact aaaattagat 1920
   cctcatttta ttgtagttgg ttcaattaca ctaattctaa aagcatccat gcatatttat 1980
   atctccagtc tctgttcagg aaaaggaaca tattgaatat tttcctcagg aatatggacc 2040
   agaattgtat cccttcacac aaacatacac atacacatat gcacatcatt caggtagtat 2100
   atgttctttt gttttcttca tgcttctgac tgcatcagaa tcacattcca aattctcttt 2160
   tcttatgaag aagagatgtc agatcatcaa ttttagtaaa taaaatataa aatgtccccc 2220
   tgcaaggaca gttttcaggt acttaaaact tttcatcagt attggacaga aatcaattag 2280
   ttgttgattt ggtttttctc caaatggata aaatattgaa aattgaattg ccaattgaca 2340
   aaataattat tacaacaaac tatttcttat tattttcagt tctgagagga acgtaaggtt 2400
   ctatttctat aaacacttag agtgtcctat gatctttggt tgcactgtta gcatttatta 2460
   taagcactta taactatgat gcttcattta gatttttatc tcttgcgctt gttttaggtt 2520
   aggaaattaa gttaccaagc acgttgctct gtgctggacc tccaagagtg atcatccgat 2580
   caatgagtat tcatggaggc atactcagaa ctgcagtgag tcctgagaaa atgtagaaga 2640
   gttgaaaaag atatggtctt actcctaagg tagttaatga gaatgcatga aaaacaaaaa 2700
   caaaagatag aagacatata gaataaaaaa tctaagcata aactgtaaat acagaaggtc 2760
   agagaaaatg aaaatgactg acaaacttta tgggaacagc agaatttgac ttctctttga 2820
   aaggcattaa ttgagtgaaa gtgagggcat ataaaggaat aataccaacc aaatgataaa 2880
   agtgagaata ataggggaag tatctttata ggactttaat tagaaagatc tggatgccct 2940
   agaagatgtc tgttggggag ttctgaagca taagactgac aacaggttgt gaaggccctt 3000
   agaaaactat ggtgaaataa tatagtatta atataataga aacaaatctt tttagtagca 3060
   gggaggatgc taaaaactaa aatatatatt actaatgagt gagatgtaat ctttcatgaa 3120
   tattaagctt tgggataaat tttgaaagga tcttttagtt gcttctcaca tggaaaatat 3180
   gtcactatga aatgtgagac cctaatgctg ataaaagtag aaaaatagta aatcattatg 3240
   gtataataat tatctcaggc ttagcttgta tcatttgctc agaatttgga gaattaaaat 3300
   aattttattg tttgacatgg aaaaataatt actaacttct gggtacagtc attaaagcct 3360
   gatagataaa ttcatctgaa atatgccatg tgaaacagct attagataca tcttctcaat 3420
   aattttcaag acgatattac atacttttaa ttttataaca aaattccact tcattatctg 3480
   agttgataat ttgattaaat gttaaaaatt atagataagg aatatatttt ggagttcata 3540
   gaaaacacac cactttatta gatagagact ggcttcagtt ctcattttat ctgctcttct 3600
   catttcttca tattttgagt agacttgcac tgatgatgtc attactcagt cattattttc 3660
   tgttctgtta aggtacaact actgggcctt gaaatctatc cttcacaatc ttggctgaga 3720
   taaggacttg gcacaggatg atagagcctg gacacaagag atctggagag ggaactgcta 3780
   tttgctgcta cattcagatt atgagatgtc agggaactgt aaaaagggta atgaattttt 3840
   aaagagagta aagagttatg ctgtgtcccg gtcaactgaa atacattaaa aattaattag 3900
   atggtagttc ttaacttttt aaggcagata ccttgagaat ctagtgagtt atagaccctc 3960
   tccctagaaa agtatagaac tgtttatatg gacaataatt cacatgcatt cagagatctt 4020
   agggatctcc tgaatgtttt cttggatccc aagtgaaaaa ttcctgaatc aggtctctta 4080
   ggtgtgcggt taatgtcatg tataacaatg gggggaccct tacttatcta aatataatta 4140
   tcccaatcct aataatgatg aggttgcata ggaaagtaat agtgtaccaa actttagatt 4200
   attcactaaa aaagttgttc atttatgaag tagtcgttta tcaaacatgt cctccccact 4260
   cagcaaacta tgccttctat ttattatata tgggtcagtt tcactataat tactgagtgt 4320
   tataaacatc tgaccataac attttgaaat gatgcaaata aatttccaaa caaaaatagt 4380
   gtgaatttaa aagcaaatta tttgagtatc taagaaacaa gatagacttc tagaaaaatt 4440
   tgactctcta gaatatttct tgcagaaatg agatttttca taatagtaaa agaggcatat 4500
   gtttatcaaa caatgctgtc acaaaaagca tcaactgtaa tgggactatt aatgcataat 4560
   tattgattta ttcattcaat taatatacaa ttatcccttt tcatttaaag atttaattca 4620
   taattacaat tataataaaa cttcctttaa agtaagatac aataatttta ttgtttttca 4680
   ttcttttttc aacaaaatat cccatccaat cattttttat attattaaat attggctgct 4740
   ttttcttgga ttcacattaa acagcccttt ccaacttcca attgtcttaa aataatgatg 4800
   acctcctgtg agtagataca gctctttaca atttttttct ttagtgcctt ttcttcttga 4860
   atttttccta tatcaaatgg agaatatatg tacagatggt attttctcag tttataggca 4920
   tatcagtgac catggctttc tttatatagg tttttaaaaa agccctaaat aataaatagc 4980
   cagatgagct ggggacattg agaaatagcc ttcctcttcc tttttcaact catttttttc 5040
   ccacctacat gactgtaaat caaatattta atagctctta cttaaaaaaa cagatacaaa 5100
   gaatgtcttg atttggtgtg ctcatttacc ataatgtcat gaggggaatt agatttcaca 5160
   actttaaaag gaatatattt ttattttatt ttgaaaaact gagtcatata ggaattttct 5220
   tatacttcaa ggcatcatgg aaacactttt ttcctgtttg gatattgtgg aatttaaacg 5280
   ttcaaataaa taaatggcat aactaagtgt tccaaatttt tttacaatgt ctttgaccct 5340
   attcaaacac tttaggtatt tactgaccgt ctgatgtgta agatgtggaa taaaactgga 5400
   atcaattaat tatttcactg tgttatcagc gcaagatcaa ccatctgggt ttcttaaaga 5460
   cacccgaagg attgaatttt gtttcagtat tgataatggc atagtctcta tgtgctacat 5520
   ggaattacat catttatccc tccagtgccc tatatgttga taagtatgtc agtttgactt 5580
   agtatacata tatacagaga tttcattaca tttacctaat aaatacaaaa tacatttcga 5640
   gtgttactga tctcctatgt tacatgagcc tcctgtaatc attgtgcatt gatgttccaa 5700
   tgttttattg tttgtatgaa ttttaatttg aaaacaagga aacaatccaa aagcagaaaa 5760
   aatagctttt cttaaaattt tcagtgctac attttccctc tgaggtccat agagatttga 5820
   atgtatagga gattatccga aaacagctat tttgattaaa aaatatatct cccaggattc 5880
   aaccaactta atgatgaagt actattgtct actgctttat acataaaagg gaacttttta 5940
   tctgcttgta aagggatttt tatgtgtatt tctgcataat cagatgactt ctattgtgtt 6000
   ttctactgat gaaattctct gtaaaatgtc tttttcttac attatccaac aggcataaag 6060
   aataacagta aagacttttg tgtttgtaat actacctctt ttatccctgc actactgttt 6120
   tattgcaaaa attctatatt gtcactgtat tttttccata gaatataaat tttgttcttg 6180
   tgctaaagct ggtagtttat gtagcagaca aaatatacaa ataaaagaag agactgattt 6240
   tgctgaaaga attatatata atcaagaagt tacatattgt tctttaaata tgatactgaa 6300
   ttttaaaagc aaacgaaatt caagaatctt atctaacagc atagcagttg cttatggcat 6360
   acaaggctaa aattaattca gctatttaat cttaataatt attatgtagt taaaaatctt 6420
   tgactttaat agtgttttac atatacaaat agctgaagta acattcctat aattttaatc 6480
   tgacattggt tagatcaaga aaacattgtt aataagactg tagaatttgt aattattgct 6540
   atttttcatt tttaataaca aagtaatgtg tcttattttc taagaaaatg gagaactttg 6600
   gtgtacttta atacatacaa aaatctttgt aaaaatacct taaaatgtac caatattttc 6660
   tttgcatata ttaaatgaaa gactataatt atgaaatgtt 6700
<210> 9
   <211> 62
   <212> DNA
   <213> Artificial Sequence
<220>
   <221> source
   <223> /note="Description of Artificial Sequence: Synthetic oligonucleotide"
<400> 9
   cccagcagga gcaggagcag gagcgggagc gggatcccca gcaggagcag gagcgggagc 60
   gg 62
<210> 10
   <211> 65
   <212> DNA
   <213> Artificial Sequence
<220>
   <221> source
   <223> /note="Description of Artificial Sequence: Synthetic oligonucleotide"
<400> 10
   ctgcctgtgt cagggaatct gcttgatgtg tacagtagtc aaggcgtggc cacaccagcc 60
   atcac 65
<210> 11
   <211> 21
   <212> DNA
   <213> Artificial Sequence
<220>
   <221> source
   <223> /note="Description of Artificial Sequence: Synthetic primer"
<400> 11
   cattgagcat ggcatcgtca c 21
<210> 12
   <211> 20
   <212> DNA
   <213> Artificial Sequence
<220>
   <221> source
   <223> /note="Description of Artificial Sequence: Synthetic primer"
<400> 12
   ggtttggaca tggcaagctc 20
<210> 13
   <211> 287
   <212> DNA
   <213> Artificial Sequence
<220>
   <221> source
   <223> /note="Description of Artificial Sequence: Synthetic polynucleotide"
<400> 13
   cattgagcat ggcatcgtca ccaactggga cgacatggag aagatctggc accacacctt 60
   ctacaacgag ctgcgcgtgg ccccggagga gcacccagtg ctgctgaccg aggcccccct 120
   gaaccccaag gccaacagag agaagatgac tcaggtgcag acccacctcg aaaaccccac 180
   caagtaccac atacagcaag cccaacggca gcaggtaaag cagtaccttt ctaccacttt 240
   agcaaataaa catgccaacc aagtcctgag cttgccatgt ccaaacc 287
<210> 14
   <211> 95
   <212> PRT
   <213> Artificial Sequence
<220>
   <221> source
   <223> /note="Description of Artificial Sequence: Synthetic polypeptide"
<400> 14
   Ile Glu His Gly Ile Val Thr Asn Trp Asp Asp Met Glu Lys Ile Trp
   1 5 10 15
   His His Thr Phe Tyr Asn Glu Leu Arg Val Ala Pro Glu Glu His Pro
   20 25 30
   Val Leu Leu Thr Glu Ala Pro Leu Asn Pro Lys Ala Asn Arg Glu Lys
   35 40 45
   Met Thr Gln Val Gln Thr His Leu Glu Asn Pro Thr Lys Tyr His Ile
   50 55 60
   Gln Gln Ala Gln Arg Gln Gln Val Lys Gln Tyr Leu Ser Thr Thr Leu
   65 70 75 80
   Ala Asn Lys His Ala Asn Gln Val Leu Ser Leu Pro Cys Pro Asn
   85 90 95
<210> 15
   <211> 70
   <212> DNA
   <213> Artificial Sequence
<220>
   <221> source
   <223> /note="Description of Artificial Sequence: Synthetic oligonucleotide"
<400> 15
   tgagaagaga gatccacatc tggcctgtgt tgcttatgaa cgtggccaat gtgatctgga 60
   acttattaat 70
<210> 16
   <211> 66
   <212> DNA
   <213> Artificial Sequence
<220>
   <221> source
   <223> /note="Description of Artificial Sequence: Synthetic oligonucleotide"
<400> 16
   ctacccctgt ccagcagcca cctgaatgtg tacagcagcg acccccaggt cacagcctcc 60
   ctggtg 66
<210> 17
   <211> 20
   <212> DNA
   <213> Artificial Sequence
<220>
   <221> source
   <223> /note="Description of Artificial Sequence: Synthetic primer"
<400> 17
   gcttctgcct tggctagagg 20
<210> 18
   <211> 21
   <212> DNA
   <213> Artificial Sequence
<220>
   <221> source
   <223> /note="Description of Artificial Sequence: Synthetic primer"
<400> 18
   gtcgctgctg tacacattca g 21
<210> 19
   <211> 268
   <212> DNA
   <213> Artificial Sequence
<220>
   <221> source
   <223> /note="Description of Artificial Sequence: Synthetic polynucleotide"
<400> 19
   gcttctgcct tggctagagg ccagaattca tgagggctgt gaggagcctg ctactcacaa 60
   tgccttagcc aaaatctaca tagacagtaa taacaacccg gagagatttc ttcgtgaaaa 120
   tccctactat gacagtcgcg ttgttggaaa gtattgtgag aagagagatc cacatctggc 180
   ctgtgttgct tatgaacgtg gccaatgtga tctggaactt attaatctac ccctgtccag 240
   cagccacctg aatgtgtaca gcagcgac 268
<210> 20
   <211> 89
   <212> PRT
   <213> Artificial Sequence
<220>
   <221> source
   <223> /note="Description of Artificial Sequence: Synthetic polypeptide"
<400> 20
   Leu Leu Pro Trp Leu Glu Ala Arg Ile His Glu Gly Cys Glu Glu Pro
   1 5 10 15
   Ala Thr His Asn Ala Leu Ala Lys Ile Tyr Ile Asp Ser Asn Asn Asn
   20 25 30
   Pro Glu Arg Phe Leu Arg Glu Asn Pro Tyr Tyr Asp Ser Arg Val Val
   35 40 45
   Gly Lys Tyr Cys Glu Lys Arg Asp Pro His Leu Ala Cys Val Ala Tyr
   50 55 60
   Glu Arg Gly Gln Cys Asp Leu Glu Leu Ile Asn Leu Pro Leu Ser Ser
   65 70 75 80
   Ser His Leu Asn Val Tyr Ser Ser Asp
   85
<210> 21
   <211> 69
   <212> DNA
   <213> Artificial Sequence
<220>
   <221> source
   <223> /note="Description of Artificial Sequence: Synthetic oligonucleotide"
<400> 21
   cggccagcgc gcattcggcc ccggacgaag gtactcgcag cacttggagc gcagaaccgg 60
   ccgcgcccg 69
<210> 22
   <211> 70
   <212> DNA
   <213> Artificial Sequence
<220>
   <221> source
   <223> /note="Description of Artificial Sequence: Synthetic oligonucleotide"
<400> 22
   atcatgatgc tgccgccacc gccgccacca cggagcgaga agcccagata gacgccccgg 60
   cggccccggg 70
<210> 23
   <211> 20
   <212> DNA
   <213> Artificial Sequence
<220>
   <221> source
   <223> /note="Description of Artificial Sequence: Synthetic primer"
<400> 23
   gtactcgcag cacttggagc 20
<210> 24
   <211> 21
   <212> DNA
   <213> Artificial Sequence
<220>
   <221> source
   <223> /note="Description of Artificial Sequence: Synthetic primer"
<400> 24
   atctgggctt ctcgctccgt g 21
<210> 25
   <211> 88
   <212> DNA
   <213> Artificial Sequence
<220>
   <221> source
   <223> /note="Description of Artificial Sequence: Synthetic oligonucleotide"
<400> 25
   gtactcgcag cacttggagc gcagaaccgg ccgcgcccga tcatgatgct gccgccaccg 60
   ccgccaccac ggagcgagaa gcccagat 88
<210> 26
   <211> 4659
   <212> DNA
   <213> Artificial Sequence
<220>
   <221> source
   <223> /note="Description of Artificial Sequence: Synthetic polynucleotide"
<400> 26
   atggaagaag agatcgccgc gctggtcatt gacaatggct ccggcatgtg caaagctggt    60
   tttgctgggg acgacgctcc ccgagccgtg tttccttcca tcgtcgggcg ccccagacac   120
   cagggcgtca tggtgggcat gggccagaag gactcctacg tgggcgacga ggcccagagc   180
   aagcgtggca tcctgaccct gaagtacccc attgagcatg gcatcgtcac caactgggac   240
   gacatggaga agatctggca ccacaccttc tacaacgagc tgcgcgtggc cccggaggag 300
   cacccagtgc tgctgaccga ggcccccctg aaccccaagg ccaacagaga gaagatgact 360
   caggtgcaga cccacctcga aaaccccacc aagtaccaca tacagcaagc ccaacggcag 420
   caggtaaagc agtacctttc taccacttta gcaaataaac atgccaacca agtcctgagc 480
   ttgccatgtc caaaccagcc tggcgatcat gtcatgccac cggtgccggg gagcagcgca 540
   cccaacagcc ccatggctat gcttacgctt aactccaact gtgaaaaaga gggattttat 600
   aagtttgaag agcaaaacag ggcagagagc gagtgcccag gcatgaacac acattcacga 660
   gcgtcctgta tgcagatgga tgatgtaatc gatgacatca ttagcctaga atcaagttat 720
   aatgaggaaa tcttgggctt gatggatcct gctttgcaaa tggcaaatac gttgcctgtc 780
   tcgggaaact tgattgatct ttatggaaac caaggtctgc ccccaccagg cctcaccatc 840
   agcaactcct gtccagccaa ccttcccaac ataaaaaggg agctcacaga gtctgaagca 900
   agagcactgg ccaaagagag gcagaaaaag gacaatcaca acctgattga acgaagaaga 960
   agatttaaca taaatgaccg cattaaagaa ctaggtactt tgattcccaa gtcaaatgat 1020
   ccagacatgc gctggaacaa gggaaccatc ttaaaagcat ccgtggacta tatccgaaag 1080
   ttgcaacgag aacagcaacg cgcaaaagaa cttgaaaacc gacagaagaa actggagcac 1140
   gccaaccggc atttgttgct cagaatacag gaacttgaaa tgcaggctcg agctcatgga 1200
   ctttccctta ttccatccac gggtctctgc tctccagatt tggtgaatcg gatcatcaag 1260
   caagaacccg ttcttgagaa ctgcagccaa gacctccttc agcatcatgc agacctaacc 1320
   tgtacaacaa ctctcgatct cacggatggc accatcacct tcaacaacaa cctcggaact 1380
   gggactgagg ccaaccaagc ctatagtgtc cccacaaaaa tgggatccaa actggaagac 1440
   atcctgatgg acgacaccct ttctcccgtc ggtgtcactg atccactcct ttcctcagtg 1500
   tcccccggag cttccaaaac aagcagccgg aggagcagta tgagcatgga agagacggag 1560
   cacacttgtt agcgaatcct ccctgcactg cattcgcaca aactgcttcc tttcttgatt 1620
   cgtagattta ataacttacc tgaaggggtt ttcttgataa ttttccttta atatgaaatt 1680
   ttttttcatg ctttatcaat agcccaggat atattttatt tttagaattt tgtgaaacag 1740
   acttgtatat tctattttac aactacaaat gcctccaaag tattgtacaa ataagtgtgc 1800
   agtatctgtg aactgaattc accacagact ttagctttct gagcaagagg attttgcgtc 1860
   agagaaatgt ctgtccattt ttattcaggg gaaacttgat ttgagatttt tatgcctgtg 1920
   acttccttgg aaatcaaatg taaagtttaa ttgaaagaat gtaaagcaac caaaaagaaa 1980
   aaaaaaaaga aagaaagagg aaaagaaatc catactaacc cttttccatt ttataaatgt 2040
   attgattcat tggtactgcc ttaaagatac agtacccctc tagctttgtt tagtctttat 2100
   actgcaaact atttaaagaa atatgtattc tgtaaaagaa aaaaaaaatg cggccttttc 2160
   atgaggatcg tctggttaga aaacataact gataccaacc gaaactgaag ggagttagac 2220
   caaggctctg aaatataaag tctaatcttg ctctctttta ttctgtgctg ttacagtttt 2280
   cttcatcaat gagtgtgatc cagtttttca taagatattt tattttgaaa tggaaattaa 2340
   tgtcctctca aagtaaaata ttgaggagca ctgaaagtat gttttacttt ttttttattt 2400
   tatttttgct tttgataaga aaaccgaact gggcatattt ctaattggct ttactatttt 2460
   tatttttaaa ttatgtttta ctgttcattt gatttgtaca gattctttat tatcattgtt 2520
   cttttcaata tatttgtatt aatttgtaag aatatgcatc ttaaaatggc aagttttcca 2580
   tatttttaca actcactggt ggttttccgc attctttgta cacccatgaa agaaaacttt 2640
   tatgcaaggt cttgcattta aaagacagct ttgcgaatat tttgtaaatt acagtctcac 2700
   tcagaactgt ttttggacac atttaaggtg tagtattaat aggttaaaac caggctttct 2760
   agaaagaata aacttacata tttattttta ggacatgaaa atagcaatat tcttggagat 2820
   tgataaccat agcattaata cgcccattat ggtcatttaa attggggttt atttcagcaa 2880
   acttgttgaa tttattttta agaaagaaat actgtattgg gaagttactg ttacttgata 2940
   acaatgtttt aacaagaagc aatgttataa agttagtttc agtgcattat ctacttgtgt 3000
   agtcctatgc aataacagta gtgttacatg tatcaagcct agatgtttta tacagatgcc 3060
   atatagtgtt atgagccagg ctgttgaatg gaatttctca gtagcagcct acaactgaat 3120
   agcaagtggc ataaagcata tccattcaga atgaagtgcc ttaaatatag cagtagtctt 3180
   ttttggacta gcactgactg aactgtaatg taggggaaag tttcatgatg gtatctatag 3240
   tcaagacgaa catgtagcat ggtgcctatg tagacaatat aagagcttcc aattttcctt 3300
   cagatatttt taatattaaa tatattttag tgacagagtg ccaacttctt tcatcaggaa 3360
   accttattca ggagggtttt taaaaagtgt ttaaatgtca aatgtgaatt ggtgatgggt 3420
   gatggagggt tcagagagga gtgatcgtca gatgtgtgaa tggacggttt aggtgaaaat 3480
   aatcaactgc atagttccca tgcacgctgg gcaatgagaa tccttggaaa cattggtgat 3540
   gctatcagtt ttatagcttt atttcttaag gggtagggga aaattagttc ccattctttc 3600
   aaccccctta actgtatagc tcttttccta gaatagtgac gcaaatctgc atgaacagct 3660
   aattgtacca tagtgttcat tgatacaatc atagcattgt ctatttttct cttcatattt 3720
   atatgggggg gagggcgctg gatgcaaaag ttgaagatcg tgatgctatg atgttagttt 3780
   tccttagctg attttgaggg tttttaaaaa taaagcaagg ttgactaacc tacggccacg 3840
   ggaacaggac catggttaag caaccatata gaaagctttg ttgaaagaaa gtatggcatc 3900
   ttgtaccact gccctgactg tcacaactcc taaccttgcc attgcctgcc tccccctccc 3960
   cttctcctta agagacaatt tctgcaggtg gcaggtgagc aagcccagga gaatgctgca 4020
   atcttggggg tggttttatt tatttctttt ttgccaaata gagtgtggat tcatttcagg      4080
   ggctagctaa gccaagaggc agtggtttgg gcttgttgtt tgtaacaaga aaatgatcca      4140
   caccactccc ccgattcccg ggtgcagaat tgtaactcgg ggttgggcct ctatatggag      4200
   tgaccaaaat gccaaaattg tccatctgcc tctgagtagg gcaatggaaa taccaaacct      4260
   tctgactttg ccaaaaagca tacaagcaac ctggtcatac ataggatgac aaaattcttt      4320
   ctggttgttt ttaaacaata aagcaataag aacaaataca atacatagga agttaaaagc      4380
   acaaaggaat gaacttatta atatttttga aaaatgcact gggaaaaagt tgatgtcaat      4440
   aacagtataa aacagcccta tttcttgata aaaaatgaca aatgactgtc tcttgcggat      4500
   gcttggtact gtaatgttaa taatagtcac ctgctgttgg atgcagcaat aatttctgta      4560
   tggtccatag cactgtatat tatggatcga tattaatgta tccaatgaaa taatcgactt      4620
   gttcttgata gcctcattaa agcatttggt ttttcacat                             4659
<210> 27
   <211> 523
   <212> PRT
   <213> Artificial Sequence
<220>
   <221> source
   <223> /note="Description of Artificial Sequence: Synthetic polypeptide"
<400> 27
   Met Glu Glu Glu Ile Ala Ala Leu Val Ile Asp Asn Gly Ser Gly Met
   1               5                   10                  15
   Cys Lys Ala Gly Phe Ala Gly Asp Asp Ala Pro Arg Ala Val Phe Pro
               20                  25                  30
   Ser Ile Val Gly Arg Pro Arg His Gln Gly Val Met Val Gly Met Gly
           35                  40                  45
   Gln Lys Asp Ser Tyr Val Gly Asp Glu Ala Gln Ser Lys Arg Gly Ile
       50                  55                  60
   Leu Thr Leu Lys Tyr Pro Ile Glu His Gly Ile Val Thr Asn Trp Asp
   65                  70                  75                  80
   Asp Met Glu Lys Ile Trp His His Thr Phe Tyr Asn Glu Leu Arg Val
                   85                  90                  95
   Ala Pro Glu Glu His Pro Val Leu Leu Thr Glu Ala Pro Leu Asn Pro
               100                 105                 110
   Lys Ala Asn Arg Glu Lys Met Thr Gln Val Gln Thr His Leu Glu Asn
           115                 120                 125
   Pro Thr Lys Tyr His Ile Gln Gln Ala Gln Arg Gln Gln Val Lys Gln
       130                 135                 140
   Tyr Leu Ser Thr Thr Leu Ala Asn Lys His Ala Asn Gln Val Leu Ser
   145                 150                 155                 160
   Leu Pro Cys Pro Asn Gln Pro Gly Asp His Val Met Pro Pro Val Pro
                   165                 170                 175
   Gly Ser Ser Ala Pro Asn Ser Pro Met Ala Met Leu Thr Leu Asn Ser
               180                 185                 190
   Asn Cys Glu Lys Glu Gly Phe Tyr Lys Phe Glu Glu Gln Asn Arg Ala
           195                 200                 205
   Glu Ser Glu Cys Pro Gly Met Asn Thr His Ser Arg Ala Ser Cys Met
       210                 215                 220
   Gln Met Asp Asp Val Ile Asp Asp Ile Ile Ser Leu Glu Ser Ser Tyr
   225                 230                 235                 240
   Asn Glu Glu Ile Leu Gly Leu Met Asp Pro Ala Leu Gln Met Ala Asn
                   245                 250                 255
   Thr Leu Pro Val Ser Gly Asn Leu Ile Asp Leu Tyr Gly Asn Gln Gly
               260                 265                 270
   Leu Pro Pro Pro Gly Leu Thr Ile Ser Asn Ser Cys Pro Ala Asn Leu
           275                 280                 285
   Pro Asn Ile Lys Arg Glu Leu Thr Glu Ser Glu Ala Arg Ala Leu Ala
       290                 295                 300
   Lys Glu Arg Gln Lys Lys Asp Asn His Asn Leu Ile Glu Arg Arg Arg
   305                 310                 315                 320
   Arg Phe Asn Ile Asn Asp Arg Ile Lys Glu Leu Gly Thr Leu Ile Pro
                   325                 330                 335
   Lys Ser Asn Asp Pro Asp Met Arg Trp Asn Lys Gly Thr Ile Leu Lys
               340                 345                 350
   Ala Ser Val Asp Tyr Ile Arg Lys Leu Gln Arg Glu Gln Gln Arg Ala
           355                 360                 365
   Lys Glu Leu Glu Asn Arg Gln Lys Lys Leu Glu His Ala Asn Arg His
       370                 375                 380
   Leu Leu Leu Arg Ile Gln Glu Leu Glu Met Gln Ala Arg Ala His Gly
   385                 390                 395                 400
   Leu Ser Leu Ile Pro Ser Thr Gly Leu Cys Ser Pro Asp Leu Val Asn
                   405                 410                 415
   Arg Ile Ile Lys Gln Glu Pro Val Leu Glu Asn Cys Ser Gln Asp Leu
               420                 425                 430
   Leu Gln His His Ala Asp Leu Thr Cys Thr Thr Thr Leu Asp Leu Thr
           435                 440                 445
   Asp Gly Thr Ile Thr Phe Asn Asn Asn Leu Gly Thr Gly Thr Glu Ala
       450                 455                 460
   Asn Gln Ala Tyr Ser Val Pro Thr Lys Met Gly Ser Lys Leu Glu Asp
   465                 470                 475                 480
   Ile Leu Met Asp Asp Thr Leu Ser Pro Val Gly Val Thr Asp Pro Leu
                   485                 490                 495
   Leu Ser Ser Val Ser Pro Gly Ala Ser Lys Thr Ser Ser Arg Arg Ser
               500                 505                 510
   Ser Met Ser Met Glu Glu Thr Glu His Thr Cys
           515                 520
<210> 28
   <211> 4298
   <212> DNA
   <213> Artificial Sequence
<220>
   <221> source
   <223> /note="Description of Artificial Sequence: Synthetic polynucleotide"
<400> 28
   atggcccaga ttctgccaat tcgttttcag gagcatctcc agctccagaa cctgggtatc        60
   aacccagcaa acattggctt cagtaccctg actatggagt ctgacaaatt catctgcatt       120
   agagaaaaag taggagagca ggcccaggtg gtaatcattg atatgaatga cccaagtaat       180
   ccaattcgaa gaccaatttc agcagacagc gccatcatga atccagctag caaagtaatt       240
   gcactgaaag ctgggaaaac tcttcagatt tttaacattg aaatgaaaag taaaatgaag       300
   gctcatacca tgactgatga tgtcaccttt tggaaatgga tctctttgaa tacggttgct       360
   cttgttacgg ataatgcagt ttatcactgg agtatggaag gagagtctca gccagtgaaa      420
   atgtttgatc gccattctag ccttgcaggg tgccagatta tcaattaccg tacagatgca      480
   aaacaaaagt ggttacttct gactggtata tctgcacagc aaaatcgtgt ggtgggagct      540
   atgcagctat attctgtaga taggaaagtg tctcagccca ttgaaggaca tgcagctagc      600
   tttgcacagt ttaagatgga aggaaatgca gaagaatcaa cgttattttg ttttgcagtt      660
   cggggccaag ctggagggaa gttacatatt attgaagttg gcacaccacc tacagggaac      720
   cagccctttc caaagaaggc agtggatgtc ttctttcctc cagaagcaca aaatgatttt      780
   cctgttgcaa tgcagatcag tgaaaagcat gatgtggtgt tcttgataac caagtatggt      840
   tatatccacc tctatgatct tgagactggt acctgcatct acatgaatag aatcagtgga      900
   gaaacaattt ttgttactgc acctcatgaa gccacagctg gaataattgg agtaaacaga      960
   aagggacaag ttctgtcagt gtgtgtggaa gaagaaaaca taattcctta catcaccaat     1020
   gttctacaaa atcctgattt ggctctgaga atggctgtac gtaataactt agccggtgct     1080
   gaagaactct ttgcccggaa atttaatgct ctttttgccc agggaaatta ctcggaggca     1140
   gcaaaggtgg ctgctaatgc accaaaggga attcttcgta ctccagacac tatccgtcgg     1200
   ttccagagtg tcccagccca gccaggtcaa acttctcctc tacttcagta ctttggtatc     1260
   cttttggacc agggacagct caacaaatac gaatccttag agctttgtag gcctgtactt     1320
   cagcaagggc gaaaacagct tttggagaaa tggttaaaag aagataagct ggaatgttct     1380
   gaagaactgg gtgatcttgt gaaatctgtg gaccctacat tggcacttag tgtgtaccta     1440
   agggctaacg tcccaaataa agtcattcag tgctttgcag aaacaggtca agtccaaaag     1500
   attgttttat atgctaaaaa agttggatac actccagatt ggatatttct gctgagaaat     1560
   gtaatgcgaa tcagtccaga tcagggacag cagtttgccc aaatgttagt tcaagatgaa     1620
   gagcctcttg ctgacatcac acagattgta gatgtcttta tggaatacaa tctaattcag     1680
   cagtgtactg cattcttgct tgatgctctg aagaataatc gcccatctga aggtccttta     1740
   cagacgcggt tacttgagat gaaccttatg catgcgcctc aagttgcaga tgctattcta     1800
   ggcaatcaga tgttcacaca ttatgaccgg gctcatattg ctcaactgtg tgaaaaggct     1860
   ggcctactgc agcgtgcatt agaacatttc actgatttat atgatataaa acgtgcagtg     1920
   gttcacaccc atcttcttaa ccctgagtgg ttagtcaact actttggttc cttatcagta     1980
   gaagactccc tagaatgtct cagagccatg ctgtctgcca acatccgtca gaatctgcag     2040
   atttgtgttc aggtggcttc taaatatcat gaacaactgt caactcagtc tctgattgaa     2100
   ctttttgaat ctttcaagag ttttgaaggt ctcttttatt ttctgggatc cattgttaac     2160
   tttagccagg acccagatgt gcactttaaa tatattcagg cagcttgcaa gactgggcaa     2220
   atcaaagaag tagaaagaat ctgtagagaa agcaactgct acgatcctga gcgagtcaag 2280
   aattttctta aggaagcaaa actaacagat cagctaccac ttatcattgt gtgtgatcga 2340
   tttgactttg tccatgattt ggtgctctat ttatatagaa ataatcttca aaagtatata 2400
   gagatatatg tacagaaggt gaatccaagt cgacttcctg tagttattgg aggattactt 2460
   gatgttgact gttctgaaga tgtcataaaa aacttgattc ttgttgtaag aggtcaattc 2520
   tctactgatg agcttgttgc tgaggttgaa aaaagaaaca gattgaaact gcttctgcct 2580
   tggctagagg ccagaattca tgagggctgt gaggagcctg ctactcacaa tgccttagcc 2640
   aaaatctaca tagacagtaa taacaacccg gagagatttc ttcgtgaaaa tccctactat 2700
   gacagtcgcg ttgttggaaa gtattgtgag aagagagatc cacatctggc ctgtgttgct 2760
   tatgaacgtg gccaatgtga tctggaactt attaatctac ccctgtccag cagccacctg 2820
   aatgtgtaca gcagcgaccc ccaggtcaca gcctccctgg tgggcgtcac cagcagctcc 2880
   tgccctgcgg acctgaccca gaagcgagag ctcacagatg ctgagagcag ggccctggcc 2940
   aaggagcggc agaagaaaga caatcacaac ttaattgaaa ggagacgaag gttcaacatc 3000
   aatgaccgca tcaaggagtt gggaatgctg atccccaagg ccaatgacct ggacgtgcgc 3060
   tggaacaagg gcaccatcct caaggcctct gtggattaca tccggaggat gcagaaggac 3120
   ctgcaaaagt ccagggagct ggagaaccac tctcgccgcc tggagatgac caacaagcag 3180
   ctctggctcc gtatccagga gctggagatg caggctcgag tgcacggcct ccctaccacc 3240
   tccccgtccg gcatgaacat ggctgagctg gcccagcagg tggtgaagca ggagctgcct 3300
   agcgaagagg gcccagggga ggccctgatg ctgggggctg aggtccctga ccctgagcca 3360
   ctgccagctc tgcccccgca agccccgctg cccctgccca cccagccacc atccccattc 3420
   catcacctgg acttcagcca cagcctgagc tttgggggca gggaggacga gggtcccccg 3480
   ggctaccccg aacccctggc gccggggcat ggctccccat tccccagcct gtccaagaag 3540
   gatctggacc tcatgctcct ggacgactca ctgctaccgc tggcctctga tccacttctg 3600
   tccaccatgt cccccgaggc ctccaaggcc agcagccgcc ggagcagctt cagcatggag 3660
   gagggcgatg tgctgtgacc ctggctgccc ctgtgccagg gaacaggggc cggcctgggg 3720
   gctgggaggg ccaggggcac ctccctccca cccttcaggc tgcactgtgt gtgaagtagc 3780
   cacctgccct gcctccctcc tccccgttgg cccctgtttg gacttagtgc ctgtctggca 3840
   gcctgtgggg tcaggagaag cacccccagg gcagccctct tgactggcgc agtgggaaga 3900
   ggccttcagc ccctctcccg gagatggaat cgcggggcag ggaggggcag ggtgttctag 3960
   aggtgagaag agggcctggt ggagattccc tgtcttctga gcccgagccc ctcattacca 4020
   gtgaaggaca tgcttgaggg gttcgggaag ctcctcatct gaggcaactg gtcctggggg 4080
   tgctcaggcc tgcctttttg ggactcagat ggcaggaggt ccaccccgca gcctggtcct 4140
   cggctctccc acaggtgggc accccccact ttggtgctaa tagctctcca ccaggtggtg 4200
   tgagcgcggg ggctgccaga agcgggaggg gtcactgccg gaagagcagc tgccctccga 4260
   cccctcactt tgtgccttta gtaaacactg tgctttgt 4298
<210> 29
   <211> 1225
   <212> PRT
   <213> Artificial Sequence
<220>
   <221> source
   <223> /note="Description of Artificial Sequence: Synthetic polypeptide"
<400> 29
   Met Ala Gln Ile Leu Pro Ile Arg Phe Gln Glu His Leu Gln Leu Gln
   1 5 10 15
   Asn Leu Gly Ile Asn Pro Ala Asn Ile Gly Phe Ser Thr Leu Thr Met
   20 25 30
   Glu Ser Asp Lys Phe Ile Cys Ile Arg Glu Lys Val Gly Glu Gln Ala
   35 40 45
   Gln Val Val Ile Ile Asp Met Asn Asp Pro Ser Asn Pro Ile Arg Arg
   50 55 60
   Pro Ile Ser Ala Asp Ser Ala Ile Met Asn Pro Ala Ser Lys Val Ile
   65 70 75 80
   Ala Leu Lys Ala Gly Lys Thr Leu Gln Ile Phe Asn Ile Glu Met Lys
   85 90 95
   Ser Lys Met Lys Ala His Thr Met Thr Asp Asp Val Thr Phe Trp Lys
   100 105 110
   Trp Ile Ser Leu Asn Thr Val Ala Leu Val Thr Asp Asn Ala Val Tyr
   115 120 125
   His Trp Ser Met Glu Gly Glu Ser Gln Pro Val Lys Met Phe Asp Arg
   130 135 140
   His Ser Ser Leu Ala Gly Cys Gln Ile Ile Asn Tyr Arg Thr Asp Ala
   145 150 155 160
   Lys Gln Lys Trp Leu Leu Leu Thr Gly Ile Ser Ala Gln Gln Asn Arg
   165 170 175
   Val Val Gly Ala Met Gln Leu Tyr Ser Val Asp Arg Lys Val Ser Gln
   180 185 190
   Pro Ile Glu Gly His Ala Ala Ser Phe Ala Gln Phe Lys Met Glu Gly
   195 200 205
   Asn Ala Glu Glu Ser Thr Leu Phe Cys Phe Ala Val Arg Gly Gln Ala
   210 215 220
   Gly Gly Lys Leu His Ile Ile Glu Val Gly Thr Pro Pro Thr Gly Asn
   225 230 235 240
   Gln Pro Phe Pro Lys Lys Ala Val Asp Val Phe Phe Pro Pro Glu Ala
   245 250 255
   Gln Asn Asp Phe Pro Val Ala Met Gln Ile Ser Glu Lys His Asp Val
   260 265 270
   Val Phe Leu Ile Thr Lys Tyr Gly Tyr Ile His Leu Tyr Asp Leu Glu
   275 280 285
   Thr Gly Thr Cys Ile Tyr Met Asn Arg Ile Ser Gly Glu Thr Ile Phe
   290 295 300
   Val Thr Ala Pro His Glu Ala Thr Ala Gly Ile Ile Gly Val Asn Arg
   305 310 315 320
   Lys Gly Gln Val Leu Ser Val Cys Val Glu Glu Glu Asn Ile Ile Pro
   325 330 335
   Tyr Ile Thr Asn Val Leu Gln Asn Pro Asp Leu Ala Leu Arg Met Ala
   340 345 350
   Val Arg Asn Asn Leu Ala Gly Ala Glu Glu Leu Phe Ala Arg Lys Phe
   355 360 365
   Asn Ala Leu Phe Ala Gln Gly Asn Tyr Ser Glu Ala Ala Lys Val Ala
   370 375 380
   Ala Asn Ala Pro Lys Gly Ile Leu Arg Thr Pro Asp Thr Ile Arg Arg
   385 390 395 400
   Phe Gln Ser Val Pro Ala Gln Pro Gly Gln Thr Ser Pro Leu Leu Gln
   405 410 415
   Tyr Phe Gly Ile Leu Leu Asp Gln Gly Gln Leu Asn Lys Tyr Glu Ser
   420 425 430
   Leu Glu Leu Cys Arg Pro Val Leu Gln Gln Gly Arg Lys Gln Leu Leu
   435 440 445
   Glu Lys Trp Leu Lys Glu Asp Lys Leu Glu Cys Ser Glu Glu Leu Gly
   450 455 460
   Asp Leu Val Lys Ser Val Asp Pro Thr Leu Ala Leu Ser Val Tyr Leu
   465 470 475 480
   Arg Ala Asn Val Pro Asn Lys Val Ile Gln Cys Phe Ala Glu Thr Gly
   485 490 495
   Gln Val Gln Lys Ile Val Leu Tyr Ala Lys Lys Val Gly Tyr Thr Pro
   500 505 510
   Asp Trp Ile Phe Leu Leu Arg Asn Val Met Arg Ile Ser Pro Asp Gln
   515 520 525
   Gly Gln Gln Phe Ala Gln Met Leu Val Gln Asp Glu Glu Pro Leu Ala
   530 535 540
   Asp Ile Thr Gln Ile Val Asp Val Phe Met Glu Tyr Asn Leu Ile Gln
   545 550 555 560
   Gln Cys Thr Ala Phe Leu Leu Asp Ala Leu Lys Asn Asn Arg Pro Ser
   565 570 575
   Glu Gly Pro Leu Gln Thr Arg Leu Leu Glu Met Asn Leu Met His Ala
   580 585 590
   Pro Gln Val Ala Asp Ala Ile Leu Gly Asn Gln Met Phe Thr His Tyr
   595 600 605
   Asp Arg Ala His Ile Ala Gln Leu Cys Glu Lys Ala Gly Leu Leu Gln
   610 615 620
   Arg Ala Leu Glu His Phe Thr Asp Leu Tyr Asp Ile Lys Arg Ala Val
   625 630 635 640
   Val His Thr His Leu Leu Asn Pro Glu Trp Leu Val Asn Tyr Phe Gly
   645 650 655
   Ser Leu Ser Val Glu Asp Ser Leu Glu Cys Leu Arg Ala Met Leu Ser
   660 665 670
   Ala Asn Ile Arg Gln Asn Leu Gln Ile Cys Val Gln Val Ala Ser Lys
   675 680 685
   Tyr His Glu Gln Leu Ser Thr Gln Ser Leu Ile Glu Leu Phe Glu Ser
   690 695 700
   Phe Lys Ser Phe Glu Gly Leu Phe Tyr Phe Leu Gly Ser Ile Val Asn
   705 710 715 720
   Phe Ser Gln Asp Pro Asp Val His Phe Lys Tyr Ile Gln Ala Ala Cys
   725 730 735
   Lys Thr Gly Gln Ile Lys Glu Val Glu Arg Ile Cys Arg Glu Ser Asn
   740 745 750
   Cys Tyr Asp Pro Glu Arg Val Lys Asn Phe Leu Lys Glu Ala Lys Leu
   755 760 765
   Thr Asp Gln Leu Pro Leu Ile Ile Val Cys Asp Arg Phe Asp Phe Val
   770 775 780
   His Asp Leu Val Leu Tyr Leu Tyr Arg Asn Asn Leu Gln Lys Tyr Ile
   785 790 795 800
   Glu Ile Tyr Val Gln Lys Val Asn Pro Ser Arg Leu Pro Val Val Ile
   805 810 815
   Gly Gly Leu Leu Asp Val Asp Cys Ser Glu Asp Val Ile Lys Asn Leu
   820 825 830
   Ile Leu Val Val Arg Gly Gln Phe Ser Thr Asp Glu Leu Val Ala Glu
   835 840 845
   Val Glu Lys Arg Asn Arg Leu Lys Leu Leu Leu Pro Trp Leu Glu Ala
   850 855 860
   Arg Ile His Glu Gly Cys Glu Glu Pro Ala Thr His Asn Ala Leu Ala
   865 870 875 880
   Lys Ile Tyr Ile Asp Ser Asn Asn Asn Pro Glu Arg Phe Leu Arg Glu
   885 890 895
   Asn Pro Tyr Tyr Asp Ser Arg Val Val Gly Lys Tyr Cys Glu Lys Arg
   900 905 910
   Asp Pro His Leu Ala Cys Val Ala Tyr Glu Arg Gly Gln Cys Asp Leu
   915 920 925
   Glu Leu Ile Asn Leu Pro Leu Ser Ser Ser His Leu Asn Val Tyr Ser
   930 935 940
   Ser Asp Pro Gln Val Thr Ala Ser Leu Val Gly Val Thr Ser Ser Ser
   945 950 955 960
   Cys Pro Ala Asp Leu Thr Gln Lys Arg Glu Leu Thr Asp Ala Glu Ser
   965 970 975
   Arg Ala Leu Ala Lys Glu Arg Gln Lys Lys Asp Asn His Asn Leu Ile
   980 985 990
   Glu Arg Arg Arg Arg Phe Asn Ile Asn Asp Arg Ile Lys Glu Leu Gly
   995 1000 1005
   Met Leu Ile Pro Lys Ala Asn Asp Leu Asp Val Arg Trp Asn Lys
   1010 1015 1020
   Gly Thr Ile Leu Lys Ala Ser Val Asp Tyr Ile Arg Arg Met Gln
   1025 1030 1035
   Lys Asp Leu Gln Lys Ser Arg Glu Leu Glu Asn His Ser Arg Arg
   1040 1045 1050
   Leu Glu Met Thr Asn Lys Gln Leu Trp Leu Arg Ile Gln Glu Leu
   1055 1060 1065
   Glu Met Gln Ala Arg Val His Gly Leu Pro Thr Thr Ser Pro Ser
   1070 1075 1080
   Gly Met Asn Met Ala Glu Leu Ala Gln Gln Val Val Lys Gln Glu
   1085 1090 1095
   Leu Pro Ser Glu Glu Gly Pro Gly Glu Ala Leu Met Leu Gly Ala
   1100 1105 1110
   Glu Val Pro Asp Pro Glu Pro Leu Pro Ala Leu Pro Pro Gln Ala
   1115 1120 1125
   Pro Leu Pro Leu Pro Thr Gln Pro Pro Ser Pro Phe His His Leu
   1130 1135 1140
   Asp Phe Ser His Ser Leu Ser Phe Gly Gly Arg Glu Asp Glu Gly
   1145 1150 1155
   Pro Pro Gly Tyr Pro Glu Pro Leu Ala Pro Gly His Gly Ser Pro
   1160 1165 1170
   Phe Pro Ser Leu Ser Lys Lys Asp Leu Asp Leu Met Leu Leu Asp
   1175 1180 1185
   Asp Ser Leu Leu Pro Leu Ala Ser Asp Pro Leu Leu Ser Thr Met
   1190 1195 1200
   Ser Pro Glu Ala Ser Lys Ala Ser Ser Arg Arg Ser Ser Phe Ser
   1205 1210 1215
   Met Glu Glu Gly Asp Val Leu
   1220 1225
<210> 30
   <211> 8
   <212> PRT
   <213> Artificial Sequence
<220>
   <221> source
   <223> /note="Description of Artificial Sequence: Synthetic peptide"
<400> 30
   Lys Met Thr Gln Val Gln Thr His
   1 5
<210> 31
   <211> 11
   <212> PRT
   <213> Artificial Sequence
<220>
   <221> source
   <223> /note="Description of Artificial Sequence: Synthetic peptide"
<400> 31
   Asp Leu Glu Leu Ile Asn Leu Pro Leu Ser Ser
   1 5 10
<210> 32
   <211> 50
   <212> PRT
   <213> Artificial Sequence
<220>
   <221> source
   <223> /note="Description of Artificial Sequence: Synthetic polypeptide"
<400> 32
   Glu Glu Arg Thr Cys Lys Val Cys Leu Asp Arg Ala Val Ser Ile Val
   1 5 10 15
   Phe Val Pro Cys Gly His Leu Val Cys Ala Glu Cys Ala Pro Gly Leu
   20 25 30
   Gln Leu Cys Pro Ile Cys Arg Ala Pro Val Arg Ser Arg Val Arg Thr
   35 40 45
   Phe Leu
   50
<210> 33
   <211> 14
   <212> PRT
   <213> Artificial Sequence
<220>
   <221> source
   <223> /note="Description of Artificial Sequence: Synthetic peptide"
<400> 33
   Cys Arg Ala Pro Val Arg Ser Arg Val Arg Thr Phe Leu Ser
   1 5 10
<210> 34
   <211> 21
   <212> DNA
   <213> Artificial Sequence
<220>
   <221> source
   <223> /note="Description of Artificial Sequence: Synthetic oligonucleotide"
<400> 34
   ggccgcgccc gatcatgatg c 21
<210> 35
   <211> 120
   <212> DNA
   <213> Artificial Sequence
<220>
   <221> source
   <223> /note="Description of Artificial Sequence: Synthetic polynucleotide"
<400> 35
   agcgcgcatt cggccccgga cgaaggtact cgcagcactt ggagcgcaga accggccgcg 60
   cccgatcatg atgctgccgc caccgccgcc accacggagc gagaagccca gatagacgcc 120
<210> 36
   <211> 75
   <212> DNA
   <213> Artificial Sequence
<220>
   <221> source
   <223> /note="Description of Artificial Sequence: Synthetic oligonucleotide"
<400> 36
   gcgcgcattc ggccccggac gaaggtactc gcagcacttg gagcgcagaa ccggccgcgc 60
   ccgatcatga tgctg 75
<210> 37
   <211> 75
   <212> DNA
   <213> Artificial Sequence
<220>
   <221> source
   <223> /note="Description of Artificial Sequence: Synthetic oligonucleotide"
<400> 37
   cgcattcggc cccggacgaa ggtactcgca gcacttggag cgcagaaccg gccgcgcccg 60
   atcatgatgc tgccg 75
<210> 38
   <211> 75
   <212> **DNA**
   <213> Artificial Sequence
<220>
   <221> source
   <223> /note="Description of Artificial Sequence: Synthetic oligonucleotide"
<400> 38
   aggtactcgc agcacttgga gcgcagaacc ggccgcgccc gatcatgatg ctgccgccac 60
   cgccgccacc acgga 75
<210> 39
   <211> 75
   <212> DNA
   <213> Artificial Sequence
<220>
   <221> source
   <223> /note="Description of Artificial Sequence: Synthetic oligonucleotide"
<400> 39
   acttggagcg cagaaccggc cgcgcccgat catgatgctg ccgccaccgc cgccaccacg 60
   gagcgagaag cccag 75
<210> 40
   <211> 75
   <212> DNA
   <213> Artificial Sequence
<220>
   <221> source
   <223> /note="Description of Artificial Sequence: Synthetic oligonucleotide"
<400> 40
   agcgcagaac cggccgcgcc cgatcatgat gctgccgcca ccgccgccac cacggagcga 60
   gaagcccaga tagac 75
<210> 41
   <211> 69
   <212> DNA
   <213> Artificial Sequence
<220>
   <221> source
   <223> /note="Description of Artificial Sequence: Synthetic oligonucleotide"
<400> 41
   ccggccgcgc ccgatcatga tgctgccgcc accgccgcca ccacggagcg agaagcccag 60
   atagacgcc 69
<210> 42
   <211> 20
   <212> DNA
   <213> Artificial Sequence
<220>
   <221> source
   <223> /note="Description of Artificial Sequence: Synthetic oligonucleotide"
<400> 42
   acttattaat ctacccctgt 20
<210> 43
   <211> 116
   <212> DNA
   <213> Artificial Sequence
<220>
   <221> source
   <223> /note="Description of Artificial Sequence: Synthetic polynucleotide"
<400> 43
   gatccacatc tggcctgtgt tgcttatgaa cgtggccaat gtgatctgga acttattaat 60
   ctacccctgt ccagcagcca cctgaatgtg tacagcagcg acccccaggt cacagc 116
<210> 44
   <211> 76
   <212> DNA
   <213> Artificial Sequence
<220>
   <221> source
   <223> /note="Description of Artificial Sequence: Synthetic oligonucleotide"
<400> 44
   ccacatctgg cctgtgttgc ttatgaacgt ggccaatgtg atctggaact tattaatcta 60
   cccctgtcca gcagcc 76
<210> 45
   <211> 76
   <212> DNA
   <213> Artificial Sequence
<220>
   <221> source
   <223> /note="Description of Artificial Sequence: Synthetic oligonucleotide"
<400> 45
   tggcctgtgt tgcttatgaa cgtggccaat gtgatctgga acttattaat ctacccctgt 60
   ccagcagcca cctgaa 76
<210> 46
   <211> 76
   <212> DNA
   <213> Artificial Sequence
<220>
   <221> source
   <223> /note="Description of Artificial Sequence: Synthetic oligonucleotide"
<400> 46
   ttatgaacgt ggccaatgtg atctggaact tattaatcta cccctgtcca gcagccacct 60
   gaatgtgtac agcagc 76
<210> 47
   <211> 76
   <212> DNA
   <213> Artificial Sequence
<220>
   <221> source
   <223> /note="Description of Artificial Sequence: Synthetic oligonucleotide"
<400> 47
   gtggccaatg tgatctggaa cttattaatc tacccctgtc cagcagccac ctgaatgtgt 60
   acagcagcga ccccca 76
<210> 48
   <211> 76
   <212> DNA
   <213> Artificial Sequence
<220>
   <221> source
   <223> /note="Description of Artificial Sequence: Synthetic oligonucleotide"
<400> 48
   aatgtgatct ggaacttatt aatctacccc tgtccagcag ccacctgaat gtgtacagca 60
   gcgaccccca ggtcac 76
<210> 49
   <211> 76
   <212> DNA
   <213> Artificial Sequence
<220>
   <221> source
   <223> /note="Description of Artificial Sequence: Synthetic oligonucleotide"
<400> 49
   atgtgatctg gaacttatta atctacccct gtccagcagc cacctgaatg tgtacagcag 60
   cgacccccag gtcaca 76
<210> 50
   <211> 20
   <212> DNA
   <213> Artificial Sequence
<220>
   <221> source
   <223> /note="Description of Artificial Sequence: Synthetic oligonucleotide"
<400> 50
   gatgactcag gtgcagaccc 20
<210> 51
   <211> 114
   <212> DNA
   <213> Artificial Sequence
<220>
   <221> source
   <223> /note="Description of Artificial Sequence: Synthetic polynucleotide"
<400> 51
   ctgctgaccg aggcccccct gaaccccaag gccaacagag agaagatgac tcaggtgcag 60
   acccacctcg aaaaccccac caagtaccac atacagcaag cccaacggca gcag 114
<210> 52
   <211> 76
   <212> DNA
   <213> Artificial Sequence
<220>
   <221> source
   <223> /note="Description of Artificial Sequence: Synthetic oligonucleotide"
<400> 52
   tgctgaccga ggcccccctg aaccccaagg ccaacagaga gaagatgact caggtgcaga 60
   cccacctcga aaaccc 76
<210> 53
   <211> 76
   <212> DNA
   <213> Artificial Sequence
<220>
   <221> source
   <223> /note="Description of Artificial Sequence: Synthetic oligonucleotide"
<400> 53
   cgaggccccc ctgaacccca aggccaacag agagaagatg actcaggtgc agacccacct 60
   cgaaaacccc accaag 76
<210> 54
   <211> 76
   <212> DNA
   <213> Artificial Sequence
<220>
   <221> source
   <223> /note="Description of Artificial Sequence: Synthetic oligonucleotide"
<400> 54
   ctgaacccca aggccaacag agagaagatg actcaggtgc agacccacct cgaaaacccc 60
   accaagtacc acatac 76
<210> 55
   <211> 76
   <212> DNA
   <213> Artificial Sequence
<220>
   <221> source
   <223> /note="Description of Artificial Sequence: Synthetic oligonucleotide"
<400> 55
   cccaaggcca acagagagaa gatgactcag gtgcagaccc acctcgaaaa ccccaccaag 60
   taccacatac agcaag 76
<210> 56
   <211> 76
   <212> DNA
   <213> Artificial Sequence
<220>
   <221> source
   <223> /note="Description of Artificial Sequence: Synthetic oligonucleotide"
<400> 56
   aggccaacag agagaagatg actcaggtgc agacccacct cgaaaacccc accaagtacc 60
   acatacagca agccca 76
<210> 57
   <211> 76
   <212> DNA
   <213> Artificial Sequence
<220>
   <221> source
   <223> /note="Description of Artificial Sequence: Synthetic oligonucleotide"
<400> 57
   agagagaaga tgactcaggt gcagacccac ctcgaaaacc ccaccaagta ccacatacag 60
   caagcccaac ggcagc 76